(19) Europäisches Patentamt
European Patent Office
Office européen des brevets

(11) EP 0 638 566 B1

(12) # EUROPEAN PATENT SPECIFICATION

(45) Date of publication and mention of the grant of the patent: **07.01.1999 Bulletin 1999/01**

(51) Int. Cl.$^6$: **C07D 311/30**, A61K 31/35

(21) Application number: **94112509.8**

(22) Date of filing: **10.08.1994**

(54) **5-Aminoflavone derivatives, their preparation and their use as antibacterial, anti-estrogenic and/or antitumor agent**

5-Aminoflavon Derivate, deren Herstellung und deren Verwendung als antibakterielle und/oder anti-östrogene Mittel und/oder Antitumormittel

Dérivés de 5-aminoflavone, leur préparation et leur utilisation comme agents antibactériens et/ou antioestrogènes et/ou antitumoraux

(84) Designated Contracting States:
**DE ES FR GB IT**

(30) Priority: **11.08.1993 JP 199310/93**
**18.08.1993 JP 204356/93**

(43) Date of publication of application:
**15.02.1995 Bulletin 1995/07**

(73) Proprietor:
**KYOWA HAKKO KOGYO CO., LTD.**
**Chiyoda-ku, Tokyo-to (JP)**

(72) Inventors:
- **Akama, Tsutomu**
  **Sunto-gun, Shizuoka-ken (JP)**
- **Ikeda, Shun-ichi**
  **Numazu-shi, Shizuoka-ken (JP)**
- **Ishida, Hiroyuki**
  **Sunto-gun, Shizuoka-ken (JP)**
- **Kimura, Uichiro**
  **Sunto-gun, Shizuoka-ken (JP)**
- **Gomi, Katsushige**
  **Susono-gun, Shizuoka-ken (JP)**
- **Saito, Hiromitsu**
  **Kawasaki-shi, Kanagawa-ken (JP)**

(74) Representative:
**VOSSIUS & PARTNER**
**Siebertstrasse 4**
**81675 München (DE)**

(56) References cited:
**EP-A- 0 374 789** **EP-A- 0 556 720**

- **INDIAN JOURNAL OF CHEMISTRY vol. 1 , November 1963 pages 477 - 479 JOSHI K.C. & JAUHAR A.K. 'Studies in fluorochalkones & related compounds: Part II - Synthesis of fluorocoumaranones & fluoroflavones'**
- **CHEMICAL ABSTRACTS, vol. 113, no. 19, 5 November 1990, Columbus, Ohio, US; abstract no. 171775n, SUGAYA T. ET AL. 'Preparation of 5-aminoflavone derivatives as antitumor agents' page 682 ;column 1 ; & JP-A-02 138 277 (KYOWA HAKKO KOGYO CO., LTD.) 28 May 1990**
- **CHEMICAL ABSTRACTS, vol. 119, no. 1, 5 July 1993, Columbus, Ohio, US; abstract no. 8593s, SHIDA Y. ET AL. 'Preparation of 5-aminochromone derivatives as neoplasm inhibitors' page 882 ;column 2 ; & DATABASE WPI Week 9247, Derwent Publications Ltd., London, GB; AN 92-384929 & JP-A-04 282 380 (KYOWA HAKKO KOGYO CO., LTD.) 7 October 1992**
- **CHEMICAL ABSTRACTS, vol. 115, no. 21, 25 November 1991, Columbus, Ohio, US; abstract no. 228319q, SHAH K.G. ET AL. 'Phytochemical studies and antiestrogenic activity of Butea frondosa flowers' page 567 ;column 2 ; & INDIAN J. PHARM. SCI. vol. 52, no. 6 , 1990 pages 272 - 275**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7/3.6

## Description

The present invention relates to novel 5-aminoflavone derivatives possessing antibacterial activity, anti-estrogenic activity and antitumor activity.

Indian J. of Chem. (1963) 1, 477-479 discloses 6-fluoro-8-halo flavones having no amino groups in position 4' and 5 and lacking a substituent in position 7. EP-A-0 374 789 describes 4',5-diamino flavones bearing no fluoro in position 8 and no substituents in positions 6 and 7, which show anti-cellular and anti-tumor activity. In Chem. Abs. (1990) 113, 171775n, 5-amino-flavones with anti-tumor activity are described which bear no 4'-amino substituent and no substituent in position 6. In Chem. Abs. (1993) 119, 8593s and WPI abstract (1992) 92-384929, 5-amino-flavones bearing a 4' amino group as part of a 5-ring or 6-ring heterocycle are disclosed as cytotoxic agents. EP-A-0 556 720 published on August 25, 1993 comprises 4',5-diamino-6,8-dihaloflavones, lacking a substituent at position 7 which exhibit antibacterial and antitumor activity.

As other derivatives having an amino group at 5-position, there are disclosed compounds having a hydroxyl group at 6-position (Chem. Abst., 41, 120f (1947)), compounds having and an alkoxy group at 3-position with antiviral activity (EP-A-233105), compounds having anti-allergy activity and the like (GB-A-1461777) and compounds having a methyl group at 7-position [Arch. Pharm. (Weinheim), 322, 589 (1989)].

Further, anti-estrogenic activity is not known in the respective flavone derivatives described above.

The main object of the present invention is to provide novel 5-aminoflavone derivatives possessing antibacterial activity, anti-estrogenic activity and antitumor activity.

This object has been achieved by

5-aminoflavone derivatives represented by the formula (I):

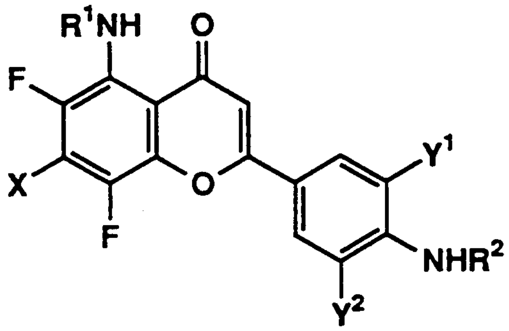

(I)

wherein $R^1$ and $R^2$ are the same or different and represent hydrogen, or substituted or unsubstituted lower alkyl, X represents substituted or unsubstituted lower alkyl, lower alkenyl, lower alkynyl, halogen, hydroxy, substituted or unsubstituted lower alkoxy, $NR^3R^4$ (wherein $R^3$ and $R^4$ are the same or different and represent hydrogen, or substituted or unsubstituted lower alkyl, or $R^3$ and $R^4$ are taken together to form a heterocyclic group containing the nitrogen atom in the ring), lower alkylthio, lower alkylsulfinyl, lower alkylsulfonyl, carboxy, lower alkoxycarbonyl, lower alkanoyl, azido, cyano, substituted or unsubstituted carbamoyl or lower alkylthiothiocarbonyl, $Y^1$ and $Y^2$ are the same or different and represent hydrogen, halogen or lower alkyl (hereinafter referred to as compound (I); compounds (Ia), (Ib) and the like mean to be included in compound (I)); or pharmaceutically acceptable salts thereof.

The 5-aminoflavone derivatives of the present invention have antibacterial activity, anti-estrogenic activity and antitumor activity.

In the definition of the respective groups in the formula (I), examples of the lower alkyl are straight or branched alkyls having 1 to 8 carbon atoms, for example, methyl, ethyl, propyl, isopropyl, butyl, isobutyl, sec-butyl, tert-butyl, pentyl, isopentyl, neopentyl, hexyl, isohexyl, heptyl, isoheptyl and octyl Examples of the lower alkenyl are straight or branched alkenyls having 2 to 6 carbon atoms, for example, vinyl, allyl, methacryl, crotyl, 3-butenyl, pentenyl and hexenyl. Examples of the lower alkynyl are alkynyls having 2 to 6 carbon atoms, for example, ethynyl, propynyl, butynyl, pentynyl and hexynyl. Halogen represents fluorine, chlorine, bromine and iodine. Examples of the heterocyclic group are pyrrolidinyl, piperidino, morpholino, substituted or unsubstituted piperazinyl. The substituted piperazinyl has the same or different 1 to 2 substituents, for example, lower alkyl. The lower alkyl is the same as defined above. Examples of the lower alkanoyl are straight or branched alkanoyls having 1 to 7 carbon atoms, for example, formyl, acetyl, propionyl, butanoyl, pentanoyl, pivaloyl, hexanoyl, and heptanoyl. The alkyl moiety of the lower alkoxy, lower alkylthio, lower alkylsulfinyl, lower alkylsulfonyl, lower alkoxycarbonyl and lower alkylthiothiocarbonyl is the same as defined for the above lower alkyl. The substituted lower alkyl and substituted lower alkoxy have the same or different 1 to 3 substituents, for example, halogen, hydroxy, lower alkoxy, lower alkanoyloxy, hydroxysulfonyloxy and $NR^5R^6$ (wherein $R^5$ and $R^6$ are the same as defined for the above $R^3$ and $R^4$). Halogen, and the alkyl moiety of the lower alkoxy and the lower alkanoyloxy are the same as defined above. The substituted carbamoyl has the same or different 1 to 2 substituents, for example, lower alkyl. The lower alkyl is the same as defined above.

As pharmaceutically acceptable salts of compound (I), there are pharmaceutically acceptable acid or base addition salts, for example, inorganic acid salts such as hydrochloride, hydrobromide, sulfate and phosphate and organic acid salts such as methanesulfonate, oxalate, acetate, malonate, succinate, fumarate, maleate, tartrate, citrate as well as base addition salts such as the sodium salt and the potassium salt.

Then, a process for producing compound (I) is explained below.

In addition, in the process described below, when the defined groups may be changed under the process conditions or are not suitable for the process, such inconvenience can be avoided by a method usually used in organic synthetic chemistry, for example, protection and deprotection of functional groups.

Preparation 1

Compound (Ia) which is compound (I) wherein $R^1$ and $R^2$ are hydrogen can be produced according to the following Scheme.

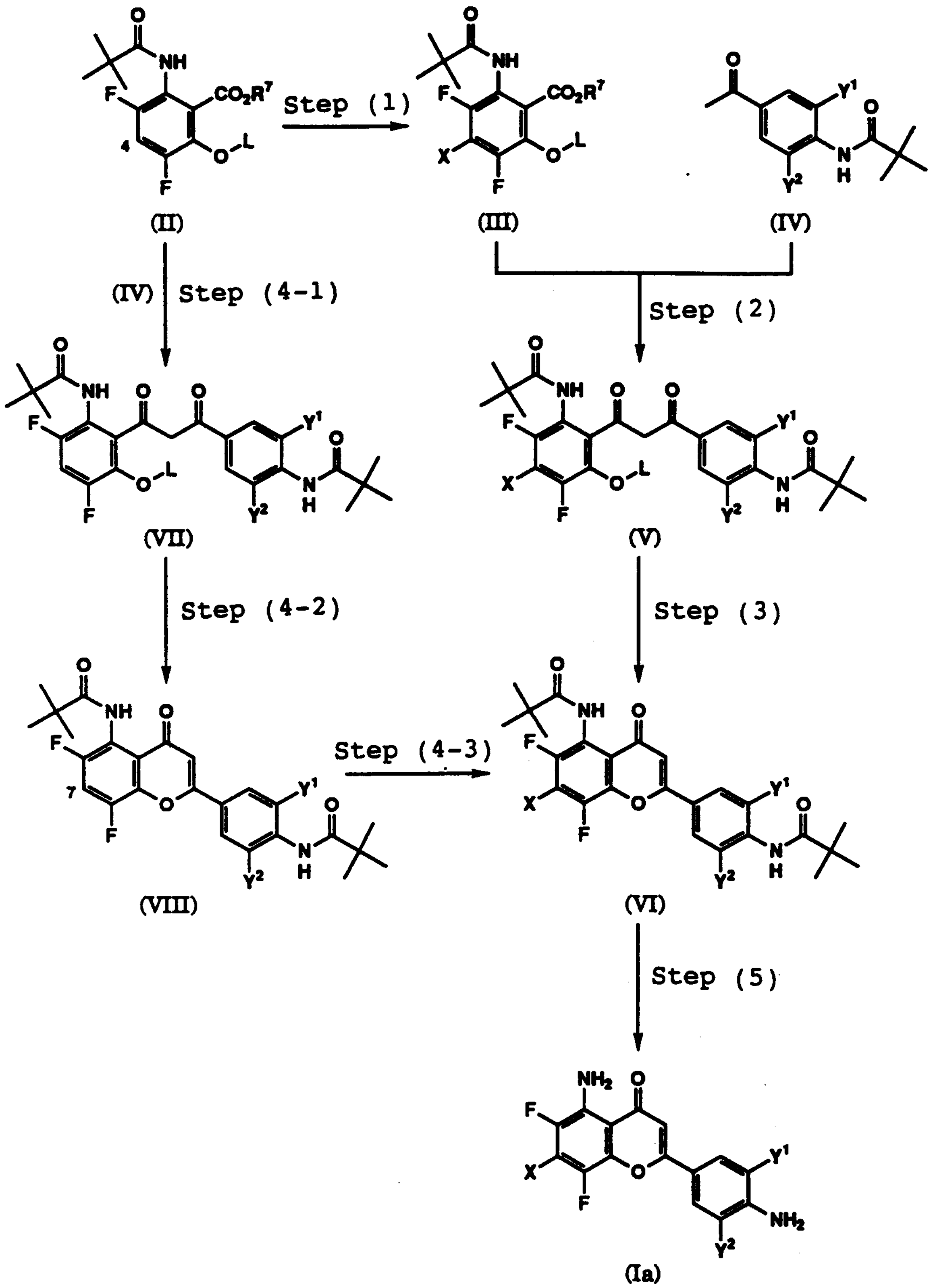


wherein $R^7$ represents lower alkyl, L represents a protecting group for hydroxy, and X, $Y^1$ and $Y^2$ are the same as defined above. The lower alkyl is the same as defined above. Examples of L are tetrahydropyranyl and methoxymethyl

Step (I)

Compound (III) is prepared by lithiating 4-position of compound (II) obtained according to the method described in

Reference Example 1 or modified method thereof, then reacting the lithiated compound with 1 to 10 equivalents of an electrophile. Examples of a base used in the lithiation are lithium diisopropylamide, sec-butyllithium and tert-butyllithium. Examples of a solvent used in the lithiation are tetrahydrofuran, ether and dimethoxyethane. Examples of the electrophile are alkyl halides such as iodomethane, iodoethane, bromoethane, 1-iodobutane, 1-iodohexane, N-chlorosuccinimide, N-bromosuccinimide, dialkyl disulfides such as dimethyl disulfide, 1,2-dibromoethane, bromine, trimethoxyborane, carbon dioxide, carbon disulfide, ethyl chloroformate, p-toluenesulfonyl azide, p-toluenesulfonyl cyanide, aldehydes such as acetaldehyde and, dimethylformamide. The reaction is carried out by lithiation at -78 to 0 °C, preferably at -60 °C or below for 0.5 to 3 hours, adding an electrophile and, if necessary, allowing to raise the temperature between 0 °C and room temperature. The reaction completes in 0.1 to 1 hours. When trimethoxyborane is used as an electrophile, a hydroxyl group can be introduced by later treatment with acetic acid and hydrogen peroxide. When dimethylformamide is used as an electrophile, a formyl group is introduced. This formyl group can be converted to a hydroxymethyl group using a reducing agent such as sodium borohydride in methanol or ethanol. When a hydroxy group is present as a substituent (such as a hydroxy group and hydroxymethyl group), the hydroxy group is desirably used in the next step after it is protected with a protecting group stable under the basic conditions, for example, a tetrahydropyranyl group, and trialkylsilyl group according to the conventional method. When carbon disulfide is used as an electrophile, iodomethane is added thereto to give a dithiocarbonic acid ester which can be converted to a trifluoromethyl group according to the method described in the literature [Chem. Lett., 827 (1992)].

Step (2)

Compound (V) is prepared by condensing compound (III) with compound (IV) obtained by the method described in Reference Examples 2 to 5 in the presence of 3 to 5 equivalents of a base in an inert solvent. Examples of the base are sodium hydride, potassium hydride, sodium methoxide, sodium ethoxide and potassium tert-butoxide. As the suitable inert solvent, ethers such as tetrahydrofuran, dioxane and the like, alcohols such as methanol and ethanol, toluene and dimethylformamide are used sole or in combination with each other. The reaction is carried out normally at an elevated temperature, preferably at 40 °C to the boiling point of the solvent employed. The reaction completes in 1 to 12 hours.

Step (3)

Compound (VI) is prepared by treating compound (V) with 1 to 6 N hydrochloric acid or sulfuric acid in a solvent such as an alcohol, for example, methanol and ethanol, an ether, for example, dioxane, tetrahydrofuran or acetic acid at 0 to 50 °C for 0.5 to 12 hours to deprotect a protected group L and cyclize the deprotected compound.

Step (4)

Alternatively, compound (VI) is prepared by the following method. Compound (VII) is prepared starting from compound (II) and compound (IV) according to Step (2). According to Step (3), compound (VII) is converted into compound (VIII) which is finally subjected to Step (1) to give compound (VI). When compound (VIII) has the low solubility in the solvent a compound such as hexamethylphosphoric triamide can be added to aid the dissolution.

Step (5)

Compound (Ia) is prepared by depivaloylation by reacting compound (VI) with an acid such as hydrochloric acid, hydrobromic acid and sulfuric acid in, if necessary, a lower alcohol such as methanol and ethanol, an ether such as dioxane, tetrahydrofuran, or acetic acid at room temperature to 100 °C or at the boiling point of the solvent employed for 0.1 to 10 hours.

At this stage, a part of compounds (VI) obtained in Step (3) or Step (4) may be modified at the substituent X and thereafter subjected to Step (5) to give a new derivative (Ia).

For example, compound (VIa) which is compound (VI) wherein X is an amino group is prepared by reacting compound (VIb) which is compound (VI) wherein X is azido with 1 to 10 equivalents of triphenylphosphine in an inert solvent such as tetrahydrofuran, ethyl acetate and acetonitrile at 0 °C to the boiling point of the solvent for 1 to 12 hours, and then adding dilute hydrochloric acid thereto to react them at room temperature for 1 to 12 hours, or reacting compound (VIb) with 1 to 10 equivalents of sodium borohydride and water in a lower alcohol such as methanol and ethanol.

Compound (VIc) which is compound (VI) wherein X is dialkylamino is prepared by reacting compound (VIa) and an equivalent amount to an excess amount of alkyl halide such as iodomethane, iodoethane, bromoethane and chloroethane in the presence of an excess amount of a base in an inert solvent. Examples of the base are sodium hydride, potassium hydride, sodium carbonated and potassium carbonate. Examples of the inert solvent are dimethylformamide, dimethyl sulfoxide, tetrahydrofuran, diethyl ether, dioxane and acetone.

Compound (VId) which is compound (VI) wherein X is monoalkyl (acetyl) amino is prepared by reacting an amino group of compound (VIa) with acetic anhydride in pyridine, if necessary, in the presence of a catalytic amount of N,N-dimethylaminopyridine to give an acetamide, which is alkylated under the same conditions as those for obtaining compound (VIc). An acetyl group is hydrolyzed in the next Step (5).

Compound (VIe) which is compound (VI) wherein X is a cyclic amino group such as piperazinyl is prepared by reacting compound (VIf) which is compound (VI) wherein X is bromine with an excess amount of corresponding cyclic amine in an inert solvent such as dimethyl sulfoxide, and dimethylformamide at room temperature to the boiling point of the solvent employed, preferably at 80 to 120 °C, for 1 to 24 hours.

Compound (VIg) which is compound (VI) wherein X is substituted or unsubstituted lower alkoxy is prepared by alkylating compound (VIh) which is compound (VI) wherein X is hydroxy under the same conditions as those for obtaining compound (VIc).

Compound (VII) which is compound (VI) wherein X is lower alkylsulfinyl or lower alkylsulfonyl is prepared by treating compound (VIj) which is compound (VI) wherein X is lower alkylthio with an equivalent amount to an excess amount of an oxidizing agent in an inert solvent. As the inert solvent, a halogenated solvent such as dichloromethane and 1,2-dichloroethane are preferably used. As the oxidizing agent, m-chloroperbenzoic acid is preferably used. The reaction is carried out at 0 °C to room temperature and completes in 0.5 to 12 hours.

Compound (VIk) which is compound (VI) wherein X is lower alkanoyl is prepared by treating compound (VIm) which is compound (VI) wherein X is corresponding 1-hydroxyalkyl with an equivalent amount to an excess amount of an oxidizing agent in an inert solvent. As the inert solvent, an aromatic hydrocarbon such as toluene, benzene, a halogenated solvent such as dichloromethane, and 1,2-dichloroethane are used. As the oxidizing agent, manganese dioxide is preferably used. The reaction is carried out at room temperature to the boiling point of the solvent employed and completes in 0.5 to 24 hours.

Compound (VIn) which is compound (VI) wherein X is lower alkynyl is prepared by reacting compound (VIf) with an acetylene derivative. For example, compound (VIn) wherein X is ethynyl is prepared by coupling compound (VIf) with trimethylsilylacetylene in the presence of a base in an inert solvent using 0.01 to 0.3 equivalents of a palladium catalyst. As the inert solvent, solvents such as dimethylformamide, and toluene are used. As the base, bases such as triethylamine and diethylamine are used. In some cases, the base is also used as the solvent. Examples of the palladium catalyst are palladium catalysts having a ligand such as triphenylphosphine. If necessary, palladium catalysts are used after ligand exchange in the reaction system. The reaction is carried out at room temperature to the boiling point of the solvent employed and completes in 0.5 to 24 hours.

Compound (VIo) which is compound (VI) wherein X is substituted or unsubstituted aminoalkyl is prepared by converting a hydroxy group of compound (VIp) which is compound (VI) wherein X is hydroxyalkyl to a leaving group, followed by substitution with various amines. For example, compound (VIp) is first reacted with 1 to 10 equivalents of methanesulfonyl chloride or p-toluenesulfonyl chloride in the presence of a base in an inert solvent to give a corresponding sulfonate or chlorinated compound. Examples of the inert solvent, are dimethylformamide, and dichloromethane. Examples of the base are triethylamine and pyridine. In some cases, the base is also used as the solvent. The reaction is carried out at 0 °C to room temperature and completes in 0.1 to 2 hours. The resulting sulfonate or chlorinated compound may be reacted with various amines in an inert solvent, if necessary, in the presence of a base to give compound (VIo). Examples of the inert solvent are dimethylformamide, dimethyl sulfoxide and dichloromethane. Examples of the base are triethylamine, potassium carbonate, sodium carbonate The amine may be used at an equivalent amount to a largely excess amount, if necessary, in the form of a solution or salt. The reaction is carried out at 0 °C to the boiling point of the solvent employed, preferably at 0 °C to room temperature, and completes in 0.1 to 24 hours.

Compound (VIq) which is compound (VI) wherein X is lower alkoxyalkyl is prepared by reacting a sulfonate or chlorinated compound of the above compound (VIp) with a lower alcohol which is also used as a solvent, such as methanol, and ethanol The reaction is carried out at room temperature to the boiling point of the solvent, preferably at 50 to 100 °C, and completes in 1 to 48 hours.

A part of the resulting compound (Ia) may be used as a synthetic intermediate to give a new derivative (Ia).

Compound (Iaa) which is compound (Ia) wherein X is carboxy is prepared by treating compound (Iab) which is compound (Ia) wherein X is alkoxycarbonyl with an aqueous alkali solution in a solvent. As the solvent, inert water-miscible solvents, for example, a lower alcohol such as methanol, ethanol, dioxane and tetrahydrofuran are used sole or in combination with each other. An example of the aqueous alkali solution is a 1 to 10 N aqueous solution of sodium hydroxide, and potassium hydroxide The reaction is carried out at 0 °C to the boiling point of the solvent employed, preferably at 40 to 60 °C and completes in 0.5 to 12 hours.

Compound (Iac) which is compound (Ia) wherein X is carbamoyl is prepared by reacting compound (Iab) with ammonia in a lower alcohol such as methanol and ethanol. The reaction is carried out at room temperature or, if necessary, under heating in a sealed tube and completes in 1 to 7 days.

Compound (Iad) which is compound (Ia) wherein X is cyano is prepared by dehydrating compound (Iac) with a compound such as phosphorus oxychloride, thionyl chloride, if necessary, in an inert solvent at 0 °C to the boiling point

of the solvent employed, preferably at room temperature. Examples of the inert solvent are dimethylformamide and dichloromethane, pyridine.

Compound (Iae) which is compound (Ia) wherein X is lower alkanoyloxyalkyl is prepared by heating compound (Iaf) which is compound (Ia) wherein X is hydroxyalkyl in a solvent of a lower fatty acid corresponding to a lower alkanoyl moiety in the presence of concentrated sulfuric acid. Concentrated sulfuric acid is preferably used at a concentration of 1 to 18 N in a solvent. The reaction is carried out at room temperature to the boiling point of the solvent employed, preferably at 50 to 100 °C and completes in 0.1 to 10 hours. Alternatively, compound (VIp) having a protected group may be used as a starting compound. In this case, depivaloylation and esterification proceed simultaneously under the same conditions to give compound (Iae).

Compound (Iag) which is compound (Ia) wherein X is hydroxysulfonyloxyalkyl is prepared by treating compound (Iaf) with concentrated sulfuric acid at 50 to 100 °C for 0.1 to 10 hours. Compound (VIp) having a protected group may be used as a starting compound as described above. In this case, depivaloylation and esterification proceed simultaneously under the same conditions to give compound (Iag).

Compound (Iah) which is compound (Ia) wherein X is formyl is prepared by treating compound (Iaf-I) which is compound (Iaf) wherein X is hydroxymethyl with 10 to 50 equivalents of an oxidizing agent in an inert solvent. Examples of the inert solvent are an aromatic hydrocarbon such as toluene, benzene, halogenated solvent such as dichloromethane, 1,2-dichloroethane, and ethyl acetate. As the oxidizing agent, manganese dioxide is preferably used. The reaction is carried out at room temperature to the boiling point of the solvent employed, preferably at the boiling point of the solvent, and completes in 1 to 48 hours.

Preparation 2

Compounds (Ib) to (Id) which fall within compound (I) wherein $R^1$ and/or $R^2$ is substituted or unsubstituted lower alkyl are prepared using compound (Ia) as a starting compound according to the following Scheme.

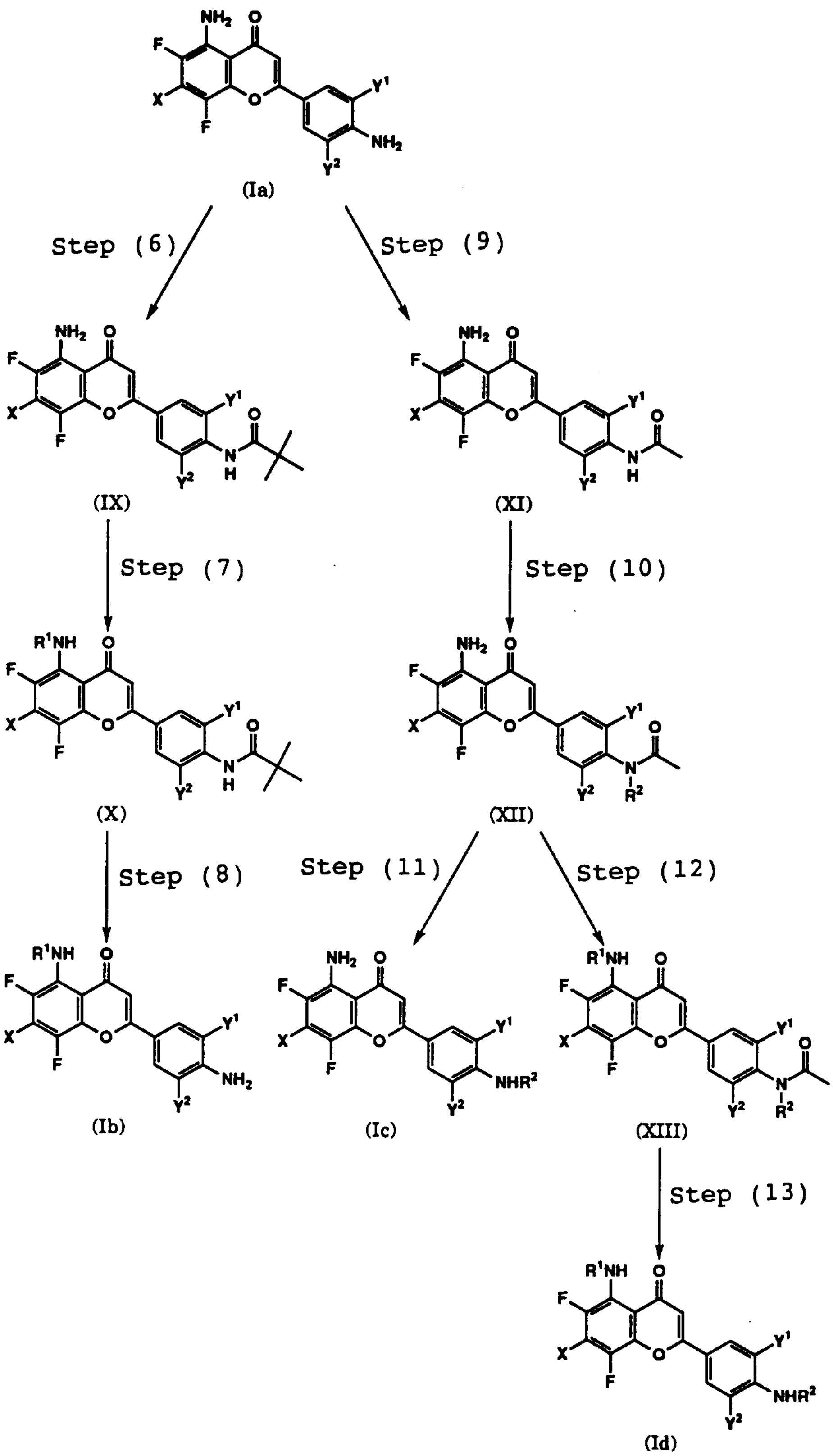


(Ia) (IX) (XI) (X) (XII) (Ib) (Ic) (XIII) (Id)

wherein $R^1$, $R^2$, X, $Y^1$ and $Y^2$ are the same as defined above.

Preparation of compound (Ib) which is compound (I) wherein $R^1$ is substituted or unsubstituted lower alkyl and $R^2$ is hydrogen:

Step (6)

Compound (IX) is prepared by reacting compound (Ia) with an equivalent amount to a slightly excess amount of pivaloyl chloride in an inert solvent in the presence of a base. Examples of the inert solvent are dichloromethane, 1,2-dichloroethane, dimethylformamide and tetrahydrofuran Examples of the base are pyridine, triethylamine and diisopropylethylamine. In some cases, the base is used also as the solvent. The reaction is carried out at 0 °C to room temperature and completes in 0.1 to 12 hours.

Step (7)

Compound (X) is prepared by reacting compound (IX) with 1 to 10 equivalents of an alkyl halide such as iodomethane, iodoethane, bromoethane, chloroethane, 1-iodopropane, 1-iodobutane, 1-iodopentane, 1-iodohexane and 1-iodoheptane in an inert solvent in the presence of an equivalent amount to an excess amount of a base. Examples of the base are sodium hydride, potassium hydride, sodium carbonate and potassium carbonate. Examples of the inert solvent are dimethylformamide, dimethyl sulfoxide, tetrahydrofuran and dioxane. The reaction is carried out at 0 °C to the boiling point of the solvent employed and completes in 1 to 12 hours.

Step (8)

Compound (Ib) is prepared by depivaloylating compound (X) under the same conditions as those in Step (5).

Preparation of compound (Ic) which is compound (I) wherein $R^1$ is hydrogen and $R^2$ is substituted or unsubstituted lower alkyl:

Step (9)

Compound (XI) is prepared by reacting compound (Ia) with an equivalent amount to a slightly excess amount of an acetylating agent in an inert solvent in the presence of a base. As the acetylating agent, acetic anhydride, acetyl chloride are preferably used. Examples of the inert solvent are dichloromethane, 1,2-dichloroethane, dimethylformamide, tetrahydrofuran, methanol and ethanol. Examples of the base are pyridine, triethylamine, diisopropylethylamine. In some cases, the base is used also as a solvent. The reaction is carried out at 0 to 50 °C and completes in 1 to 48 hours.

Step (10)

Compound (XII) is prepared by reacting compound (XI) with 1 to 10 equivalents of an alkyl halide such as iodomethane, iodoethane, bromoethane, chloroethane, 1-iodopropane, 1-iodobutane, 1-iodopentane, 1-iodohexane, 1-iodoheptane in an inert solvent in the presence of an equivalent amount to a slightly excess amount of a base. Examples of the base are sodium hydride, potassium hydride, sodium carbonate and potassium carbonate Examples of the inert solvent are dimethylformamide, dimethyl sulfoxide, tetrahydrofuran and dioxane. The reaction is carried out at 0 °C to the boiling point of the solvent employed and completes in 1 to 12 hours.

Step (11)

Compound (Ic) is prepared by deacetylating compound (XII) under the same conditions as those in step (5).

Preparation of compound (Id) which is compound (I) wherein $R^1$ and $R^2$ are substituted or unsubstituted lower alkyl:

Step (12)

Compound (XIII) is prepared by alkylating compound (XII) under the same conditions as those in Step (7).

Step (13)

Compound (Id) is prepared by deacetylating compound (XIII) under the same conditions as those in Step (5).

Preparation 3

Compound (Iai) which is compound (Ia) wherein $R^1$ and $R^2$ are hydrogen and X is fluorine is prepared according to the following Scheme.

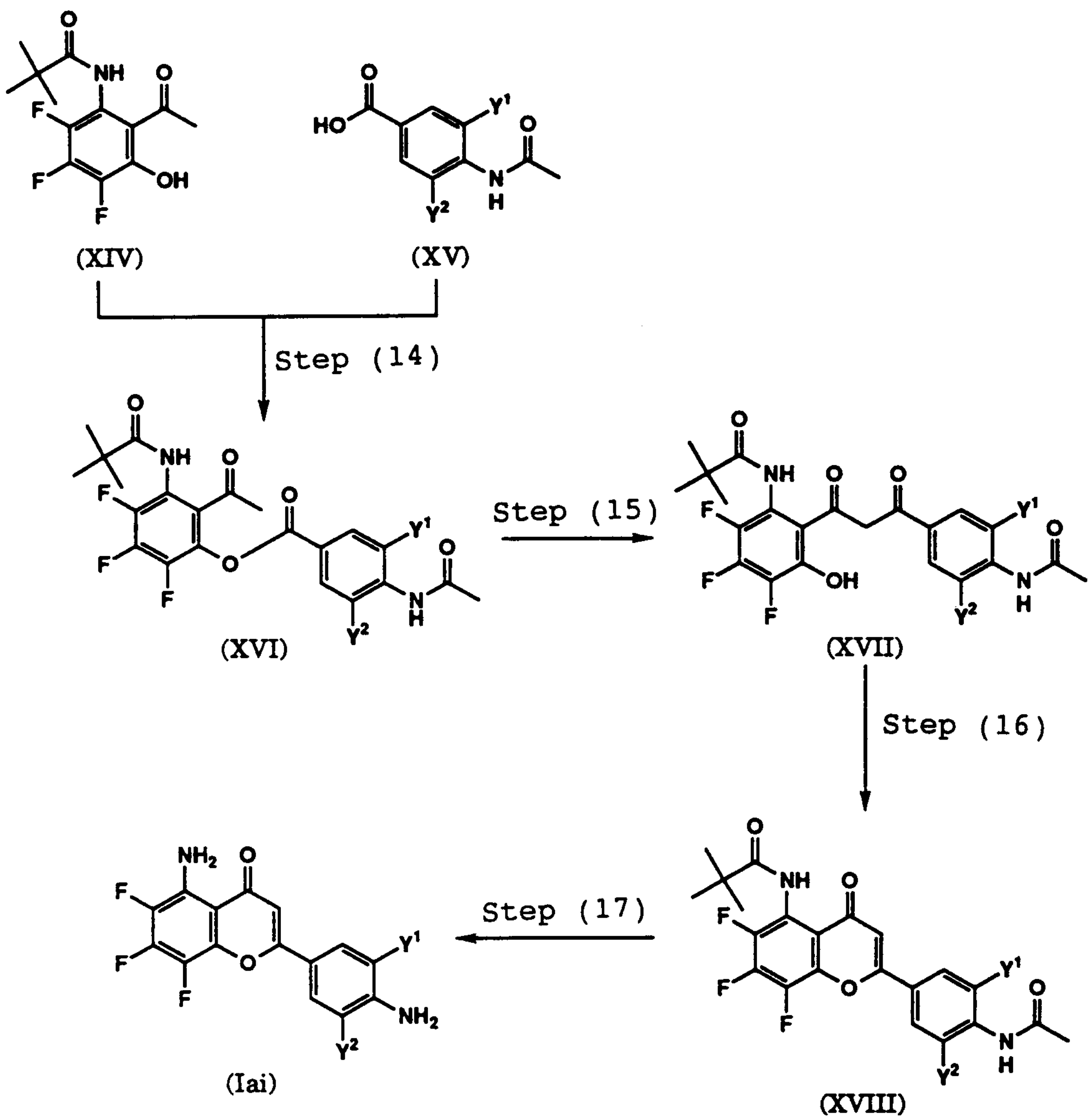


(XIV) (XV) (XVI) (XVII) (XVIII) (Iai)

wherein $Y^1$ and $Y^2$ are the same as defined above.

Step (14)

Compound (XVI) is prepared by condensing compound (XIV) obtained by the method described in Reference Example 7 with 1 to 5 equivalents of a reactive derivative, for example, acid chloride or acid anhydride of compound (XV) obtained by the method described in Reference Example 8 in an inert solvent in the presence of 1 to 5 equivalents of a base, or by reacting compound (XIV) with 1 to 5 equivalents of compound (XV) in an inert solvent in the presence of a suitable condensing agent. Examples of the base are sodium hydride, potassium hydride, triethylamine and pyridine. Examples of the inert solvent are tetrahydrofuran, dioxane, dimethylformamide, dichloromethane and pyridine used sole or in combination with each other. Examples of the condensing agent are trifluoroacetic anhydride, dicyclohexylcarbodiimide, 2-chloro-1-methylpyridinium iodide. The reaction is carried out normally at 0 to 30 °C and completes in 0.5 to 10 hours.

Step (15)

Compound (XVII) is prepared by subjecting compound (XVI) to the rearrangement reaction in an inert solvent in the presence of a base. Examples of the base are sodium hydride, potassium hydride and potassium tert-butoxide. Examples of the inert solvent are tetrahydrofuran, dimethyl sulfoxide, dioxane and diethyl ether. The reaction is carried out normally at 0 to 30 °C and completes in 1 to 10 hours.

Step (16)

Compound (XVIII) is prepared by cyclizing compound (XVII) under the same conditions as those in Step (3).

Step (17)

Compound (Iai) is prepared by deprotecting compound (XVIII) under the same conditions as those in Step (5).

Compound (I) having desired functional groups at desired positions can be obtained by appropriately combining the above-described processes with each other.

Intermediates and final compounds in the above processes can be isolated and purified by purifying methods normally used in organic synthetic chemistry, for example, filtration, extraction, washing, drying, concentration, recrystallization, and various chromatographies. In addition, intermediates may be subjected to the subsequent step without purification.

When the product is synthesized in the salted form, the salted compound can be subjected to known purification or isolation processes to give the salted product. If the product is synthesized in the unsalted form, to obtain a salt for compound (I), it can be converted into a salt by, for example, dissolving or suspending it in an appropriate organic solvent and adding an appropriate acid or base.

Compound (I) and salts thereof can also be present in the form of addition products to water or various solvents. Such addition products are also included within the scope of the present invention.

Particular compounds (I) of the present invention are shown in Table 1.

Table 1

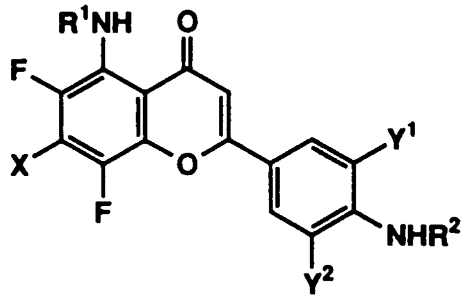

| Compound No. | Example No. | $R^1$ | $R^2$ | X | $Y^1$ | $Y^2$ |
|---|---|---|---|---|---|---|
| 1 | 1 | H | H | $CH_3$ | F | H |
| 2 | 2 | H | H | $CH_2CH_3$ | F | H |
| 3 | 3 | H | H | $(CH_2)_3CH_3$ | F | H |
| 4 | 4 | H | H | $(CH_2)_5CH_3$ | F | H |
| 5 | 12 | H | H | Cl | F | H |
| 6 | 5 | H | H | Br | F | H |
| 7 | 7 | H | H | OH | F | H |
| 8 | 16 | H | H | $OCH_3$ | F | H |
| 9 | 17 | H | H | $O(CH_2)_2N(CH_3)_2$ | F | H |
| 10 | 6 | H | H | $SCH_3$ | F | H |
| 11 | 18 | H | H | $SOCH_3$ | F | H |
| 12 | 19 | H | H | $SO_2CH_3$ | F | H |
| 13 | 13 | H | H | $CO_2CH_2CH_3$ | F | H |
| 14 | 20 | H | H | $CO_2H$ | F | H |
| 15 | 14 | H | H | $N_3$ | F | H |
| 16 | 21 | H | H | $NH_2$ | F | H |
| 17 | 22 | H | H | $N(CH_3)_2$ | F | H |
| 18 | 23 | H | H | $NH(CH_2)_3N(CH_3)_2$ | F | H |
| 19 | 24 | H | H | 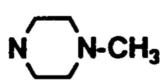 | F | H |
| 20 | 15 | H | H | $CH(OH)CH_3$ | F | H |

Table 1 (continued)

| Compound No. | Example No. | $R^1$ | $R^2$ | X | $Y^1$ | $Y^2$ |
|---|---|---|---|---|---|---|
| 21 | 25 | H | H | $CH{=}CH_2$ | F | H |
| 22 | 26 | H | H | $COCH_3$ | F | H |
| 23 | 27 | $CH_3$ | H | $N(CH_3)_2$ | F | H |
| 24 | 8 | H | H | $CH_3$ | Cl | H |
| 25 | 9 | H | H | $CH_3$ | Cl | Cl |
| 26 | 10 | H | H | $CH_3$ | $CH_2CH_3$ | H |
| 27 | 11 | H | H | $NH_2$ | Cl | Cl |
| 28 | 28 | $(CH_2)_5CH_3$ | H | $CH_3$ | F | H |
| 29 | 29 | $(CH_2)_6CH_3$ | H | $CH_3$ | F | H |
| 30 | 30 | $(CH_2)_4CH_3$ | $(CH_2)_2CH_3$ | $CH_3$ | F | H |
| 31 | 31 | $(CH_2)_4CH_3$ | $(CH_2)_3CH_3$ | $CH_3$ | F | H |
| 32 | 32 | $(CH_2)_4CH_3$ | $(CH_2)_4CH_3$ | $CH_3$ | F | H |
| 33 | 33 | H | H | $C{\equiv}CH$ | F | H |
| 34 | 34 | H | H | F | F | H |
| 35 | 35 | $(CH_2)_2CH(CH_3)_2$ | H | $CH_3$ | F | H |
| 36 | 36 | $(CH_2)_3CH(CH_3)_2$ | H | $CH_3$ | F | H |
| 37 | 37 | $(CH_2)_3N(CH_3)_2$ | H | $CH_3$ | F | H |
| 38 | 38 | $(CH_2)_3N(CH_2CH_3)_2$ | H | $CH_3$ | F | H |
| 39 | 39 | H | H | $CH_2OH$ | F | H |
| 40 | 40 | H | H | $CH_2OSO_3H$ | F | H |
| 41 | 41 | H | H | $CH_2NH_2$ | F | H |
| 42 | 42 | H | H | $CH_2N(CH_3)_2$ | F | H |
| 43 | 43 | H | H | $CH_2OCH_3$ | F | H |
| 44 | 44 | H | H | $CH_2OCOCH_3$ | F | H |
| 45 | 45 | H | H | $CH_2OCOCH_2CH_3$ | F | H |
| 46 | 46 | H | H | $CH_2OCO(CH_2)_4CH_3$ | F | H |

Anti-estrogenic activity of compound (I) can be demonstrated by the antagonism against the increase in uterus weight of immature mice treated with estradiol. Oral administration of compound (I) significantly inhibits the increase in the uterus weight observed in an estradiol-treated group, this confirming the anti-estrogenic activity. On the other hand,

a known anti-estrogenic agent, tamoxifen, has the weak anti-estrogenic activity and this is due to its partial agonist action. Tamoxifen rather significantly increases the weight of the uterus in an estradiol-untreated group. Compound (I) is an anti-estrogenic agent possessing the uterus weight-decreasing activity even in an estradiol-untreated group. In addition, compound (I) inhibits the growth of human mammary cancer cells in the medium in a microplate and inhibits the growth of human mammary cancers transplanted to a nude mouse.

The compound possessing such the bioactivity is useful not only for treatment of the same symptoms as those for which tamoxifen is useful, for example, mammary cancer, non-ovulatory sterility or paramenia but also for treatment of the symptoms for which tamoxifen is not useful, for example, endometriosis or endometrial cancer.

Then, the activity of the representative compounds (I) is shown by experiments.

Test 1

The effect against the increase in uterus weight of immature mice

Immature female BALB/c mice, 4 weeks age, were divided into two groups and 50 µg of estradiol was subcutaneously administered to the animals of one group in the chest. The test compounds were repeatedly administered orally to each group (8 animals per group) for three days since the date of estradiol administration. After four days, the uteri were isolated and the weight thereof was measured.

The results are shown in Table 2. In Table 2, "estradiol (-)" represents an estradiol-untreated group and "estradiol (+)" represents an estradiol-treated group.

Table 2

| Compound No. | Dose (mg/kg) | Estradiol | Weight of uterus (mg) |
|---|---|---|---|
| Untreated | - | - | 24 |
| 1 | 50 | - | 17 |
| Tamoxifen | 5 | - | 49 |
| Untreated | - | + | 49 |
| 1 | 50 | + | 38 |
| Tamoxifen | 5 | + | 47 |

Test 2

Human mammary cancer MCF-7 cell growth inhibition test

Each 0.1 ml of MCF-7 cells which had been prepared to 5 x $10^4$/ml using a medium (referred to as Medium B hereinafter) prepared by adding 10% bovine fetal serum, $10^{-8}$M estradiol (manufactured by Sigma), 100 units/ml penicillin and 100 µg/ml streptomycin to RPMI1640 medium was distributed in each well of 96 well-microtiter plate. The plate was cultured at 37 °C for 20 hours in a $CO_2$ gas incubator, each 0.05 ml of samples (test compound) which had been appropriately diluted with Medium B was added thereto and the mixture was cultured at 37 °C for 72 hours in a $CO_2$ gas incubator. Supernatant was removed, each 0.1 ml of Medium B containing 0.02% neutral red was added to the residue, the mixture was cultured at 37 °C for 1 hour in a $CO_2$ gas incubator and the cells were stained by neutral red dye. Supernatant was removed and the residue was washed once with a physiological saline. Then, the dye was extracted with 0.001N hydrochloric acid/30% ethanol and the absorbance at 550 nm was determined with a microplatereader. Sample concentration at which the growth of cell is inhibited by 50% ($IC_{50}$) was calculated by comparing the absorbance of untreated cells and sample-treated cells.

The results are shown in Table 3.

Table 3

| Compound No. | $IC_{50}$ (µM) |
|---|---|
| 1 | 0.013 |
| 2 | 0.13 |

Table 3 (continued)

| Compound No. | $IC_{50}$ (μM) |
|---|---|
| 5 | 0.21 |
| 6 | 0.16 |
| 8 | 0.10 |
| 9 | 1.4 |
| 10 | 0.19 |
| 11 | 2.5 |
| 13 | 1.4 |
| 16 | 0.0077 |
| 17 | 0.039 |
| 24 | 0.092 |
| 26 | 0.065 |
| 34 | 0.22 |
| 39 | 0.062 |
| 41 | 0.026 |
| 42 | 0.072 |
| 43 | 0.046 |
| 44 | 0.026 |
| Tamoxifen | 23 |

Test 3

Antitumor effects against estrogen-dependent human mammary cancer MCF-7

The tumor fragment (2mm x 2mm x 2mm) of human hormone dependent mammary cancer MCF-7 was transplanted subcutaneously in the flank of female BALB/c-nu/nu mice (Nihon Crea), 7 to 9 weeks age. For promoting the growth of tumor, 12.5 μg of estradiol propionate was intramuscularlly administered in the femoral region total two times, on the date of transplantation and two weeks after transplantation. 3 to 4 Weeks after transplantation, mice of the tumor volume of 25 to 200 $mm^3$ were selected, and the test compounds were orally administered repeatedly to the groups (5 animals per group) for 5 days per a week, for total two weeks. In addition, estradiol propionate was administered again on the date of initial administration of the test compounds. Length and width of the tumor were determined every day, and the tumor volumes were calculated by means of ellipsoid approximation according to the following equation:

$$\text{Tumor volume } (mm^3)=\{\text{Length (mm) x [Width (mm)]}^2\}/2$$

The tumor volume at initial administration ($V_o$) and on the day of judgement (V) was calculated, and the tumor growth rate ($V/V_o$) was calculated. T/C value was obtained as the ratio of $V/V_o$ value of treated group relative to that of control group.

The results are shown in Table 4.

Table 4

| Compound No. | Dose (mg/kg) | T/C | Judgement Date (Day) |
|---|---|---|---|
| 1 | 50 | 0.064 | 18 |
| 6 | 50 | 0.54 | 21 |
| 9 | 50 | 0.42 | 15 |

Table 4 (continued)

| Compound No. | Dose (mg/kg) | T/C | Judgement Date (Day) |
|---|---|---|---|
| 16 | 12.5 | 0.18 | 22 |
| 39 | 50 | 0.016 | 14 |
| 41 | 50 | 0.011 | 18 |
| 44 | 25 | 0.061 | 14 |
| Tamoxifen | 5 | 0.25 | 22 |

Test 4

Antitumor effects against estrogen-dependent human mammary cancer Br-10

The tumor fragment (2mm x 2mm x 2mm) of human hormone dependent mammary cancer Br-10 was transplanted subcutaneously in the flank of female BALB/c-nu/nu mice (Nihon Crea), 7 to 9 weeks age. For promoting the growth of tumor, 12.5 μg of estradiol propionate was intramuscularlly administered in the femoral region total two times, on the date of transplantation and two weeks after transplantation. 3 to 4 Weeks after transplantation, mice the tumor volume of which increased to 30 to 150 $mm^3$ were selected, and the test compounds were orally administered repeatedly to the groups (5 animals per group) for 5 days per a week, for total two to four weeks. Estradiol propionate was additionally administered on the date of initial administration of the test compounds and 14 days after the administration. Length and breadth of the tumor were determined every day, and the tumor volumes were calculated by means of ellipsoid approximation according to the above equation. The tumor volume at initial administration ($V_o$) and on the day of judgement (V) was calculated, and the tumor growth rate ($V/V_o$) was calculated. T/C value was obtained as the ratio of $V/V_o$ value of treated group relative to that of control group.

The results are shown in Table 5.

Table 5

| Compound No. | Dose (mg/kg) | T/C | Judgement Date (Day) |
|---|---|---|---|
| 1 | 25 | 0.12 | 18 |
| 16 | 12.5 | 0.24 | 18 |
| 17 | 25 | 0.22 | 18 |
| Tamoxifen | 5 | 0.58 | 25 |

Test 5

Antibacterial activity

Antibacterial activity of compound (I) against Bacillus subtilis #10107 [Minimum Inhibition Concentration(MIC; μg/ml)] is shown in Table 6. Minimum Inhibition Concentration was determined by agar dilution method at pH 7.0.

Table 6

| Compound No. | MIC (μg/ml) |
|---|---|
| 9 | 52.1 |
| 18 | 52.1 |
| 22 | 13.0 |
| 37 | 13.0 |
| 38 | 13.0 |
| 41 | 3.26 |

The following Examples and Reference Examples further illustrate the present invention in detail.

Physicochemical data on respective compounds in the following Examples and Reference Examples were determined using the apparatus:

$^1H$ NMR JEOL JNM-GX270 (270MHz)
JEOL JNM-EX270 (270MHz)
HITACHI R-90H (90MHz)
MS JEOL JMS-D300
JEOL JMS-SX102
SHIMAZU QP-1000

Example 1

5-Amino-2-(4-amino-3-fluorophenyl)-6,8-difluoro-7-methyl-4H-1-benzopyran-4-one (Compound 1)

(1) 70.0 mL (500 mmol) of diisopropylamine was dissolved in 140 mL of tetrahydrofuran under argon atmosphere and 288 mL of a 1.6 M solution of 460 mmol of n-butyl lithium in n-hexane was added dropwise while keeping an internal temperature at -10 to 0 °C. The reaction solution was cooled to -60 °C or below (internal temperature) and a solution of 77.0 g (200 mmol) of compound II-1 obtained in Reference Example 1 in 600 mL of tetrahydrofuran was added dropwise. The mixture was stirred at the same temperature for 2 hours to lithiate 4-position. 19 mL (0.30 mol) of iodomethane was added thereto and the mixture was further stirred for 30 minutes. Water was added to the reaction solution, the temperature of the solution was raised to room temperature and the solution was extracted once with ethyl acetate. The organic layer was washed once with an aqueous saturated solution of sodium chloride and dried over anhydrous sodium sulfate. The solvent was distilled off under reduced pressure and the residue was triturated with n-hexane to give 75.9 g of ethyl 3,5-difluoro-4-methyl-6-pivaloylamino-2-(2-tetrahydropyranyloxy)benzoate (yield: 95%).

NMR (90MHz, $CDCl_3$) δ (ppm) 1.19 (s, 9H), 1.37 (t, 3H, J=7.0Hz), 1.4-2.0 (m,6H), 2.23 (t,3H,J=2.2Hz), 3.4-4.2 (m, 2H), 4.34 (q, 2H, J=7.0Hz), 5.28 (brs, 1H), 7.69 (brs, 1H)
FAB-MS (M/Z) 400 ($M^+$+H)
Molecular formula $C_{20}H_{27}F_2NO_5$=399

(2) 430 mg (10.8 mmol) of sodium hydride (60% oil dispersion) was suspended in a mixed solvent of 2 mL of toluene and 2 mL of 1,4-dioxane under argon atmosphere, a solution obtained by dissolving 1.51 g (3.78 mmol) of the above ethyl 3,5-difluoro-4-methyl-6-pivaloylamino-2-(2-tetrahydropyranyloxy)benzoate and 750 mg (3.15 mmol) of compound IV-1 obtained in Reference Example 2 in a mixed solvent of 10 mL of toluene and 10 mL of 1,4-dioxane was added thereto dropwise while heating at reflux and the mixture was further stirred for 50 minutes. The reaction solution was cooled on ice, water was added and the mixture was extracted once with ethyl acetate. The organic layer was washed once with an aqueous saturated solution of sodium chloride, dried over anhydrous sodium sulfate and the solvent was distilled off under reduced pressure. The residue was dissolved in 40 mL of ethanol, 10 mL of concentrated sulfuric acid was added thereto and the mixture was stirred at room temperature for 12 hours. Water was added to the reaction solution and the precipitated crystals were collected by filtration to give 834 mg of 6,8-difluoro-2-(3-fluoro-4-pivaloylaminophenyl)-7-methyl-5-pivaloylamino-4H-1-benzopyran-4-one (yield: 54%).

NMR (90MHz, $CDCl_3$) δ (ppm) 1.36 (s, 9H), 1.38 (s, 9H), 2.41 (t, 3H, J=2.2Hz), 6.64 (s, 1H), 7.5-7.9 (m, 3H), 8.59 (t, 1H, J=8.4Hz), 10.5 (brs, 1H)
EIMS (M/Z) 488 ($M^+$)
Molecular formula $C_{26}H_{27}F_3N_2O_4$=488

(3) 50 mL of 1,4-dioxane and 25 mL of concentrated hydrochloric acid were added to 800 mg (1.64 mmol) of the above 6,8-difluoro-2-(3-fluoro-4-pivaloylaminophenyl)-7-methyl-5-pivaloylamino-4H-1-benzopyran-4-one and the mixture was stirred under heating at reflux for 2 hours. The reaction solution was poured into ice water, and the solution was made basic and extracted once with chloroform. The organic layer was washed once with an aqueous saturated solution of sodium chloride and dried over anhydrous sodium sulfate. The solvent was distilled off under reduced pressure, and the residue was purified by silica gel column chromatography (chloroform:methanol=40:1) and recrystallized from ethyl acetate/n-hexane to give 183 mg of Compound 1 (yield: 35%).

NMR (270MHz, $CDCl_3$) δ (ppm) 2.33 (t, 3H, J=2.0Hz), 4.17 (brs, 2H), 6.12 (brs, 2H), 6.45 (s, 1H), 6.84 (t, 1H,

J=8.4Hz), 7.5-7.6 (m, 2H)
EIMS (M/Z) 320 ($M^+$)
Molecular formula $C_{16}H_{11}F_3N_2O_2$=320

Example 2

5-Amino-2-(4-amino-3-fluorophenyl)-7-ethyl-6,8-difluoro-4H-1-benzopyran-4-one (Compound 2)

(1) Substantially the same manner as that in Example 1 (1) was repeated except that 2.40 mL (30.0 mmol) of iodoethane was used in place of iodomethane and the resulting compound was purified by silica gel column chromatography (n-hexane:ethyl acetate=3:1), to give 2.78 g of ethyl 4-ethyl-3,5-difluoro-6-pivaloylamino-2-(2-tetrahydropyranyloxy)benzoate (yield: 67%).

NMR (90MHz, $CDCl_3$) δ (ppm) 1.20 (t, 3H, J=7.7Hz), 1.29 (s, 18H), 1.37 (t, 3H, J=7.0Hz), 1.4-2.0 (m, 6H), 2.72 (q, 2H, J=7.7Hz), 3.4-4.2 (m, 2H), 4.34 (q, 2H, J=7.0Hz), 5.28 (brs, 1H), 7,67 (brs, 1H)
FAB-MS (M/Z) 414 ($M^+$+H)
Molecular formula $C_{21}H_{29}F_2NO_5$=413

(2) Substantially the same manner as that in Example 1 (2) was repeated except that 2.00 g of the above ethyl 4-ethyl-3,5-difluoro-6-pivaloylamino-2-(2-tetrahydropyranyloxy)benzoate and 960 mg (4.04 mmol) of compound IV-1 obtained in Reference Example 2 were used, to give 840 mg of 7-ethyl-6,8-difluoro-2-(3-fluoro-4-pivaloylaminophenyl)-5-pivaloylamino-4H-1-benzopyran-4-one (yield: 41%).

NMR (90MHz, $CDCl_3$) δ (ppm) 1.35 (t, 3H, J=7.5Hz), 1.36 (s, 9H), 1.38 (s, 9H), 2.7-3.1 (m, 2H), 6.65 (s, 1H), 7,5-7.9 (m, 3H), 8.59 (t, 1H, 8.4Hz), 10.5 (brs, 1H)
EIMS (M/Z) 502 ($M^+$)
Molecular formula $C_{27}H_{29}F_3N_2O_4$=502

(3) Substantially the same manner as that in Example 1 (3) was repeated except that 800 mg (1.59 mmol) of the above 7-ethyl-6,8-difluoro-2-(3-fluoro-4-pivaloylaminophenyl)-5-pivaloylamino-4H-1-benzopyran-4-one was used, to give 235 mg of Compound 2 (yield: 44%).

NMR (270MHz, DMSO-$d_6$) δ (ppm) 1.21 (t, 3H, J=7.6Hz), 2.74 (q, 2H, J=7.6Hz), 6.08 (brs, 2H), 6.66 (s, 1H), 6.87 (t, 1H, J=8.8Hz), 6.99 (brs, 2H), 7.59 (dd, 1H, J=8.6, 2.0Hz), 7.65 (dd, 1H, J=13.0, 2.0Hz)
EIMS (M/Z) 334 ($M^+$)
Molecular formula $C_{17}H_{13}F_3N_2O_2$=334

Example 3

5-Amino-2-(4-amino-3-fluorophenyl)-7-(1-butyl)-6,8-difluoro-4H-1-benzopyran-4-one (Compound 3)

(1) Substantially the same manner as that in Example 1 (1) was repeated except that 3.41 mL (30.0 mmol) of 1-iodobutane was used in place of iodomethane and the resulting compound was purified by silica gel column chromatography (n-hexane:ethyl acetate=4:1), to give 1.30 g of ethyl 4-(1-butyl)-3,5-difluoro-6-pivaloylamino-2-(2-tetrahydropyranyloxy)benzoate (yield: 29%).

NMR (90MHz, $CDCl_3$) δ (ppm) 0.92 (t, 3H, J=5.9Hz), 1.29 (s, 9H), 1.37 (t, 3H, J=7.0Hz), 1.4-2.0 (m, 10H), 2.69 (brt, 2H), 3.4-4.2 (m, 2H), 4.34 (q, 2H, J=7.0Hz), 5,27 (brs, 1H), 7.67 (brs, 1H)
FAB-MS (Negative) (M/Z) 440 (M-$H^-$)
Molecular formula $C_{23}H_{33}F_2NO_5$=441

(2) Substantially the same manner as that in Example 1 (2) was repeated except that 1.30 g (2.95 mmol) of the above ethyl 4-(1-butyl)-3,5-difluoro-6-pivaloylamino-2-(2 tetrahydropyranyloxy)benzoate and 585 mg (2.46 mmol) of compound IV-1 obtained in Reference Example 2 were used, to give 470 mg of 7-(1-butyl)-6,8-difluoro-2-(3-fluoro-4-pivaloylaminophenyl)-5-pivaloylamino-4H-1-benzopyran-4-one (yield: 36%).

NMR (90MHz, DMSO-$d_6$) δ (ppm) 0.96 (t, 3H, J=6.3Hz), 1.2-1.9 (m, 7H), 1.36 (s, 9H), 1.39 (s, 9H), 2.7-3.0 (m, 2H), 6.64 (s, 1H), 7.5-7.9 (m, 3H), 8.59 (t, 1H, J=8.4Hz), 10.5 (brs, 1H)

EIMS (M/Z) 530 ($M^+$)
Molecular formula $C_{29}H_{33}F_3N_2O_4$=530

(3) Substantially the same manner as that in Example 1 (3) was repeated except that 450 mg (0.849 mmol) of the above 7-(1-butyl)-6,8-difluoro-2-(3-fluoro-4-pivaloylaminophenyl)-5-pivaloylamino-4H-1-benzopyran-4-one was used, to give 144 mg of Compound 3 (yield: 47%).

NMR (270MHz, $CDCl_3$) δ (ppm) 0.95 (t, 3H, J=7.4Hz), 1.40 (sextet, 2H, J=7.4Hz), 1.63 (quint., 2H, J=7.4Hz), 2.79 (t, 2H, J=7.4Hz), 4.16 (brs, 2H), 6.12 (brs, 2H), 6.46 (s, 1H), 6.84 (t, 1H, J=8.1Hz), 7.5-7.6 (m, 2H)
EIMS (M/Z) 362 (M+)
Molecular formula $C_{19}H_{17}F_3N_2O_2$=362

Example 4

5-Amino-2-(4-amino-3-fluorophenyl)-6,8-difluoro-7-(1-hexyl)-4H-1-benzopyran-4-one (Compound 4)

(1) Substantially the same manner as that in Example 1 (1) was repeated except that 4.43 mL (30.0 mmol) of 1-iodohexane was used in place of iodomethane and the resulting compound was purified by silica gel column chromatography (n-hexane:ethyl acetate=4:1), to give 3.32 g of ethyl 3,5-difluoro-4-(1-hexyl)-6-pivaloylamino-2-(2-tetrahydropyranyloxy)benzoate (yield: 71%).

NMR (90MHz, $CDCl_3$) δ (ppm) 0.7-1.0 (m, 3H), 1.29 (s, 9H), 1.37 (t, 3H, J=7.0Hz), 1.4-2.0 (m, 14H), 2.5-2.9 (m, 2H), 3.4-4.2 (m, 2H), 4.34 (q, 2H, J=7.0Hz), 5.26 (brs, 1H), 7.67 (brs, 1H)
FAB-MS (Negative) (M/Z) 468 ($M-H^-$)
Molecular formula $C_{25}H_{37}F_2NO_5$=469

(2) Substantially the same manner as that in Example 1 (2) was repeated except that 3.31 g (7.06 mmol) of the above ethyl 3,5-difluoro-4-(1-hexyl)-6-pivaloylamino-2-(2-tetrahydropyranyloxy)benzoate and 1.40 g (5.88 mmol) of compound IV-1 obtained in Reference Example 2 were used, to give 1.41 g of 6,8-difluoro-2-(3-fluoro-4-pivaloylaminophenyl)-7-(1-hexyl)-5-pivaloylamino-4H-1- benzopyran-4-one (yield: 43%).

NMR (90MHz, $CDCl_3$) δ (ppm) 0.8-1.0 (m, 3H), 1.1-1.8 (m, 8H), 1.36 (s, 9H), 1.38 (s, 9H), 2.7-3.0 (m, 2H), 6.64 (s, 1H), 7.5-7.9 (m, 3H), 8.59 (t, 1H, 8.4Hz), 10.5 (brs, 1H)
EIMS (M/Z) 558 ($M^+$)
Molecular formula $C_{31}H_{37}F_3N_2O_4$=558

(3) Substantially the same manner as that in Example 1 (3) was repeated except that 1.35 g (2.42 mmol) of the above 6,8-difluoro-2-(3-fluoro-4-pivaloylaminophenyl)-7-(1-hexyl)-5-pivaloylamino-4H-1-benzopyran-4-one was used, to give 499 mg of Compound 4 (yield: 53%).

NMR (270MHz, $CDCl_3$) δ (ppm) 0.89 (t, 3H, J=6.9Hz), 1.2-1.5 (m, 6H), 1.64 (quint., 2H, J=7.4Hz), 2.78 (t, 2H, J=7.4Hz), 4.16 (brs, 2H), 6.16 (brs, 2H), 6.46 (s, 1H), 6.84 (t, 1H, J=8.9Hz), 7.5-7.6 (m, 2H)
EIMS (M/Z) 390 ($M^+$)
Molecular formula $C_{21}H_{21}F_3N_2O_2$=390

Example 5

5-Amino-2-(4-amino-3-fluorophenyl)-7-bromo-6,8-difluoro-4H-1-benzopyran-4-one (Compound 6)

(1) Substantially the same manner as that in Example 1 (1) was repeated except that 2.59 mL (30.0 mmol) of 1,2-dibromoethane was used in place of iodomethane and the resulting compound was purified by silica gel column chromatography (n-hexane:ethyl acetate=4:1), to give 2.26 g of ethyl 4-bromo-3,5-difluoro-6-pivaloylamino-2-(2-tetrahydropyranyloxy)benzoate (yield: 49%).

NMR (90MHz, $CDCl_3$) δ (ppm) 1.29 (s, 9H), 1.38 (t, 3H, J=7.0Hz), 1.4-2.0 (m, 6H), 3.4-4.2 (m, 2H), 4.35 (q, 2H, J=7.0Hz), 5.32 (brs, 1H), 7.65 (brs, 1H)
FAB-MS (Negative) (M/Z) 462,464 ($M-H^-$)
Molecular formula $C_{19}H_{24}{}^{79}BrF_2NO_5$=463

(2) Substantially the same manner as that in Example 1 (2) was repeated except that 2.05 g (4.42 mmol) of the above ethyl 4-bromo-3,5-difluoro-6-pivaloylamino-2-(2-tetrahydropyranyloxy)benzoate and 956 mg (4.02 mmol) of compound IV-1 obtained in Reference Example 2 were used, to give 1.27 g of 7-bromo-6,8-difluoro-2-(3-fluoro-4-pivaloylaminophenyl)-5-pivaloylamino-4H-1-benzopyran-4-one (yield: 57%).

NMR (90MHz, $CDCl_3$) δ (ppm) 1.37 (s, 9H), 1.38 (s, 9H), 6.68 (s, 1H), 7.5-8.0 (m, 3H), 8.60 (t, 1H, J=8.4Hz), 10.6 (brs, 1H)
FAB-MS (M/Z) 553, 555 ($M^+$+H)
Molecular formula $C_{25}H_{24}{}^{79}BrF_3N_2O_4$=552

(3) Substantially the same manner as that in Example 1 (3) was repeated except that 500 mg (0.938 mmol) of the above 7-bromo-6,8-difluoro-2-(3-fluoro-4-pivaloylaminophenyl)-5-pivaloylamino-4H-1-benzopyran-4-one was used, to give 138 mg of Compound 6 (yield: 38%).

NMR (270MHz, DMSO-$d_6$) δ (ppm) 6.13 (brs, 2H), 6.72 (s, 1H), 6.87 (t, 1H, J=8.9Hz), 7.27 (brs, 2H), 7.59 (dd, 1H, J=8.4, 1.5Hz), 7.66 (dd, 1H, J=12.7, 1.5Hz)
EIMS (M/Z) 386, 384 ($M^+$)
Molecular formula $C_{15}H_8{}^{79}BrF_3N_2O_2$=384

Example 6

5-Amino-2-(4-amino-3-fluorophenyl)-6,8-difluoro-7-methylthio-4H-1-benzopyran-4-one (Compound 10)

(1) Substantially the same manner as that in Example 1 (1) was repeated except that 1.80 mL (20.0 mmol) of dimethyl disulfide was used in place of iodomethane, to give 3.82 g of ethyl 3,5-difluoro-4-methylthio-6-pivaloylamino-2-(2-tetrahydropyranyloxy)benzoate (yield: 89%).

NMR (90MHz, $CDCl_3$) δ (ppm) 1.29 (s, 9H), 1.37 (t, 3H, J=7.0Hz), 1.4-2.0 (m, 6H), 3.4-4.2 (m, 2H), 4.34 (q, 2H, J=7.0Hz), 5.28 (brs, 1H), 7.63 (brs, 1H)
FAB-MS (Negative) (M/Z) 430 ($M-H^-$)
Molecular formula $C_{20}H_{27}NO_3S$=431

(2) Substantially the same manner as that in Example 1 (2) was repeated except that 3.56 g (8.26 mmol) of the above ethyl 3,5-difluoro-4-methylthio-6-pivaloylamino-2-(2-tetrahydropyranyloxy)benzoate and 1.63 g (6.88 mmol) of compound IV-l obtained in Reference Example 2 were used, to give 2.07 g of 6,8-difluoro-2-(3-fluoro-4-pivaloylaminophenyl)-7-methylthio-5-pivaloylamino-4H-1-benzopyran-4-one (yield: 58%).

NMR (90MHz, $CDCl_3$) δ (ppm) 1.36 (s, 9H), 1.38 (s, 9H), 2.66 (t, 3H, J=1.3Hz), 6.65 (s, 1H), 7.5-7.9 (m, 2H), 8.60 (t, 1H, J=8.4Hz)
EIMS (M/Z) 520 ($M^+$)
Molecular formula $C_{26}H_{27}F_3N_2O_4S$=520

(3) 511 mg (0.983 mmol) of the above 6,8-difluoro-2-(3-fluoro-4-pivaloylaminophenyl)-7-methylthio-5-pivaloylamino-4H-1-benzopyran-4-one was dissolved in 20 mL of concentrated sulfuric acid and the solution was stirred at 50 °C for 10 minutes. The reaction solution was poured into ice water, the solution was made neutral and the precipitated crystals were collected by filtration. The crystals were purified by silica gel column chromatography (chloroform) and recrystallized from ethyl acetate/n-hexane to give 309 mg of Compound 10 (yield: 89%).

NMR (270MHz, DMSO-$d_6$) δ (ppm) 2.59 (s, 3H), 6.11 (brs, 2H), 6.69 (s, 1H), 6.86 (t, 1H, J=8.7Hz), 7.10 (brs, 2H), 7.59 (dd, 1H, J=8.4, 2.0Hz), 7.66 (dd, 1H, J=12.9, 2.0Hz) EIMS (M/Z) 352 ($M^+$)
Molecular formula $C_{16}H_{11}F_3N_2O_2S$=352

Example 7

5-Amino-2-(4-amino-3-fluorophenyl)-6,8-difluoro-7-hydroxy-4H-1-benzopyran-4-one (Compound 7)

(1) 7.00 mL (50.0 mmol) of diisopropylamine was dissolved in 14 mL of tetrahydrofuran under argon atmosphere

and 30 mL of a 1.6 M solution of 48 mmol of n-butyl lithium in n-hexane was added thereto dropwise while keeping an internal temperature at -10 to 0 °C. The reaction solution was cooled to -60 °C or below (internal temperature) and a solution of 7.70 g (20.0 mmol) of compound II-1 obtained in Reference Example 1 in 60 mL of tetrahydrofuran was added dropwise. The mixture was stirred at the same temperature for 2 hours to lithiate 4-position. 2.7 mL (24 mmol) of trimethoxyborane was added and the temperature of the mixture was raised to 0 °C for 20 minutes. 4.0 mL of acetic acid and 8.0 mL of 30% hydrogen peroxide were added to the reaction solution and the mixture was stirred at room temperature for 20 hours. An aqueous solution of sodium hydrogensulfite was added to the reaction solution, the temperature of the mixture was raised to room temperature and the mixture was washed once with ethyl acetate. The aqueous layer was made acidic by addition of 2N hydrochloric acid and extracted once with ethyl acetate. The organic layer was washed once with water and once with an aqueous saturated solution of sodium chloride and dried over anhydrous sodium sulfate. The solvent was distilled off under reduced pressure and the residue was triturated with n-hexane to give 4.33 g of ethyl 3,5-difluoro-4-hydroxy-6-pivaloylamino-2-(2-tetrahydropyranyloxy)benzoate (yield: 54%).

NMR (90MHz, $CDCl_3$) δ (ppm) 1.30 (s, 9H), 1.37 (t, 3H, J=7.0Hz), 1.4-2.0 (m, 6H), 3.4-4.2 (m, 2H), 4.34 (g, 2H, J=7.0Hz), 5.28 (brs, 1H), 8.15 (brs, 1H)
FAB-MS (Negative) (M/Z) 400 $(M-H^-)$
Molecular formula $C_{19}H_{25}F_2NO_6$=401

(2) 4.23 g (10.5 mmol) of the above ethyl 3,5-difluoro-4-hydroxy-6-pivaloylamino-2-(2-tetrahydropyranyloxy)benzoate was dissolved in 50 mL of dichloromethane, 2.4 mL (13.7 mmol) of diisopropylethylamine and 0.96 mL (12.6 mmol) of chloromethyl methyl ether were added under ice-cooling and the mixture was stirred at the same temperature for 30 minutes. Water was added to the reaction solution and the mixture was extracted once with chloroform. The organic layer was washed once with an aqueous saturated solution of sodium chloride and dried over anhydrous sodium sulfate. The solvent was distilled off under reduced pressure and the residue was triturated with n-hexane to give 4.33 g of ethyl 3,5-difluoro-4-methoxymethoxy-6-pivaloylamino-2-(2-tetrahydropyranyloxy)benzoate (yield: 93%).

NMR (90MHz, $CDCl_3$) δ (ppm) 1.29 (s, 9H), 1.37 (t, 3H, J=7.0Hz), 1.4-2.0 (m, 6H), 3.57 (s, 3H), 4.34 (q, 2H, J=7.0Hz), 5.21 (s, 2H), 5.28 (brs, 1H), 7,80 (brs, 1H)
FAB-MS (Negative ) (M/Z) 444 $(M-H^-)$
Molecular formula $C_{21}H_{29}F_2NO_7$=445

(3) substantially the same manner as that in Example 1 (2) was repeated except that 3.98 g (8.94 mol) of the above ethyl 3,5-difluoro-4-methoxymethoxy-6-pivaloylamino-2-(2-tetrahydropyranyloxy)benzoate and 2.17 g (9.17 mmol) of compound IV-1 obtained in Reference Example 2 were used, to give 1.15 g of 6,8-difluoro-2-(3-fluoro-4-pivaloylaminophenyl)-7-hydroxy-5-pivaloylamino-4H-1-benzopyran-4-one (yield: 26%).

NMR (90MHz, $CDCl_3$) δ (ppm) 1.36 (s, 9H), 1.37 (s, 9H), 6.59 (s, 1H), 7.5-7.9 (m, 3H), 8.53 (t, 1H, J=8.4Hz), 10.6 (brs, 1H), 10.9 (brs, 1H)
EIMS (M/Z) 490 $(M^+)$
Molecular formula $C_{25}H_{25}F_3N_2O_5$=490

(4) Substantially the same manner as that in Example 6 (3) was repeated except that 409 mg (0.834 mmol) of the above 6,8-difluoro-2-(3-fluoro-4-pivaloylaminophenyl)-7-hydroxy-5-pivaloylamino-4H-1-benzopyran-4-one was used, and the resulting compound was purified by silica gel column chromatography (chloroform:methanol=200:1-9:1) and recrystallized from methanol/isopropyl ether, to give 139 mg of Compound 7 (yield: 52%).

NMR (270MHz, DMSO-$d_6$) δ (ppm) 6.02 (brs, 2H), 6.57 (s, 1H), 6.87 (t, 1H, J=8.7Hz), 6.97 (brs, 2H), 7.56 (dd, 1H, J=8.4, 2.0Hz), 7.62 (dd, 1H, J=12.9, 2.0Hz), 11.3 (brs, 1H)
EIMS (M/Z) 322 $(M^+)$
Molecular formula $C_{15}H_9F_3N_2O_3$=322

Example 8

5-Amino-2-(4-amino-3-chlorophenyl)-6,8-difluoro-7-methyl-4H-1-benzopyran-4-one (Compound 24)

(1) Substantially the same manner as that in Example 1 (2) was repeated except that 14.6 g (36.7 mmol) of ethyl

3,5-difluoro-4-methyl-6-pivaloylamino-2-(2-tetrahydropyranyloxy)benzoate obtained in Example 1 (1) and 7.73 g (30.6 mmol) of compound IV-2 obtained in Reference Example 3 were used, to give 8.41 g of 2-(3-chloro-4-pivaloylaminophenyl)-6,8-difluoro-7-methyl-5-pivaloylamino-4H-1-benzopyran-4-one (yield: 55%).

NMR (90MHz, $CDCl_3$) δ (ppm) 1.38 (s, 18H), 2.41 (t, 3H, J=2.2Hz), 6.64 (s, 1H), 7.82 (dd, 1H, J=9.0, 2.0Hz), 7.95 (d, 1H, J=2.0Hz), 8.22 (brs, 1H), 8.66 (d, 1H, J=9.0Hz)
EIMS (M/Z) 504 ($M^+$)
Molecular formula $C_{26}H_{27}{}^{35}ClF_2N_2O_4$=504

(2) 8.29 g (16.5 mmol) of the above 2-(3-chloro-4-pivaloylaminophenyl)-6,8-difluoro-7-methyl-5-pivaloylamino-4H-1-benzopyran-4-one was dissolved in 60 mL of concentrated sulfuric acid and the solution was stirred at 50 °C for 10 minutes. The reaction solution was poured into ice water and the precipitated crystals were collected by filtration. The crystals were triturated with 1N aqueous solution of sodium hydroxide and collected by filtration again. The crystals were recrystallized twice from ethyl acetate/n-hexane to give 2.42 g of Compound 24 (yield: 44%).

NMR (270MHz, DMSO-$d_6$) δ (ppm) 2.27 (t, 3H, J=1.7Hz), 6.24 (brs, 2H), 6.66 (s, 1H), 6.89 (d, 1H, J=8.4Hz), 6.97 (brs, 2H), 7.69 (dd, 1H, J=8.9, 2.0Hz), 7.83 (d, 1H, J=2.0Hz)
EIMS (M/Z) 336 ($M^+$)
Molecular formula $C_{16}H_{11}{}^{35}ClF_2N_2O_2$=336

Example 10

5-Amino-2-(4-amino-3-ethylphenyl)-6,8-difluoro-7-methyl-4H-1-benzopyran-4-one (Compound 26)

(1) Substantially the same manner as that in Example 1 (2) was repeated except that 6.90 g (17.3 mmol) of ethyl 3,5-difluoro-4-methyl-6-pivaloylamino-2-(2-tetrahydropyranyloxy)benzoate obtained in Example 1 (1) and 2.84 g (11.5 mmol) of compound IV-3 were used, and the resulting compound was recrystallized from ethyl acetate/n-hexane, to give 1.15 g of 2-(3-ethyl-4-pivaloylaminophenyl)-6,8-difluoro-7-methyl-5-pivaloylamino-4H-1-benzopyran-4-one (yield: 20%).

NMR (90MHz, $CDCl_3$) δ (ppm) 1.33 (t, 3H, J=7.7Hz), 1.37 (s, 9H), 1.38 (s, 9H), 2.40 (t, 3H, J=2.2Hz), 2.67 (q, 2H, J=7.7Hz), 6.67 (s, 1H), 7.56 (brs, 1H), 7.6-7.9 (m, 2H), 8.24 (d, 1H, J=9.0Hz)
EIMS (M/Z) 498 ($M^+$)
Molecular formula $C_{28}H_{32}F_2N_2O_4$=498

(2) Substantially the same manner as that in Example 8 (2) was repeated except that 1.10 g (2.21 mmol) of the above 2-(3-ethyl-4-pivaloylaminophenyl)-6,8-difluoro-7-methyl-5-pivaloylamino-4H-1-benzopyran-4-one was used, and the resulting compound was recrystallized from ethyl acetate/n-hexane, to give 488 mg of Compound 26 (yield: 67%).

NMR (270MHz, DMSO-$d_6$) δ (ppm) 1.18 (t, 3H, J=7.4Hz), 2.27 (brs, 3H), 2.4-2.6 (m, 2H), 5.83 (brs, 2H), 6.56 (s, 1H), 6.70 (d, 1H, J=9.4Hz), 6.96 (brs, 2H), 7.5-7.6 (m, 2H)
EIMS (M/Z) 330 ($M^+$)
Molecular formula $C_{18}H_{16}F_2N_2O_2$=330

Example 11

5,7-Diamino-2-(4-amino-3,5-dichlorophenyl)-6,8-difluoro-4H-1-benzopyran-4-one (Compound 27)

(1) Substantially the same manner as that in Example 1 (1) was repeated except that 7.88 g (40.0 mmol) of p-toluenesulfonyl azide was used in place of iodomethane and the resulting compound was purified by silica gel column chromatography (n-hexane:ethyl acetate=6:1-3:1), to give 7.14 g of ethyl 4-azido-3,5-difluoro-6-pivaloylamino-2-(2-tetrahydropyranyloxy)benzoate (yield: 84%).

NMR (90MHz, $CDCl_3$) δ (ppm) 1.29 (s, 9H), 1.37 (t, 3H, J=7.0Hz), 1.4-2.0 (m, 6H), 3.4-4.2 (m, 2H), 4.34 (q, 2H, J=7.0Hz), 5.28 (brs, 1H), 7.81 (brs, 1H)
FAB-MS (M/Z) 427 ($M^+$+H)
Molecular formula $C_{19}H_{24}F_2NO_5$=426

(2) 4.64 g (10.9 mmol) of the above ethyl 4-azido-3,5-difluoro-6-pivaloylamino-2-(2-tetrahydropyranyloxy)benzoate was dissolved in 100 mL of methanol, 2.90 g (76.7 mmol) of sodium borohydride was added in several portions under ice-cooling, 10 mL of water was further added and the mixture was stirred at room temperature for 4.5 hours. The solvent was distilled off under reduced pressure, water was added and the mixture was extracted once with ethyl acetate. The organic layer was washed with an aqueous saturated solution of sodium chloride and dried over anhydrous sodium sulfate. The solvent was distilled off under reduced pressure and the residue was purified by silica gel column chromatography (chloroform:methanol=100:1) to give 4.09 g of ethyl 4-amino-3,5-difluoro-6-pivaloylamino-2-(2-tetrahydropyranyloxy)benzoate (yield: 94%).

NMR (90MHz, $CDCl_3$) δ (ppm) 1.29 (s, 9H), 1.36 (t, 3H, J=7.0Hz), 1.4-2.0 (m, 6H), 3.4-4.2 (m, 2H), 4.06 (brs, 2H), 4.32 (q, 2H, J=7.0Hz), 5.24 (brs, 1H), 8.27 (brs, 1H)
FAB-MS (M/Z) 401 ($m^+$+H)
Molecular formula $C_{19}H_{26}F_2N_2O_5$=400

(3) 809 mg (2.02 mmol) of the above ethyl 4-amino-3,5-difluoro-6-pivaloylamino-2-(2-tetrahydropyranyloxy)benzoate was dissolved in 20 mL of dimethylformamide under argon atmosphere, 240 mg (6.00 mmol) of sodium hydride and 1.4 mL (6.0 mmol) of di-tert-butyl dicarbonate were added under ice-cooling and the mixture was stirred at the same temperature for 6 hours. Water was added to the reaction solution and the mixture was extracted once with ethyl acetate. The organic layer was washed with an aqueous saturated solution of sodium chloride and dried over anhydrous sodium sulfate. The solvent was distilled off under reduced pressure and the residue was purified by silica gel column chromatography (n-hexane:ethyl acetate=4:1) to give 740 mg of ethyl 4-tert-butoxycarbonylamino-3,5-difluoro-6-pivaloylamino-2-(2-tetrahydropyranyloxy)benzoate (yield: 73%).

NMR (90MHz, $CDCl_3$) δ (ppm) 1.29 (s, 9H), 1.39 (t, 3H, J=7.3Hz), 1.43 (s, 9H), 1.4-2.0 (m, 6H), 3.4-4.2 (m, 2H), 4.34 (q, 2H, J=7.3Hz), 5.31 (brs, 1H), 7.53 (brs, 1H)

(4) Substantially the same manner as that in Example 1 (2) was repeated except that 3.64 g (7.28 mmol) of the above ethyl 4-tert-butoxycarbonylamino-3,5-difluoro-6-pivaloylamino-2-(2-tetrahydropyranyloxy)benzoate and 1.75 g (6.08 mmol) of compound IV-4 obtained in Reference Example 5 were used, and the resulting compound was purified by silica gel column chromatography (chloroform:methanol=100:1), to give 400 mg of 7-amino-2-(3,5-dichloro-4-pivaloylaminophenyl)-6,8-difluoro-4H-1-benzopyran-4-one (yield: 12%).

NMR (90MHz, $CDCl_3$) δ (ppm) 1.38 (s, 9H), 1.40 (s, 9H), 4.57 (brs, 2H), 6.56 (s, 1H), 7.81 (s, 2H)
FAB-MS (M/Z) 540 ($M^+$+H)
Molecular formula $C_{25}H_{25}{}^{35}Cl_2F_2N_2O_4$=539

(5) Substantially the same manner as that in Example 8 (2) was repeated except that 400 mg (0.741 mmol) of the above 7-amino-2-(3,5-dichloro-4-pivaloylaminophenyl)-6,8-difluoro-4H-1-benzopyran-4-one was used, and the resulting compound was triturated with isopropyl ether, to give 202 mg of Compound 27 (yield: 73%).

NMR (270MHz, DMSO-$d_6$) δ (ppm) 6.24 (brs, 2H), 6.28 (brs, 2H), 6.68 (s, 1H), 6.84 (brs, 2H), 7.83 (s, 2H)
EIMS (M/Z) 371 ($M^+$)
Molecular formula $C_{15}H_9{}^{35}Cl_2F_2N_3O_2$=371

Example 12

5-Amino-2-(4-amino-3-fluorophenyl)-7-chloro-6,8-difluoro-4H-1-benzopyran-4-one (Compound 5)

(1) 1.54 mL (11.0 mmol) of diisopropylamine was dissolved in 10 mL of tetrahydrofuran under argon atmosphere and 6.3 mL of a 1.6 M solution of 10 mmol of n-butyl lithium in n-hexane was added dropwise while keeping an internal temperature at -10 to 0 °C. The reaction solution was cooled to -60 °C or below (internal temperature) and a solution of 1.19 g (2.50 mmol) of compound VIII-1 obtained in Reference Example 6 dissolved in a mixed solvent of 50 mL of tetrahydrofuran and 10 mL of hexamethylphosphoric triamide was added dropwise. The mixture was stirred at the same temperature for 2 hours to lithiate 7-position. 802 mg (6.00 mol) of N-chlorosuccinimide was added and the mixture was stirred for additional 10 minutes. Water was added to the reaction solution, the temperature of the mixture was raised to room temperature and the mixture was extracted once with ethyl acetate. The organic layer was washed once with an aqueous saturated solution of sodium chloride and dried over anhydrous sodium sulfate. The solvent was distilled off under reduced pressure and the residue was purified by silica gel column chromatography (chloroform:acetone=60:1) to give 384 mg of 7-chloro-6,8-difluoro-2-(3-fluoro-4-pivaloylaminophenyl)-5-pivaloylamino-4H-1-benzopyran-4-one (yield: 30%).

NMR (90MHz, $CDCl_3$) δ (ppm) 1.36 (s, 9H), 1.38 (s, 9H), 6.67 (s, 1H), 7.5-7.9 (m, 3H), 8.61 (dd, 1H, J=9.2, 8.4Hz), 10.5 (brs, 1H)
FAB-MS (M/Z) 509 ($M^+$+H)
Molecular formula $C_{25}H_{24}{}^{35}ClF_3N_2O_4$=508

(2) Substantially the same manner as that in Example 8 (2) was repeated except that 343 mg (0.675 mmol) of 7-chloro-6,8-difluoro-2-(3-fluoro-4-pivaloylaminophenyl)-5-pivaloylamino-4H-1-benzopyran-4-one was used, and the resulting compound was triturated with isopropyl ether, to give 168 mg of Compound 5 (yield: 73%).

NMR (270MHz, DMSO-$d_6$) δ (ppm) 6.11 (brs, 2H), 6.71 (s, 1H), 6.87 (t, 1H, J=8.9Hz), 7.30 (brs, 2H), 7.59 (dd, 1H, J=8.9, 2.0Hz), 7.65 (dd, 1H, J=12.9, 2.0Hz)
EIMS (M/Z) 340 ($M^+$)
Molecular formula $C_{15}H_8{}^{35}ClF_3N_2O_2$=340

Example 13

5-Amino-2-(4-amino-3-fluorophenyl)-7-ethoxycarbonyl-6,8-difluoro-4H-1-benzopyran-4-one (Compound 13)

(1) Substantially the same manner as that in Example 12 (1) was repeated except that 0.29 mL (5.0 mmol) of ethyl chloroformate was used in place of N-chlorosuccinimide and the resulting compound was purified by silica gel column chromatography (chloroform:ethyl acetate=40:1), to give 900 mg of 7-ethoxycarbonyl-6,8-difluoro-2-(3-fluoro-4-pivaloylaminophenyl)-5-pivaloylamino-4H-1-benzopyran-4-one (yield: 66%).

NMR (90MHz, $CDCl_3$) δ (ppm) 1.36 (s, 9H), 1.38 (s, 9H), 1.43 (t, 3H, J=7.0Hz), 4.49 (q, 2H, J=7.0Hz), 6.69 (s, 1H), 7.5-7.9 (m, 3H), 8.60 (t, 1H, J=8.5Hz), 10.4 (brs, 1H)
EIMS (M/Z) 546 ($M^+$)
Molecular formula $C_{28}H_{29}F_3N_2O_4$=546

(2) 740 mg (1.36 mol) of the above 7-ethoxycarbonyl-6,8-difluoro-2-(3-fluoro-4-pivaloylaminophenyl)-5-pivaloylamino-4H-1-benzopyran-4-one was subjected to the depivaloylation with concentrated sulfuric acid substantially in the same manner as that in Example 8 (2). The resulting solution was adjusted to pH 7 and extracted once with ethyl acetate. The organic layer was washed once with an aqueous saturated solution of sodium chloride and dried over anhydrous sodium sulfate. The solvent was distilled off under reduced pressure, and the residue was purified by silica gel column chromatography (chloroform) and recrystallized from ethyl acetate/n-hexane to give 259 mg of Compound 13 (yield: 50%).

NMR (270MHz, DMSO-$d_6$) δ (ppm) 1.34 (t, 3H, J=7.2Hz), 4.43 (q, 2H, J=7.2Hz), 6.15 (brs, 2H), 6.67 (s, 1H), 6.87 (t, 1H, J=8.9Hz), 7.26 (brs, 2H), 7.61 (dd, 1H, J=8.4, 2.0Hz), 7.67 (dd, 1H, J=12.9, 2.0Hz)
EIMS (M/Z) 378 ($M^+$)
Molecular formula $C_{18}H_{13}F_3N_2O_4$=378

Example 14

5-Amino-2-(4-amino-3-fluorophenyl)-7-azido-6,8-difluoro-4H-1-benzopyran-4-one (Compound 15)

(1) Substantially the same manner as that in Example 12 (1) was repeated except that 1.78 g (9.00 mmol) of p-toluenesulfonyl azide was used in place of N-chlorosuccinimide and the precipitated crystals were collected by filtration after water was added to the reaction solution and the temperature was raised to room temperature, to give 3.11 g of 7-azido-6,8-difluoro-2-(3-fluoro-4-pivaloylaminophenyl)-5-pivaloylamino-4H-1-benzopyran-4-one (yield: 81%).

NMR (90MHz, DMSO-$d_6$) δ (ppm) 1.26 (s, 9H), 1.29 (s, 9H), 7.08 (s, 1H), 7.7-8.0 (m, 3H), 9.19 (brs, 1H), 10.2 (brs, 1H)
FAB-MS (Negative) (M/Z) 514 (M-$H^-$)
Molecular formula $C_{25}H_{24}F_3N_5O_4$=515

(2) Substantially the same manner as that in Example 13 (2) was repeated except that 320 mg (0.621 mol) of the above 7-azido-6,8-difluoro-2(3-fluoro-4-pivaloylaminophenyl)-5-pivaloylamino-4H-1-benzopyran-4-one was used, and the resulting compound was purified by preparative thin layer chromatography (chloroform:methanol=20:1), to give 180 mg of compound 15 (yield: 84%).

NMR (270MHz, DMSO-$d_6$) δ (ppm) 6.12 (brs, 2H), 6.67 (s, 1H), 6.85 (t, 1H, J=8.7Hz), 7.22 (brs, 2H), 7.57 (dd, 1H, J=8.4, 2.0Hz), 7.64 (dd, 1H, J=12.9, 2.0Hz)
EIMS (M/Z) 347 ($M^+$)
Molecular formula $C_{15}H_8F_3N_5O_2$=347

Example 15

5-Amino-2-(4-amino-3-fluorophenyl)-6,8-difluoro-7-(1-hydroxyethyl)-4H-1-benzopyran-4-one (Compound 20)

(1) Substantially the same manner as that in Example 12 (1) was repeated except that 1 mL (large excess) of acetaldehyde was introduced in a gaseous condition in place of N-chlorosuccinimide and the resulting compound was purified by silica gel column chromatography (chloroform:acetonitrile=30:1-9:1), to give 730 mg of 6,8-difluoro-2-(3-fluoro-4-pivaloylaminophenyl)-7-(1-hydroxyethyl)-5-pivaloylamino-4H-1-benzopyran-4-one (yield: 70%).

NMR (90MHz, $CDCl_3$) δ (ppm) 1.37 (s, 9H), 1.38 (s, 9H), 1.72 (d, 3H, J=6.8Hz), 2.5-2.8 (m, 1H), 5.2-5.5 (m, 1H), 6.64 (s, 1H), 7.5-7.9 (m, 3H), 8.57 (t, 1H, J=8.5Hz), 10.4 (brs, 1H)
FAB-MS (M/Z) 519 ($M^+$+H)
Molecular formula $C_{27}H_{29}F_3N_2O_5$=518

(2) Substantially the same manner as that in Example 13 (2) was repeated except that 320 mg (0.621 mol) of the above 6,8-difluoro-2-(3-fluoro-4-pivaloylaminophenyl)-7-(1-hydroxyethyl)-5-pivaloylamino-4H-1-benzopyran-4-one was used, and the resulting compound was purified by preparative thin layer chromatography (chloroform:methanol=20:1), to give 8.0 mg of Compound 20 (yield: 6.1%).

NMR (270MHz, DMSO-$d_6$) δ (ppm) 1.51 (d, 3H, J=6.9Hz), 5.1-5.3 (m, 1H), 5.55 (d, 1H, J=4.5Hz), 6.09 (brs, 2H), 6.68 (s, 1H), 6.87 (t, 1H, J=8.9Hz), 6.99 (brs, 2H), 7.60 (dd, 1H, J=8.4, 2.0Hz), 7.66 (dd, 1H, J=12.9, 2.0Hz)
FAB-MS (M/Z) 351 ($M^+$+H)
Molecular formula $C_{17}H_{13}F_3N_2O_3$=350

Example 16

5-Amino-2-(4-amino-3-fluorophenyl)-6,8-difluoro-7-methoxy-4H-1-benzopyran-4-one (Compound 8)

(1) 348 mg (0.710 mmol) of 6,8-difluoro-2-(3-fluoro-4-pivaloylaminophenyl)-7-hydroxy-5-pivaloylamino-4H-1-benzopyran-4-one obtained in Example 7 (3) was dissolved in 35 mL of acetone, 166 mg (1.20 mmol) of potassium carbonate and 0.45 mL (7.1 mmol) of iodomethane were added and the mixture was heated at reflux for 40 minutes. The reaction solution was filtered, the filtrate was concentrated, water was added to the residue and the mixture was extracted once with ethyl acetate. The organic layer was washed twice with a 1N aqueous solution of sodium hydroxide, once with water and once with an aqueous saturated solution of sodium chloride and dried over anhydrous sodium sulfate. The solvent was distilled off under reduced pressure and the residue was purified by silica gel column chromatography (chloroform) to give 280 mg of 6,8-difluoro-2-(3-fluoro-4-pivaloylaminophenyl)-7-methoxy-5-pivaloylamino-4H-1-benzopyran-4-one (yield: 78%).

NMR (90MHz, $CDCl_3$) δ (ppm) 1.36 (s, 9H), 1.38 (s, 9H , 4.22 (t, 3H, 1,8Hz), 6.61 (s, 1H), 7.5-7.9 (m, 3H), 8.58 (dd, 1H, J=8.6, 7.3Hz), 10.6 (brs, 1H)
EIMS (M/Z) 504 ($M^+$)
Molecular formula $C_{26}H_{27}F_3N_2O_5$=504

(2) Substantially the same manner as that in Example 8 (2) was repeated except that 260 mg (0.516 mmol) of the above 6,8-difluoro-2-(3-fluoro-4-pivaloylaminophenyl)-7-methoxy-5-pivaloylamino-4H-1-benzopyran-4-one was used, and the resulting crystals were purified by silica gel column chromatography (chloroform:acetone=80:1), to give 126 mg of Compound 8 (yield: 73%).

NMR (270MHz, DMSO-$d_6$) δ (ppm) 4.09 (t, 3H, J=1.5Hz), 6.07 (brs, 2H), 6.64 (s, 1H), 6.87 (t, 1H, J=8.9Hz), 7.22 (brs, 2H), 7.57 (dd, 1H, J=8.4, 2.0Hz), 7.64 (dd, 1H, J=12.9, 2.0Hz)
EIMS (M/Z) 336 ($M^+$)
Molecular formula $C_{16}H_{11}F_3N_2O_3$=336

Example 17

5-Amino-2-(4-amino-3-fluorophenyl)-7-(2-dimethylaminoethoxy)-6,8-difluoro-4H-1-benzopyran-4-one (Compound 9)

(1) 490 mg (1.00 mmol) of 6,8-difluoro-2-(3-fluoro-4-pivaloylaminophenyl)-7-hydroxy-5-pivaloylamino-4H-1-benzopyran-4-one obtained in Example 7 (3) was dissolved in 40 mL of dimethylformamide, 3.60 mg (26.0 mmol) of potassium carbonate and 2.88 g (20.0 mmol) of 2-dimethylaminoethyl chloride hydrochloride were added and the mixture was stirred at 50 °C for 12 hours. The reaction solution was filtered, the filtrate was concentrated, water was added to the residue and the mixture was extracted once with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (chloroform:methanol=30:1) to give 230 mg of 7-(2-dimethylaminoethoxy)-6,8-difluoro-2-(3-fluoro-4-pivaloylaminophenyl)-5-pivaloylamino-4H-1-benzopyran-4-one (yield: 41%).

NMR (90MHz, $CDCl_3$) δ (ppm) 1.36 (s, 9H), 1.38 (s, 9H), 2.36 (s, 6H), 2.79 (t, 2H, J=5.5Hz), 4.48 (t, 2H, J=5.5Hz), 6.62 (s, 1H), 7.5-7.9 (m, 3H), 8.58 (dd, 1H, J=8.6, 7.3Hz), 10.6 (brs, 1H)
EIMS (M/Z) 561 ($M^+$)
Molecular formula $C_{29}H_{34}F_3N_3O_5$=561

(2) Substantially the same manner as that in Example 8 (2) was repeated except that 250 mg (0.446 mmol) of the above 7-(2-dimethylaminoethoxy)-6,8-difluoro-2-(3-fluoro-4-pivaloylaminophenyl)-5-pivaloylamino-4H-1-benzopyran-4-one was used, and the resulting crystals were purified by silica gel column chromatography (chloroform:methanol=30:1), to give 170 mg of Compound 9 (yield: 97%). This compound was dissolved in 10 mL of 2-propanol, 0.5 mL of a 1N hydrochloric acid/2-propanol solution was added dropwise and 5 mL of isopropyl ether was further added. The precipitated crystals were collected by filtration to give a hydrochloride of Compound 9.

NMR (279MHz, DMSO-$d_6$) δ (ppm) 2.89 (s, 6H), 3.56 (t, 2H, J=5.0Hz), 4.67 (t, 2H, J=5.0Hz), 6.08 (brs, 2H), 6.67 (s, 1H), 6.87 (t, 1H, 8.9Hz), 7.17 (brs, 2H), 7.5-7.7 (m, 2H), 10.6 (brs, 1H) (hydrochloride)
EIMS (M/Z) 393 ($M^+$)
Molecular formula $C_{19}H_{18}F_3N_3O_3$=393

Example 18

5-Amino-2-(4-amino-3-fluorophenyl)-6,8-difluoro-7-methylsulfinyl-4H-1-benzopyran-4-one (Compound 11)

(1) 204 mg (0.392 mmol) of 6,8-difluoro-2-(3-fluoro-4-pivaloylaminophenyl)-7-methylthio-5-pivaloylamino-4H-1-benzopyran-4-one obtained in Example 6 (2) was dissolved in 5 mL of dichloromethane, 86 mg (0.39 mmol) of m-chloroperbenzoic acid was added under ice-cooling and the mixture was stirred at the same temperature for 2 hours. An aqueous solution of sodium hydrogensulfite was added to the reaction solution and the mixture was extracted once with chloroform. The organic layer was washed once with an aqueous solution of sodium bicarbonate, once with water and once with an aqueous saturated solution of sodium chloride and dried over anhydrous sodium sulfate. The solvent was distilled off under reduced pressure and the residue was purified by silica gel column chromatography (chloroform:methanol=100:1) to give 200 mg of 6,8-difluoro-2-(3-fluoro-4-pivaloylaminophenyl)-7-methylsulfinyl-5-pivaloylamino-4H-1-benzopyran-4-one (yield: 95%).

NMR (90MHz, $CDCl_3$) δ (ppm) 1.36 (s, 9H), 1.38 (s, 9H), 3.23 (s, 3H), 6.71 (s, 1H), 7.5-7.9 (m, 3H), 8.60 (t, 1H, J=8.4Hz)
EIMS (M/Z) 536 ($M^+$)
Molecular formula $C_{26}H_{27}F_3N_2O_5S$=536

(2) Substantially the same manner as that in Example 8 (2) was repeated except that 170 mg (0.317 mmol) of the above 6,8-difluoro-2-(3-fluoro-4-pivaloylaminophenyl)-7-methylsulfinyl-5-pivaloylamino-4H-1-benzopyran-4-one was used, and the resulting crystals were triturated with ethyl acetate, to give 104 mg of Compound 11 (yield: 89%).

NMR (270MHz, DMSO-$d_6$) δ (ppm) 3.19 (s, 3H), 6.16 (brs, 2H), 6.76 (s, 1H), 6.87 (t, 1H, J=8.7Hz), 7.31 (brs, 2H), 7.62 (dd, 1H, J=8.4, 2.0Hz), 7.68 (dd, 1H, J=12.9, 2.0Hz)
FAB-MS (M/Z) 369 ($M^+$+H)
Molecular formula $C_{16}H_{11}F_3N_2O_3S$=368

Example 19

5-Amino-2-(4-amino-3-fluorophenyl)-6,8-difluoro-7-methylsulfonyl-4H-1-benzopyran-4-one (Compound 12)

(1) 203 mg (0.391 mmol) of 6,8-difluoro-2-(3-fluoro-4-pivaloylaminophenyl)-7-methylthio-5-pivaloylamino-4H-1-benzopyran-4-one obtained in Example 6 (2) was dissolved in 5 mL of dichloromethane, 850 mg (3.91 mmol) of m-chloroperbenzoic acid was added under ice-cooling and the mixture was stirred at room temperature for 3 hours. An aqueous solution of sodium hydrogensulfite was added to the reaction solution and the mixture was extracted once with chloroform. The organic layer was washed once with an aqueous solution of sodium bicarbonate, once with water and once with an aqueous saturated solution of sodium chloride and dried over anhydrous sodium sulfate. The solvent was distilled off under reduced pressure and the residue was purified by silica gel column chromatography (chloroform:ethyl acetate=40:1) to give 204 mg of 6,8-difluoro-2-(3-fluoro-4-pivaloylaminophenyl)-7-methylsulfonyl-5-pivaloylamino-4H-1-benzopyran-4-one (yield: 94%).

NMR (90MHz, $CDCl_3$) δ (ppm) 1.36 (s, 9H), 1.38 (s, 9H), 3.43 (s, 3H), 6.74 (s, 1H), 7.5-7.9 (m, 3H), 8.64 (t, 1H, J=8.4Hz)
EIMS (M/Z) 552 ($M^+$)
Molecular formula $C_{26}H_{27}F_3N_2O_6S$=552

(2) Substantially the same manner as that in Example 8 (2) was repeated except that 176 mg (0.319 mmol) of the above 6,8-difluoro-2-(3-fluoro-4-pivaloylaminophenyl)-7-methylsulfonyl-5-pivaloylamino-4H-1-benzopyran-4-one was used, and the resulting crystals were triturated with ethyl acetate, to give 78 mg of Compound 12 (yield: 64%).

NMR (270MHz, DMSO-$d_6$) δ (ppm) 3.50 (s, 3H), 6.19 (brs, 2H), 6.80 (s, 1H), 6.84 (t, 1H, J=9.4Hz), 7.39 (brs, 2H), 7.62 (dd, 1H, J=8.4, 2.0Hz), 7.70 (dd, 1H, J=12.9, 2.0Hz)
FAB-MS (M/Z) 385 ($M^+$+H)
Molecular formula $C_{16}H_{11}F_3N_2O_4$=384

Example 20

5-Amino-2-(4-amino-3-fluorophenyl)-7-carboxy-6,8-difluoro-4H-1-benzopyran-4-one (Compound 14)

121 mg (0.320 mmol) of Compound 13 obtained in Example 13 (2) was suspended in a mixed solvent of 10 mL of ethanol and 5 mL of methanol, 0.4 mL of 5N aqueous solution of sodium hydroxide was added and the mixture was stirred at 50 °C for 2.5 hours. The reaction solution was cooled on ice, the solution was adjusted to pH 4 and the precipitated crystals were collected by filtration to give 101 mg of Compound 14 (yield: 90%).

NMR (270MHz, DMSO-$d_6$) δ (ppm) 6.14 (brs, 2H), 6.74 (s, 1H), 6.87 (t, 1H, J=8.9Hz), 7.20 (brs, 2H), 7.60 (dd, 1H, J=8.4, 2.0Hz), 7.67 (dd, 1H, J=13.4, 2.0Hz)
FAB-MS (M/Z) 351 ($M^+$+H)
Molecular formula $C_{16}H_9F_3N_2O_4$=350

Example 21

5,7-Diamino-2-(4-amino-3-fluorophenyl)-6,8-difluoro-4H-1-benzopyran-4-one (Compound 16)

(1) 3.50 g (6.80 mmol) of 7-azido-6,8-difluoro-2-(3-fluoro-4-pivaloylaminophenyl)-5-pivaloylamino-4H-1-benzopyran-4-one obtained in Example 14 (1) was suspended in 120 mL of tetrahydrofuran, 1.96 g (7.48 mmol) of triphenylphosphine was added and the mixture was stirred at room temperature for 2 hours. To this was added 50 mL of 1N hydrochloric acid and the mixture was further stirred for 10 hours. The mixture was adjusted to pH 9 by addition of 10N aqueous solution of sodium hydroxide thereto, and the precipitated crystals were collected by filtration and purified by silica gel column chromatography (chloroform-chloroform:methanol=40:1) to give 2.83 g of 7-amino-6,8-difluoro-2-(3-fluoro-4-pivaloylaminophenyl)-5-pivaloylamino-4H-1-benzopyran-4-one (yield: 85%).

NMR (90MHz, DMSO-$d_6$) δ (ppm) 1.27 (s, 18H), 6.58 (brs, 2H), 6.87 (s, 1H), 7.7-8.0 (m, 3H), 9.18 (brs, 1H), 10.4 (brs, 1H)
EIMS (M/Z) 489 ($M^+$)
Molecular formula $C_{25}H_{26}F_3N_3O_4$=489

(2) Substantially the same manner as that in Example 6 (3) was repeated except that 404 mg (0.826 mmol) of the above 7-amino-6,8-difluoro-2-(3-fluoro-4-pivaloylaminophenyl)-5-pivaloylamino-4H-1-benzopyran-4-one was used, and the resulting compound was purified by silica gel column chromatography (chloroform:methanol=100:1) and recrystallized from ethyl acetate/n-hexane, to give 183 mg of Compound 16 (yield: 69%).

NMR (270MHz, DMSO-$d_6$) δ (ppm) 5.99 (brs, 2H), 6.20 (brs, 2H), 6.48 (s, 1H), 6.82 (brs, 2H), 6.85 (t, 1H, J=8.4Hz), 7.54 (dd, 1H, J=8.4, 2.0Hz), 7.60 (dd, 1H, J=12.9, 2.0Hz)
FAB-MS (M/Z) 322 ($M^+$+H)
Molecular formula $C_{15}H_{10}F_3N_3O_2$=321

Example 22

5-Amino-2-(4-amino-3-fluorophenyl)-7-dimethylamino-6,8-difluoro-4H-1-benzopyran-4-one (Compound 17)

(1) 510 mg (1.04 mmol) of 7-amino-6,8-difluoro-2-(3-fluoro-4-pivaloylaminophenyl)-5-pivaloylamino-4H-1-benzopyran-4-one obtained in Example 21 (1) was dissolved in 15 mL of dimethylformamide under argon atmosphere, 212 mg (5.30 mmol) of sodium hydride (60% oil suspension) and 0.17 mL (2.6 mmol) of iodomethane were added under ice-cooling and the mixture was stirred at the same temperature for 2 hours. Water was added to the reaction solution and the mixture was extracted twice with chloroform. The organic layer was washed once with water and once with an aqueous saturated solution of sodium chloride and dried over anhydrous sodium sulfate. The solvent was distilled off under reduced pressure and the residue was purified by silica gel column chromatography (chloroform:acetone=100:1) to give 290 mg of 7-dimethylamino-6,8-difluoro-2-(3-fluoro-4-pivaloylaminophenyl)-5-pivaloylamino-4H-1-benzopyran-4-one (yield: 54%).

NMR (90MHz, $CDCl_3$) δ (ppm) 1.36 (s, 9H), 1.38 (s, 9H), 3.12 (t, 3H, J=2.6Hz), 6.56 (s, 1H), 6.4-6.8 (m, 2H), 6.88 (brd, 1H), 8.47 (t, 1H, J=8.4Hz), 10.6 (brs, 1H)
EIMS (M/Z) 517 ($M^+$)
Molecular formula $C_{27}H_{30}F_3N_3O_4$=517

(2) substantially the same manner as that in Example 6 (3) was repeated except that 290 mg (0.561 mmol) of the above 7-dimethylamino-6,8-difluoro-2-(3-fluoro-4-pivaloylaminophenyl)-5-pivaloylamino-4H-1-benzopyran-4-one was used, and the resulting compound was purified by silica gel column chromatography (chloroform:acetone=70:1) and recrystallized from ethyl acetate/n-hexane, to give 66 mg of Compound 17 (yield: 34%).

NMR (270MHz, DMSO-$d_6$) δ (ppm) 3.00 (t, 6H, J=2.5Hz), 6.00 (brs, 2H), 6.56 (s, 1H), 6.86 (t, 1H, J=8.4Hz), 6.89 (brs, 2H), 7.56 (dd, 1H, J=8.4, 2.0Hz), 7.61 (dd, 1H, J=12.9, 2.0Hz)
EIMS (M/Z) 349 ($M^+$)
Molecular formula $C_{17}H_{14}F_3N_3O_2$=349

Example 23

5-Amino-2-(4-amino-3-fluorophenyl)-7-[3-(dimethylamino)propylamino]-6,8-difluoro-4H-1-benzopyran-4-one (Compound 18)

(1) 1.17 g (2.39 mmol) of 7-amino-6,8-difluoro-2-(3-fluoro-4-pivaloylaminophenyl)-5-pivaloylamino-4H-1-benzopyran-4-one obtained in Example 21 (1) was dissolved in 40 mL of pyridine, 2.3 mL (24 mmol) of acetic anhydride and 30 mg (0.24 mmol) of N,N-dimethylaminopyridine were added and the mixture was stirred at 50 °C for 18 hours. The reaction solution was poured into ice water and the precipitated crystals were collected by filtration.

The above crystals were dissolved in 15 mL of dimethylformamide, 0.94 g of potassium carbonate and 2.2 mL of 1-chloro-3-iodopropane were added and the mixture was stirred at 50 °C for 9 hours. Water was added to the reaction solution and the mixture was extracted once with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (chloroform) to give 1.16 g of 7-[N-acetyl-N-(3-chloropropyl)amino]-6,8-difluoro-2-(3-fluoro-4-pivaloylaminophenyl)-5-pivaloylamino-4H-1-benzopyran-4-one (yield: 80%).

NMR (270MHz, $CDCl_3$) δ (ppm) 1.37 (s, 9H), 1.39 (s, 9H), 2.0-2.2 (m, 2H), 2.03 (s, 3H), 3.60 (t, 2H, J=6.7Hz), 3.8-4.0 (m, 2H), 6.73 (s, 1H), 7.6-7.8 (m, 2H), 7.74 (brd, 1H, J=3.0Hz), 8.65 (t, 1H, J=8.4Hz), 10.7 (brs, 1H)
EIMS (M/Z) 607 ($M^+$)
Molecular formula $C_{30}H_{33}{}^{35}ClF_3N_3O_5$=607

(2) 932 mg (1.54 mmol) of the above 7-[N-acetyl-N-(3-chloropropyl)amino]-6,8-difluoro-2-(3-fluoro-4-pivaloylaminophenyl)-5-pivaloylamino-4H-1-benzopyran-4-one was dissolved in 10 mL of dimethylformamide under argon atmosphere, 320 mg (7.68 mmol) of sodium iodide, 1.25 g (15.4 mmol) of dimethylamine hydrochloride and 2.12 g (15.4 mmol) of potassium carbonate were added and the mixture was stirred at 70 °C for 23 hours. Water was added to the reaction solution and the mixture was extracted twice with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (chloroform-chloroform:methanol:aqueous ammonia=40:1:1) to give 603 mg of 7-[N-acetyl-N-(3-dimethylaminopropyl)amino]-6,8-difluoro-2-(3-fluoro-4-pivaloylaminophenyl)-5-pivaloylamino-4H-1-benzopyran-4-one (yield: 64%).

NMR (90MHz, $CDCl_3$) δ (ppm) 1.36 (s, 9H), 1.38 (s, 9H), 1.6-2.5 (m, 4H), 2.01 (s, 3H), 2.20 (s, 6H), 6.71 (s, 1H), 7.5-7.9 (m, 3H), 8.63 (t, 1H, J=8.4Hz), 10.6 (brs, 1H)
FAB-MS (M/Z) 617 ($M^+$+H)
Molecular formula $C_{32}H_{39}F_3N_4O_5$=616

(3) 549 mg (0.890 mmol) of the above 7-[N-acetyl-N-(3-dimethylaminopropyl)amino]-6,8-difluoro-2-(3-fluoro-4-pivaloylaminophenyl)-5-pivaloylamino-4H-1-benzopyran-4-one was dissolved in 15 mL of concentrated sulfuric acid and the solution was stirred at 50 °C for 10 minutes. The reaction solution was poured into ice water, the mixture was adjusted to pH 8 and extracted once with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (chloroform:methanol:aqueous ammonia=20:1:1) to give 297 mg of Compound 18 (yield: 82%). This compound was converted into hydrochloride according to the same manner as that in Example 17 (2).

NMR (270MHz, $CDCl_3$) δ (ppm) 1.80 (quint., 2H, J=6.4Hz), 2.27 (s, 6H), 2.44 (t, 2H, J=6.4Hz), 3.62 (m, 2H), 4.17 (brs, 2H), 5.74 (brs, 1H), 6.07 (brs, 2H), 6.37 (s, 1H), 6.82 (t, 1H, J=8.4Hz), 7.4-7.6 (m, 2H) (free base)
EIMS (M/Z) 406 ($M^+$)
Molecular formula $C_{20}H_{21}F_3N_4O_2$=406

Example 24

5-Amino-2-(4-amino-3-fluorophenyl)-6,8-difluoro-7-(4-methylpiperazin-1-yl)-4H-1-benzopyran-4-one (Compound 19)

(1) 580 mg (1.05 mmol) of 7-bromo-6,8-difluoro-2-(3-fluoro-4-pivaloylaminophenyl)-5-pivaloylamino-4H-1-benzopyran-4-one obtained in Example 5 (2) was dissolved in 6 mL of dimethyl sulfoxide, 1.2 mL (10.5 mmol) of N-methylpiperazine was added and the mixture was stirred at 100 °C for 7 hours. Water was added to the reaction solution and the mixture was extracted twice with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (chloroform:methanol=20:1) to give 350 mg of 6,8-difluoro-2-(3-fluoro-4-pivaloylaminophenyl)-7-(4-methylpiperazin-1-yl)-5-pivaloylamino-4H-1-benzopyran-4-one (yield: 58%).

NMR (90MHz, $CDCl_3$) δ (ppm) 1.36 (s, 9H), 1.38 (s, 9H), 2.38 (s, 3H), 2.5-2.7 (m, 4H), 3.4-3.6 (m, 4H), 6.58 (s, 1H), 7.5-7.9 (m, 3H), 8.58 (t, 1H, J=8.8Hz), 10.6 (brs, 1H)
EIMS (M/Z) 572 ($M^+$)
Molecular formula $C_{30}H_{35}F_3N_4O_4$=572

(2) 340 mg (0.594 mmol) of the above 6,8-difluoro-2-(3-fluoro-4-pivaloylaminophenyl)-7-(4-methylpiperazin-1-yl)-5-pivaloylamino-4H-1-benzopyran-4-one was dissolved in 20 mL of 1,4-dioxane, 10 mL of concentrated hydrochloric

acid was added and the mixture was heated at reflux for 2.5 hours. The reaction solution was cooled on ice, adjusted to pH 10 and extracted twice with chloroform (containing a small amount of methanol). The organic layer was washed once with an aqueous saturated solution of sodium chloride and dried over anhydrous sodium sulfate. The solvent was distilled off under reduced pressure and the residue was purified by silica gel column chromatography (chloroform:methanol=12:1) to give 117 mg of Compound 19 (yield: 49%).

NMR (270MHz, DMSO-$d_6$) δ (ppm) 2.23 (s, 3H), 2.4-2.5 (m, 4H), 3.2-3.4 (m, 4H), 6.05 (brs, 2H), 6.59 (s, 1H), 6,86 (t, 1H, J=8.9Hz), 6.92 (brs, 2H), 7.5-7.7 (m, 2H)
EIMS (M/Z) 404 ($M^+$)
Molecular formula $C_{20}H_{19}F_3N_4O_2$=404

Example 25

5-Amino-2-(4-amino-3-fluorophenyl)-6,8-difluoro-7-vinyl-4H-1-benzopyran-4-one (Compound 21)

Substantially the same manner as that in Example 13 (2) was repeated except that 205 mg (0.396 mmol) of 6,8-difluoro-2-(3-fluoro-4-pivaloylaminophenyl)-7-(1-hydroxyethyl)-5-pivaloylamino-4H-1-benzopyran-4-one was used, and the resulting compound was purified by preparative thin layer chromatography (chloroform:methanol=20:1), to give 18 mg of Compound 21 (yield: 14%).

NMR (270MHz, $CDCl_3$) δ (ppm) 4.17 (brs, 2H), 5.77 (dd, 1H, J=11.9, 1.0Hz), 6.17 (brs, 2H), 6.22 (d, 1H, J=17.8Hz), 6.47 (s, 1H), 6.81 (dd, 1H, J=17.8, 11.9Hz), 6.84 (t, 1H, J=8.7Hz), 7.5-7.7 (m, 2H)
EIMS (M/Z) 332 ($M^+$)
Molecular formula $C_{17}H_{11}F_3N_2O_2$=332

Example 26

7-Acetyl-5-amino-2-(4-amino-3-fluorophenyl)-6,8-difluoro-4H-1-benzopyran-4-one (Compound 22)

(1) 206 mg (0.398 mmol) of 6,8-difluoro-2-(3-fluoro-4-pivaloylaminophenyl)-7-(1-hydroxyethyl)-5-pivaloylamino-4H-1-benzopyran-4-one obtained in Example 15 (1) was suspended in 20 mL of toluene, 348 mg (3.98 mmol) of manganese dioxide was added and the mixture was heated at reflux for 2 hours. The reaction solution was filtered, the solvent was distilled off under reduced pressure and the residue was purified by silica gel column chromatography (chloroform:acetonitrile=50:1) to give 179 mg of 7-acetyl-6,8-difluoro-2-(3-fluoro-4-pivaloylaminophenyl)-5-pivaloylamino-4H-1-benzopyran-4-one (yield: 87%).

NMR (90MHz, $CDCl_3$) δ (ppm) 1.36 (s, 9H), 1.38 (s, 9H), 2.69 (brs, 3H), 6.70 (s, 1H), 7.5-7.9 (m, 3H), 8.62 (dd, 1H, J=8.7, 8.1Hz), 10.5 (brs, 1H)
FAB-MS (M/Z) 517 ($M^+$+H)
Molecular formula $C_{27}H_{27}F_3N_2O_5$=516

(2) Substantially the same manner as that in Example 6 (3) was repeated except that 158 mg (0.305 mmol) of the above 7-acetyl-6,8-difluoro-2-(3-fluoro-4-pivaloylaminophenyl)-5-pivaloylamino-4H-1-benzopyran-4-one was used, and the resulting compound was purified by silica gel column chromatography (chloroform:methanol=40:1) and triturated with isopropyl ether, to give 73 mg of Compound 22 (yield: 69%).

NMR (270MHz, DMSO-$d_6$) δ (ppm) 2.63 (s, 3H), 6.14 (brs, 2H), 6.74 (s, 1H), 6.87 (t, 1H, J=8.7Hz), 7.24 (brs, 2H), 7.60 (dd, 1H, J=8.9, 2.0Hz), 7.67 (dd, 1H, J=12.9, 2.0Hz)
EIMS (M/Z) 348 ($M^+$)
Molecular formula $C_{17}H_{11}F_3N_2O_3$=348

Example 27

2-(4-Amino-3-fluorophenyl)-7-dimethylamino-6,8-difluoro-5-methylamino-4H-1-benzopyran-4-one (Compound 23)

(1) 1.47 g (3.00 mmol) of 7-amino-6,8-difluoro-2-(3-fluoro-4-pivaloylaminophenyl)-5-pivaloylamino-4H-1-benzopyran-4-one obtained in Example 21 (1) was dissolved in 40 mL of dimethylformamide under argon atmosphere, 600 mg (15.0 mmol) of sodium hydride (60% oil dispersion) and 0.41 mL (6.6 mmol) of iodomethane were added

under ice-cooling and the mixture was stirred at the same temperature for 2 hours. Water was added to the reaction solution and the mixture was extracted twice with chloroform. The organic layer was washed once with water and once with an aqueous saturated solution of sodium chloride and dried over anhydrous sodium sulfate. The solvent was distilled off under reduced pressure and the residue was purified by silica gel column chromatography (chloroform:acetone=30:1) to give 790 mg of 7-dimethylamino-6,8-difluoro-2-(3-fluoro-4-pivaloylaminophenyl)-5-(N-methyl-N-pivaloylamino)-4H-1-benzopyran-4-one (yield: 50%).

(2) Substantially the same manner as that in Example 6 (3) was repeated except that 605 mg (1.14 mmol) of the above 7-dimethylamino-6,8-difluoro-2-(3-fluoro-4-pivaloylaminophenyl)-5-(N-methyl-N-pivaloylamino)-4H-1-benzopyran-4-one was used, and the resulting compound was purified by silica gel column chromatography (chloroform) and recrystallized from ethyl acetate/n-hexane, to give 306 mg of Compound 23 (yield: 74%).

NMR (270MHz, $CDCl_3$) δ (ppm) 3.05 (t, 6H, J=2.5Hz), 3.09 (dd, 3H, J=6.2, 5.9Hz), 4.12 (brs, 2H), 6.38 (s, 1H), 6.82 (t, 1H, J=8.7Hz), 7.4-7.6 (m, 2H)
EIMS (M/Z) 363 ($M^+$)
Molecular formula $C_{18}H_{16}F_3N_3O_2$=363

Example 28

2-(4-Amino-3-fluorophenyl)-6,8-difluoro-5-(1-hexylamino)-7-methyl-4H-1-benzopyran-4-one (Compound 28)

(1) 1.21 g (3.77 mmol) of Compound 1 obtained in Example 1 (3) was dissolved in 10 mL of pyridine, 0.46 mL (3.77 mmol) of pivaloyl chloride was added under ice-cooling and the mixture was stirred at the same temperature for 1 hour. The reaction solution was poured into ice water and the precipitated crystals were colleted by filtration to give 1.48 g of 5-amino-6,8-difluoro-2-(3-fluoro-4-pivaloylaminophenyl)-7-methyl-4H-1-benzopyran-4-one (yield: 97%).

NMR (90MHz, $CDCl_3$) δ (ppm) 1.36 (s, 9H), 2.34 (t, 3H, J=2.1Hz), 4.21 (brs, 2H), 6.55 (s, 1H), 7.5-7.9 (m, 3H), 8.57 (t, 1H, J=8.6Hz)
EIMS (M/Z) 404 ($M^+$)
Molecular formula $C_{21}H_{19}F_3N_2O_3$=404

(2) 653 mg (1.62 mmol) of the above 5-amino-6,8-difluoro-2-(3-fluoro-4-pivaloylaminophenyl)-7-methyl-4H-1-benzopyran-4-one was dissolved in 20 mL of dimethylformamide under argon atmosphere, 200 mg (5.00 mmol) of sodium hydride (60% oil dispersion) and 0.48 mL (3.2 mmol) of 1-iodohexane were added under ice-cooling and the mixture was stirred at room temperature for 20 hours. Water was added to the reaction solution and the mixture was extracted twice with ethyl acetate. The organic layer was washed once with water and once with an aqueous saturated solution of sodium chloride and dried over anhydrous sodium sulfate. The solvent was distilled off under reduced pressure and the residue was purified by medium pressure liquid chromatography to give 422 mg of 6,8-difluoro-2-(3-fluoro-4-pivaloylaminophenyl)-5-(1-hexylamino)-7-methyl-4H-1-benzopyran-4-one (yield: 54%).

NMR (90MHz, $CDCl_3$) δ (ppm) 0.8-1.0 (m, 3H), 1.1-1.9 (m, 8H), 1.36 (s, 9H), 2.31 (t, 3H, J=2.2Hz), 3.48 (m, 2H), 6.52 (s, 1H), 7.5-7.9 (m, 3H), 8.57 (t, 1H, J=8.6Hz), 8.66 (brs, 1H)
EIMS (M/Z) 488 ($M^+$)
Molecular formula $C_{27}H_{31}F_3N_2O_3$=488

(3) Substantially the same manner as that in Example 6 (3) was repeated except that 405 mg (0.830 mmol) of the above 6,8-difluoro-2-(3-fluoro-4-pivaloylaminophenyl)-5-(1-hexylamino)-7-methyl-4H-1-benzopyran-4-one was used, and the resulting compound was purified by silica gel column chromatography (chloroform), to give 270 mg of Compound 28 (yield: 81%).

NMR (270MHz, $CDCl_3$) δ (ppm) 0.89 (t, 3H, J=7.2Hz), 1.2-1.5 (m, 6H), 1.5-1.7 (m, 2H), 2.30 (t, 3H, J=2.5Hz), 3.4-3.5 (m, 2H), 4.15 (brs, 2H), 6.42 (s, 1H), 6.83 (t, 1H, J=8.9Hz), 7.3-7.5 (m, 2H), 8.70 (brs, 1H)
EIMS (M/Z) 404 ($M^+$)
Molecular formula $C_{22}H_{23}F_3N_2O_2$=404

Example 29

2-(4-Amino-3-fluorophenyl)-6,8-difluoro-5-(1-heptylamino)-7-methyl-4H-1-benzopyran-4-one (compound 29)

(1) Substantially the same manner as that in Example 28 (2) was repeated except that 650 mg (1.61 mmol) of 5-amino-6,8-difluoro-2-(3-fluoro-4-pivaloylaminophenyl)-7-methyl-4H-1-benzopyran-4-one obtained in Example 28 (1) and 0.53 mL (3.22 mol) of 1-iodoheptane were used, to give 369 mg of 6,8-difluoro-2-(3-fluoro-4-pivaloylaminophenyl)-5-(1-heptylamino)-7-methyl-4H-1-benzopyran-4-one (yield: 46%).

NMR (90MHz, $CDCl_3$) δ (ppm) 0.7-1.8 (m, 13H), 1.36 (s, 9H), 2.31 (t, 3H, J=2.2Hz), 6.51 (s, 1H), 7.4-7.8 (m, 3H), 8.57 (t, 1H, J=8.6Hz), 8.64 (brs, 1H)
EIMS (M/Z) 502 ($M^+$)
Molecular formula $C_{28}H_{33}F_3N_2O_3$=502

(2) Substantially the same manner as that in Example 6 (3) was repeated except that 351 mg (0.699 mmol) of the above 6,8-difluoro-2-(3-fluoro-4-pivaloylaminophenyl)-5-(1-heptylamino)-7-methyl-4H-1-benzopyran-4-one was used, and the resulting compound was purified by silica gel column chromatography (chloroform), to give 250 mg of Compound 29 (yield: 86%).

NMR (270MHz, $CDCl_3$) δ (ppm) 0.88 (t, 3H, J=6.9Hz), 1.2-1.5 (m, 8H), 1.5-1.7 (m, 2H), 2.30 (t, 3H, J=2.5Hz), 3.4-3.5 (m, 2H), 4.15 (brs, 2H), 6.42 (s, 1H), 6.83 (t, 1H, J=8.7Hz), 7.4-7.6 (m, 2H), 8.69 (brs, 1H)
EIMS (M/Z) 418 ($M^+$)
Molecular formula $C_{23}H_{25}F_3N_2O_2$=418

Example 30

6,8-Difluoro-2-[3-fluoro-4-(1-propylamino)phenyl]-7-methyl-5-(1-pentylamino)-4H-1-benzopyran-4-one (Compound 30)

(1) 2.68 g (8.38 mmol) of Compound 1 obtained in Example 1 (3) was dissolved in 30 mL of pyridine, 0.88 mL (9.2 mmol) of acetic anhydride was added under ice-cooling and the mixture was stirred at 50 °C for 18 hours. The reaction solution was poured into ice water and the precipitated crystals were collected by filtration to give 3.03 g of 2-(4-acetylamino-3-fluorophenyl)-5-amino-6,8-difluoro-7-methyl-4H-1-benzopyran-4-one (yield: 100%).

NMR (90MHz, DMSO-$d_6$) δ (ppm) 2.15 (s, 3H), 2.28 (t, 3H, J=2.2Hz), 6.86 (s, 1H), 6.95 (brs, 2H), 7.6-8.0 (m, 2H), 8.25 (t, 1H, J=8.8Hz), 9.98 (brs, 1H)
EIMS (M/Z) 362 ($M^+$)
Molecular formula $C_{18}H_{13}F_3N_2O_3$=362

(2) 506 mg (1.40 mmol) of the above 2-(4-acetylamino-3-fluorophenyl)-5-amino-6,8-difluoro-7-methyl-4H-1-benzopyran-4-one was dissolved in 30 mL of dimethylformamide under argon atmosphere, 60 mg (1.5 mmol) of sodium hydride (60% oil dispersion) and 0.28 mL (2.8 mmol) of 1-iodopropane were added thereto under ice-cooling and the mixture was stirred at room temperature for 6 hours. Water was added to the reaction solution and the mixture was extracted twice with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (chloroform) to give 315 mg of 2-[4-[N-acetyl-N-(1-propyl)amino]-3-fluorophenyl]-5-amino-6,8-difluoro-7-methyl-4H-1-benzopyran-4-one (yield: 56%).

NMR (90MHz, $CDCl_3$) δ (ppm) 0.90 (t, 3H, J=7.3Hz), 1.50 (quint., 2H, J=7.5Hz), 1.90 (s, 3H), 2.35 (t, 3H, J=2.2Hz), 3.68 (t, 3H, J=7.5Hz), 6.11 (brs, 2H), 6.61 (s, 1H), 7.37 (t, 1H, J=8.4Hz), 7.6-7.8 (m, 2H)
EIMS (M/Z) 404 ($M^+$)
Molecular formula $C_{21}H_{19}F_3N_2O_3$=404

(3) 299 mg (0.740 mmol) of the above 2-[4-[N-acetyl-N-(1-propyl)amino]-3-fluorophenyl]-5-amino-6,8-difluoro-7-methyl-4H-1-benzopyran-4-one was dissolved in 10 mL of dimethylformamide under argon atmosphere, 60 mg (1.5 mmol) of sodium hydride (60% oil dispersion) and 0.98 mL (7.4 mmol) of 1-iodopentane were added under ice-cooling and the mixture was stirred at room temperature for 6 hours. Water was added to the reaction solution and the mixture was extracted once with ethyl acetate. The organic layer was washed once with water and once with an

aqueous saturated solution of sodium chloride and dried over anhydrous sodium sulfate. The solvent was distilled off under reduced pressure and the residue was purified by silica gel column chromatography (chloroform) to give 91.2 mg of 2[4-[N-acetyl-N-(1-propyl)amino]-3-fluorophenyl]-6,8-difluoro-7-methyl-5-(1-pentylamino)-4H-1-benzopyran-4-one (yield: 26%).

NMR (90MHz, $CDCl_3$) δ (ppm) 0.7-2.0 (m, 14H), 1.90 (s, 3H), 2.33 (t, 3H, J=2.2Hz), 3.3-3.8 (m, 4H), 6.58 (s, 1H), 7.34 (t, 1H, J=8.4Hz), 7.6-7.8 (m, 2H)
EIMS (M/Z) 474 ($M^+$)
Molecular formula $C_{26}H_{29}F_3N_2O_3$=474

(4) Substantially the same manner as that in Example 13 (2) was repeated except that 77 mg (0.16 mmol) of the above 2-[4-[N-acetyl-N-(1-propyl)amino]-3-fluorophenyl]-6,8-difluoro-7-methyl-5-(1-pentylamino)-4H-1-benzopyran-4-onewas used, and the resulting compound was purified by silica gel column chromatography (n-hexane:ethyl acetate=5:1) and recrystallized from n-hexane, to give 36 mg of Compound 30 (yield: 51%).

NMR (270MHz, $CDCl_3$) δ (ppm) 0.91 (t, 3H, J=6.9Hz), 1.04 (t, 3H, 7.4Hz), 1.2-1.8 (m, 8H), 2.30 (t, 3H, J=2.5Hz), 3.20 (t, 2H, J=7.2Hz), 3.4-3.5 (m, 2H), 4.40 (brs, 1H), 6.42 (s, 1H), 6.73 (t, 1H, J=8.7Hz), 7.53 (dd, 1H, J=12.9, 2.0Hz), 7.59 (dd, 1H, J=8.9, 2.0Hz), 8.81 (brs, 1H)
FAB-MS (M/Z) 433 ($M^+$+H)
Molecular formula $C_{24}H_{27}F_3N_2O_2$=432

Example 31

2-[4-(1-Butylamino)-3-fluorophenyl]-6,8-difluoro-7-methyl-5-(1-pentylamino)-4H-1-benzopyran-4-one (Compound 31)

(1) Substantially the same manner as that in Example 30 (2) was repeated except that 0.33 mL (2.85 mmol) of 1-iodobutane was used in place of 1-iodopropane, to give 334 mg of 2-[4-[N-acetyl-N-(1-butyl)amino]-3-fluorophenyl]-5-amino-6,8-difluoro-7-methyl-4H-1-benzopyran-4-one (yield: 56%).

NMR (90MHz, $CDCl_3$) δ (ppm) 0.8-1.0 (m, 3H), 1.1-1.7 (m, 4H), 1.90 (s, 3H), 2.35 (t, 3H, J=2.2Hz), 3.6-3.9 (m, 2H), 6.15 (brs, 2H), 6.61 (s, 1H), 7.37 (t, 1H, J=8.4Hz), 7.6-7.8 (m, 2H)
EIMS (M/Z) 418 ($M^+$)
Molecular formula $C_{22}H_{21}F_3N_2O_3$=418

(2) Substantially the same manner as that in Example 30 (3) was repeated except that 319 mg (0.762 mmol) of the above 2-[4-[N-acetyl-N-(1-butyl)amino]-3-fluorophenyl]-5-amino-6,8-difluoro-7-methyl-4H-1-benzopyran-4-one was used, to give 105 mg of 2-[4-[N-acetyl-N-(1-butyl)amino]-3-fluorophenyl]-6,8-difluoro-7-methyl-5-(1-pentylamino)-4H-1-benzopyran-4-one (yield: 28%).

NMR (90MHz, $CDCl_3$) δ (ppm) 0.8-2.0 (m, 16H), 1.89 (s, 3H), 2.32 (t, 3H, J=2.2Hz), 3.3-3.8 (m, 4H), 6.58 (s, 1H), 7.36 (t, 1H, J=8.4Hz), 7.6-7.8 (m, 2H), 8.67 (brs, 1H)
EIMS (M/Z) 488 ($M^+$)
Molecular formula $C_{27}H_{31}F_3N_2O_3$=488

(3) Substantially the same manner as that in Example 13 (2) was repeated except that 92 mg (0.19 mmol) of the above 2-[4-[N-acetyl-N-(1-butyl)amino]-3-fluorophenyl]-6,8-difluoro-7-methyl-5-(1-pentylamino)-4H-1-benzopyran-4-one was used, and the resulting compound was purified by silica gel column chromatography (n-hexane/ethyl acetate=5:1) and recrystallized from n-hexane, to give 50 mg of Compound 31 (yield: 59%).

NMR (270MHz, $CDCl_3$) δ (ppm) 0.91 (t, 3H, J=6.9Hz), 0.98 (t, 3H, J=7.4Hz), 1.2-1.8 (m, 10H), 2.30 (t, 3H, J=2.2Hz), 3.23 (t, 2H, J=7.4Hz), 3.4-3.5 (m, 2H), 4.30 (brs, 1H), 6.42 (s, 1H), 6.72 (t, 1H, J=8.7Hz), 7.52 (dd, 1H, J=12.9, 2.0Hz), 7.59 (dd, 1H, J=8.4, 2.0Hz), 8.79 (brs, 1H)
FAB-MS (M/Z) 447 ($M^+$+H)
Molecular formula $C_{25}H_{29}F_3N_2O_2$=446

Example 32

6,8-Difluoro-2-[3-fluoro-4-(1-pentylamino)phenyl]-7-methyl-5-(1-pentylamino)-4H-1-benzopyran-4-one (Compound 32)

(1) 510 mg (1.41 mmol) of 2-(4-acetylamino-3-fluorophenyl)-5-amino-6,8-difluoro-7-methyl-4H-1-benzopyran-4-one obtained in Example 30 (1) was dissolved in 30 mL of dimethylformamide under argon atmosphere, 58 mg (1.5 mmol) of sodium hydride (60% oil dispersion) and 0.37 mL (2.8 mmol) of 1-iodopentane were added under ice-cooling and the mixture was stirred at room temperature for 4 hours. The reaction solution was cooled on ice, 117 mg (2.93 mmol) of sodium hydride (60% oil dispersion) and 0.37 mL (2.8 mmol) of 1-iodopentane were added and the mixture was stirred at room temperature for 50 minutes. An aqueous solution of ammonium chloride was added to the reaction solution and the mixture was extracted twice with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (chloroform) to give 153 mg of 2-[4-[N-acetyl-N-(1-pentyl)amino]-3-fluorophenyl)]-6,8-difluoro-7-methyl-5-(1-pentylamino)-4H-1-benzopyran-4-one (yield: 22%).

NMR (90MHz, $CDCl_3$) δ (ppm) 0.7-2.0 (m, 18H), 1.89 (s, 3H), 2.32 (t, 3H, J=2.2Hz), 3.3-3.8 (m, 4H), 6.58 (s, 1H), 7.36 (t, 1H, J=7.7Hz), 7.6-7.9 (m, 2H), 8.68 (brs, 1H)
EIMS (M/Z) 502 ($M^+$)
Molecular formula $C_{28}H_{33}F_3N_2O_3$=502

(2) Substantially the same manner as that in Example 13 (2) was repeated except that 122 mg (0.243 mmol) of the above 2-[4-[N-acetyl-N-(1-pentyl)amino]-3-fluorophenyl]-6,8-difluoro-7-methyl-5-(1-pentylamino)-4H-1-benzopyran-4-onewas used, and the resulting compound was purified by silica gel column chromatography (n-hexane/ethyl acetate) and recrystallized from n-hexane, to give 70 mg of Compound 32 (yield: 63%).

NMR (270MHz, $CDCl_3$) δ (ppm) 0.91 (t, 3H, J=6.9Hz), 0.94 (t, 3H, J=6.9Hz), 1.2-1.8 (m, 12H), 2.30 (t, 3H, J=2.5Hz), 3.22 (t, 3H, J=6.9Hz), 3.4-3.5 (m, 2H), 4.35 (brs, 1H), 6.42 (s, 1H), 6.72 (t, 1H, J=8.7Hz), 7.52 (dd, 1H, J=12.9, 2.0Hz), 7.59 (dd, 1H, J=8.4Hz), 8.82 (brs, 1H)
FAB-MS (M/Z) 461 ($M^+$+H)
Molecular formula $C_{26}H_{31}F_3N_2O_2$=460

Example 33

5-Amino-2-(4-amino-3-fluorophenyl)-7-ethynyl-6,8-difluoro-4H-1-benzopyran-4-one (Compound 33)

(1) 30 mg (0.054 mmol) of 7-bromo-6,8-difluoro-2-(3-fluoro-4-pivaloylaminophenyl)-5-pivaloylamino-4H-1-benzopyran-4-one obtained in Example 5 (2) was dissolved in a mixed solvent of 0.5 mL of dimethylformamide and 0.5 mL of triethylamine, 1 mg (0.004 mmol) of palladium (II) acetate, 2 mg (0.008 mmol) of triphenylphosphine and 38 μL (0.27 mmol) of trimethylsilylacetylene were added and the mixture was stirred at 50 °C for 2 hours. The reaction solution was cooled to room temperature, water was added thereto and the mixture was extracted once with ethyl acetate. The organic layer was washed twice with 2N hydrochloric acid, once with water and once with an aqueous saturated solution of sodium chloride and dried over anhydrous sodium sulfate. The solvent was distilled off under reduced pressure and the residue was purified by preparative thin layer chromatography (chloroform:acetonitrile=19:1) to give 12 mg of 6,8-difluoro-2-(3-fluoro-4-pivaloylaminophenyl)-5-pivaloylamino-7-(2-trimethylsilylethynyl)-4H-1-benzopyran-4-one (yield: 39%).

NMR (90MHz, $CDCl_3$) δ (ppm) 0.30 (s, 9H), 1.36 (s, 9H), 1.38 (s, 9H), 6.66 (s, 1H), 7.4-7.9 (m, 3H), 8.60 (t, 1H, J=8.1Hz)
FAB-MS (M/Z) 571 ($M^+$+H)
Molecular formula $C_{30}H_{33}F_3N_2O_4Si$=570

(2) 3 mL of concentrated sulfuric acid was added to 37 mg (0.065 mmol) of the above 6,8-difluoro-2-(3-fluoro-4-pivaloylaminophenyl)-5-pivaloylamino-7-(2-trimethylsilylethynyl)-4H-1-benzopyran-4-one and the mixture was stirred at 50 °C for 10 minutes. The reaction solution was poured into ice water and the mixture was extracted once with ethyl acetate. The organic layer was washed once with 1N aqueous solution of sodium hydroxide, once with water and once with an aqueous saturated solution of sodium chloride and dried over anhydrous sulfate. The solvent was distilled off under reduced pressure and the residue was purified by preparative thin layer chromatography

(chloroform:acetonitrile=9:1) to give 15 mg of Compound 33 (yield: 70%).

NMR (270MHz, DMSO-$d_6$) δ (ppm) 5.04 (s, 1H), 6.14 (brs, 2H), 6.73 (s, 1H), 6.87 (t, 1H, J=8.9Hz), 7.19 (brs, 2H), 7.60 (dd, 1H, J=8.4, 2.0Hz), 7.67 (dd, 1H, J=12.9, 2.0Hz)
EIMS (M/Z) 330 ($M^+$)
Molecular formula $C_{17}H_9F_3N_2O_2$=330

Example 34

5-Amino-2-(4-amino-3-fluorophenyl)-6,7,8-trifluoro-4H-1-benzopyran-4-one (Compound 34)

(1) 10 mL of thionyl chloride was added to 962 mg (4.88 mmol) of compound XV obtained in Reference Example 8 under argon atmosphere and the mixture was heated at reflux for 1 hour. Thionyl chloride was distilled off under reduced pressure, 15 mL of dichloromethane, a solution of 1.17 g (4.06 mmol) of compound XIV-1 obtained in Reference Example 7 in 20 mL of dichloromethane and 0.42 mL of triethylamine were added thereto and the mixture was stirred at room temperature for 2 hours. Water was added to the reaction solution, the organic layer was washed once with an aqueous saturated solution of sodium chloride and dried over anhydrous sodium sulfate. The solvent was distilled off under reduced pressure and the residue was purified by silica gel column chromatography (n-hexane:ethyl acetate=4:1-1:1) to give 1.21 g of 2-acetyl-4,5,6-trifluoro-3-pivaloylaminophenyl-4-acetylamino-3-fluorobenzoate (yield: 76%).

NMR (90MHz, $CDCl_3$) δ (ppm) 1.29 (s, 9H), 2.28 (s, 3H), 2.46 (s, 3H), 7.5-8.1 (m, 4H), 8.61 (dd, 1H, J=8.8, 7.5Hz)

(2) 0.83 mL (5.9 mmol) of diisopropylamine was dissolved in 10 mL of tetrahydrofuran under argon atmosphere, 3.5 mL of a 1.6 M solution of n-butyl lithium in n-hexane was added dropwise under ice-cooling and the solution was cooled to -78 °C. To this solution was added a solution of 838 mg (1.79 mmol) of the above 2-acetyl-4,5,6-trifluoro-3-pivaloylaminophenyl 4-acetylamino-3-fluorobenzoate in 20 mL of tetrahydrofuran was added dropwise and the mixture was stirred for 2 hours while the temperature of the mixture was gradually raised to 0 °C. An aqueous solution of ammonium chloride was added to the reaction solution, the mixture was extracted twice with ethyl acetate, the organic layer was washed once with an aqueous saturated solution of sodium chloride and dried over anhydrous sodium sulfate. The solvent was distilled off under reduced pressure, the residue was dissolved in 24 mL of ethanol, 6 mL of concentrated hydrochloric acid was added and the mixture was stirred at room temperature for 4 hours. Water was added to the reaction solution and the precipitated crystals were collected by filtration to give 330 mg of 2-(4-acetylamino-3-fluorophenyl)-6,7,8-trifluoro-5-pivaloylamino-4H-1-benzopyran-4-one(yield: 77%).

NMR (90MHz, DMSO-$d_6$) δ (ppm) 1.29 (s, 9H), 2.16 (s, 3H), 7.09 (s, 1H), 7.7-8.0 (m, 2H), 8.29 (t, 1H, J=8.4Hz), 10.0 (brs, 1H), 10.4 (brs, 1H)
EIMS (M/Z) 450 ($M^+$)
Molecular formula $C_{22}H_{18}F_4N_2O_4$=450

(3) Substantially the same manner as that in Example 6 (3) was repeated except that 366 mg (0.812 mmol) of the above 2-(4-acetylamino-3-fluorophenyl)-6,7,8-trifluoro-5-pivaloylamino-4H-1-benzopyran-4-one was used, and the resulting compound was purified by silica gel column chromatography (chloroform:ethyl acetate=20:1-10:1) and recrystallized from ethyl acetate/n-hexane, to give 200 mg of Compound 34 (yield: 76%).

NMR (270MHz, DMSO-$d_6$) δ (ppm) 6.11 (brs, 2H), 6.69 (s, 1H), 6.86 (t, 1H, J=8.9Hz), 7.40 (brs, 2H), 7.57 (dd, 1H, J=8.4, 2.4Hz), 7.64 (dd, 1H, J=12.9, 2.0Hz)
EIMS (M/Z) 324 ($M^+$)
Molecular formula $C_{15}H_8F_4N_2O_2$=324

Example 35

2-(4-Amino-3-fluorophenyl)-6,8-difluoro-7-methyl-5-(3-methylbutylamino)-4H-1-benzopyran-4-one (Compound 35)

(1) 1.03 g (2.54 mmol) of 5-amino-6,8-difluoro-2-(3-fluoro-4-pivaloylaminophenyl)-7-methyl-4H-1-benzopyran-4-one obtained in Example 28 (1) was dissolved in 20 mL of dimethylformamide under argon atmosphere, 308 mg (7.62 mmol) of sodium hydride (60% oil dispersion) and 0.77 mL (6.4 mmol) of 1-bromo-3-methylbutane were

added and the mixture was stirred at room temperature for 4 hours. Water was added to the reaction solution, the mixture was extracted once with ethyl acetate, the organic layer was washed once with an aqueous saturated solution of sodium chloride and dried over anhydrous sodium sulfate. The solvent was distilled off under reduced pressure and the residue was purified by silica gel column chromatography (chloroform:n-hexane=3:1) to give 708 mg of 6,8-difluoro-2-(3-fluoro-4-pivaloylaminophenyl)-7-methyl-5-(3-methylbutylamino)-4H-1-benzopyran-4-one (yield: 59%).

NMR (90MHz, $CDCl_3$) δ (ppm) 0.94 (d, 6H, J=6.2Hz), 1.35 (s, 9H), 1.4-1.8 (m, 2H), 2.31 (t, 3H, J=2.4Hz), 3.3-3.7 (m, 2H), 6.51 (s, 1H), 7.5-7.8 (m, 3H), 8.56 (t, 1H, J=8.6Hz), 8.5-8.7 (m, 1H)
EIMS (M/Z) 474 ($M^+$)
Molecular formula $C_{26}H_{29}F_3N_2O_3$=474

(2) Substantially the same manner as that in Example 6 (3) was repeated except that 692 mg (1.46 mmol) of the above 6,8-difluoro-2-(3-fluoro-4-pivaloylaminophenyl)-7-methyl-5-(3-methylbutylamino)-4H-1-benzopyran-4-one was used, and the resulting compound was purified by silica gel column chromatography (chloroform) and recrystallized from 2-propanol, to give 371 mg of Compound 35 (yield: 65%).

NMR (270MHz, $CDCl_3$) δ (ppm) 0.94 (d, 6H, J=6.4Hz), 1.53 (q, 2H, J=6.9Hz), 1.6-1.8 (m, 2H), 2.30 (t, 3H, J=2.2Hz), 3.4-3.5 (m, 2H), 4.16 (brs, 2H), 6.42 (s, 1H), 6.83 (t, 1H, J=8.6Hz), 7.4-7.6 (m, 2H), 8.6-8.7 (m, 1H)
EIMS (M/Z) 390 ($M^+$)
Molecular formula $C_{21}H_{21}F_3N_2O_2$=390

Example 36

2-(4-Amino-3-fluorophenyl)-6,8-difluoro-7-methyl-5-(4-methylpentylamino)-4H-1-benzopyran-4-one (Compound 36)

(1) Substantially the same manner as that in Example 35 (1) was repeated except that 1.02 g (2.54 mmol) of 5-amino-6,8-difluoro-2-(3-fluoro-4-pivaloylaminophenyl)-7-methyl-4H-1-benzopyran-4-one obtained in Example 28 (1), 306 mg (7.65 mmol) of sodium hydride (60% oil dispersion) and 0.90 mL (6.3 mmol) of 1-bromo-4-methylpentane were used under argon atmosphere, to give 718 mg of 6,8-difluoro-2-(3-fluoro-4-pivaloylaminophenyl)-7-methyl-5-(4-methylpentylamino)-4H-1-benzopyran-4-one (yield: 58%).

NMR (90MHz, $CDCl_3$) δ (ppm) 0.90 (d, 6H, J=6.2Hz), 1.3-1.9 (m, 4H), 1.36 (s, 9H), 2.31 (t, 3H, J=2.4Hz), 3.3-3.6 (m, 2H), 6.51 (s, 1H), 7.5-7.9 (m, 3H), 8.5-8.8 (m, 1H), 8.56 (t, 1H, J=8.5Hz)
MS (M/Z) 488 ($M^+$)
Molecular formula $C_{27}H_{31}F_3N_2O_3$=488

(2) Substantially the same manner as that in Example 6 (3) was repeated except that 685 mg (1.40 mmol) of the above 6,8-difluoro-2-(3-fluoro-4-pivaloylaminophenyl)-7-methyl-5-(4-methylpentylamino)-4H-1-benzopyran-4-one was used, and the resulting compound was purified by silica gel column chromatography (chloroform) and recrystallized from 2-propanol, to give 271 mg of Compound 36 (yield: 48%).

NMR (270MHz, $CDCl_3$) δ (ppm) 0.89 (d, 6H, J=6.4Hz), 1.2-1.4 (m, 2H), 1.5-1.7 (m, 3H), 2.30 (t, 3H, J=2.5Hz), 3.3-3.5 (m, 2H), 4.16 (brs, 2H), 6.43 (s, 1H), 6.83 (t, 1H, J=8.7Hz), 7.4-7.6 (m, 2H), 8.7-8.8 (m, 1H)
MS (M/Z) 404 ($M^+$)
Molecular formula $C_{22}H_{23}F_3N_2O_2$=404

Example 37

2-(4-Amino-3-fluorophenyl)-5-[3-(dimethylamino)propylamino]-6,8-difluoro-7-methyl-4H-1-benzopyran-4-one (Compound 37)

(1) 10.1 g (25.0 mmol) of 5-amino-6,8-difluoro-2-(3-fluoro-4-pivaloylaminophenyl)-7-methyl-4H-1-benzopyran-4-one obtained in Example 28 (1) was dissolved in 150 mL of dimethylformamide under argon atmosphere, a solution of 20.2 g (74.8 mmol) of 2-(3-iodopropyloxy)-3,4,5,6-tetrahydropyran in 10 mL of dimethylformamide and 2.00 g (50.0 mmol) of sodium hydride (60% oil dispersion) were added under ice-cooling and the mixture was stirred at room temperature for 7.3 hours. The reaction solution was cooled on ice, water was added, the solvent was distilled off under reduced pressure, water was further added and the mixture was extracted once with ethyl acetate. The

organic layer was washed once with water and once with an aqueous saturated solution of sodium chloride, dried over anhydrous sodium sulfate and the solvent was distilled off under reduced pressure. The residue was dissolved in 250 mL of ethanol, 500 mg (2.16 mmol) of dl-camphorsulfonic acid was added and the mixture was stirred at 50 °C for 7 hours. The solvent was distilled off under reduced pressure, water was added to the residue, the mixture was extracted twice with chloroform, the organic layer was washed once with an aqueous saturated solution of sodium chloride and dried over anhydrous sodium sulfate. The solvent was distilled off under reduced pressure and the residue was purified by silica gel column chromatography (chloroform:ethyl acetate=2:1) to give 3.00 g of 6,8-difluoro-2-(3-fluoro-4-pivaloylaminophenyl)-5-(3-hydroxypropylamino)-7-methyl-4H-1-benzopyran-4-one (yield: 30%).

NMR (90MHz, $CDCl_3$) δ (ppm) 1.36 (s, 9H), 1.92 (quint., 2H, J=6.8Hz), 2.32 (t, 3H, J=2.4Hz), 3.59 (td, 2H, J=6.8, 3.6Hz), 3.81 (t, 2H, J=6.8Hz), 6.53 (s, 1H), 7.5-7.8 (m, 3H), 8.57 (t, 1H, J=8.4Hz)
FAB-MS (M/Z) 463 ($M^+$+H)
Molecular formula $C_{24}H_{25}F_3N_2O_4$=462

(2) 3.96 g (8.57 mmol) of the above 6,8-difluoro-2-(3-fluoro-4-pivaloylaminophenyl)-5-(3-hydroxypropylamino)-7-methyl-4H-1-benzopyran-4-one was dissolved in 100 mL of pyridine under ice-cooling, 1.3 mL (17 mmol) of methanesulfonyl chloride was added and the mixture was stirred at the same temperature for 1 hour. Water was added to the reaction solution and the precipitated crystals were collected by filtration to give 4.62 g of 6,8-difluoro-2-(3-fluoro-4-pivaloylaminophenyl)-5-[3-(methylsulfonyloxy)propylamino]-7-methyl-4H-1-benzopyran-4-one (yield: 100%).

NMR (90MHz, $CDCl_3$) δ (ppm) 1.36 (s, 9H), 2.09 (quint., 2H, J=6.3Hz), 2.33 (t, 3H, J=2.3Hz), 3.04 (s, 3H), 3.3-3.7 (m, 2H), 4.37 (t, 2H, J=6.0Hz), 6.54 (s, 1H), 7.5-7.8 (m, 3H), 8.58 (t, 1H, J=8.4Hz)
EIMS (M/Z) 540 ($M^+$)
Molecular formula $C_{25}H_{27}F_3N_2O_6S$=540

(3) 1.02 g (1.89 mmol) of the above 6,8-difluoro-2-(3-fluoro-4-pivaloylaminophenyl)-5-[3-(methylsulfonyloxy)propylamino]-7-methyl-4H-1-benzopyran-4-one was dissolved in 30 mL of dimethylformamide, 1.54 g (18.9 mmol) of dimethylamine hydrochloride and 2.60 g (18.9 mmol) of potassium carbonate were added and the mixture was stirred at 50 °C for 24 hours. Water was added to the reaction solution and the mixture was extracted once with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (chloroform:methanol:=100:1-9:1) to give 660 mg of 6,8-difluoro-5-[3-(dimethylamino)propylamino]-2-(3-fluoro-4-pivaloylaminophenyl)-7-methyl-4H-1-benzopyran-4-one (yield: 71%).

NMR (90MHz, $CDCl_3$) δ (ppm) 1.35 (s, 9H), 1.7-1.9 (m, 2H), 2.29 (s, 9H), 2.3-2.6 (m, 2H), 3.4-3.7 (m, 2H), 6.52 (s, 1H), 7.5-7.7 (m, 3H), 8.57 (t, 1H, J=8.4Hz), 8.6-8.7 (m, 1H)
EIMS (M/Z) 489 ($M^+$)
Molecular formula $C_{26}H_{30}F_3N_3O_3$=489

(4) Substantially the same manner as that in Example 6 (3) was repeated except that 624 mg (1.28 mmol) of the above 6,8-difluoro-5-[3-(dimethylamino)propylamino]-2-(3-fluoro-4-pivaloylaminophenyl)-7-methyl-4H-1-benzopyran-4-one was used, and the resulting compound was purified by silica gel column chromatography (chloroform:methanol=9:1-chloroform:methanol:aqueous ammonia=20:1:1) and recrystallized from ethanol/n-hexane, to give 472 mg of Compound 37 (yield: 91%).

NMR (270MHz, $CDCl_3$) δ (ppm) 1.82 (quint., 2H, J=7.3Hz), 2.27 (s, 6H), 2.29 (t, 3H, J=2.3Hz), 2.42 (t, 2H, J=7.4Hz), 3.4-3.6 (m, 2H), 4.18 (brs, 2H), 6.42 (s, 1H), 6.83 (t, 1H, J=8.7Hz), 7.4-7.7 (m, 2H), 8.7-8.8 (m, 1H)
EIMS (M/Z) 405 ($M^+$)
Molecular formula $C_{21}H_{22}F_3N_3O_2$=405

Example 38

2-(4-Amino-3-fluorophenyl)-5-[3-(diethylamino)propylamino]-6,8-difluoro-7-methyl-4H-1-benzopyran-4-one (Compound 38)

(1) 1.02 g (1.89 mmol) of 6,8-difluoro-2-(3-fluoro-4-pivaloylaminophenyl)-5-[3-(methylsulfonyloxy)propylamino]-7-methyl-4H-1-benzopyran-4-one obtained in Example 37 (2) was dissolved in 30 mL of dimethylformamide, 2.0 mL (18.9 mmol) of diethylamine was added and the mixture was stirred at 80 °C for 15 hours. Water and a 1N aqueous solution of sodium hydroxide were added to the reaction solution and the mixture was extracted once with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (chloroform:methanol=100:1-9:1) to give 860 mg of 5-[3-(diethylamino)propylamino]-6,8-difluoro-2-(3-fluoro-4-pivaloylaminophenyl)-7-methyl-4H-1-benzopyran-4-one (yield: 88%).

NMR (90MHz, $CDCl_3$) δ (ppm) 1.06 (t, 6H, J=7.3Hz), 1.35 (s, 9H), 1.7-1.9 (m, 2H), 2.31 (t, 3H, J=2.4Hz), 2.5-2.8 (m, 2H), 2.60 (q, 4H, J=7.3Hz), 3.3-3.7 (m, 2H), 6.52 (s, 1H), 7.5-7.7 (m, 3H), 8.57 (t, 1H, 8.4Hz), 8.6-8.7 (m, 1H)
EIMS (M/Z) 517 ($M^+$)
Molecular formula $C_{28}H_{34}F_3N_3O_3$=517

(2) Substantially the same manner as that in Example 6 (3) was repeated except that 820 mg (1.59 mmol) of the above 5-[3-(diethylamino)propylamino]-6,8-difluoro-2-(3-fluoro-4-pivaloylaminophenyl)-7-methyl-4H-1-benzopyran-4-one was used, and the resulting compound was purified by silica gel column chromatography (chloroform:methanol=9:1-chloroform:methanol:aqueous ammonia=20:1:1) and recrystallized from ethanol/n-hexane, to give 584 mg of Compound 38 (yield: 85%).

NMR (270MHz, $CDCl_3$) δ (ppm) 1.05 (t, 6H, J=7.2Hz), 1.81 (quint., 2H, J=7.4Hz), 2.55 (q, 4H, J=7.2Hz), 2.57 (t, 2H, J=7.4Hz), 3.4-3.6 (m, 2H), 4.17 (brs, 2H), 6.42 (s, 1H), 6.83 (t, 1H, J=8.7Hz), 7.4-7.6 (m, 2H), 8.7-8.8 (m, 1H)
EIMS (M/Z) 433 ($M^+$)
Molecular formula $C_{23}H_{26}F_3N_3O_2$=433

Example 39

5-Amino-2-(4-amino-3-fluorophenyl)-6,8-difluoro-7-hydroxymethyl-4H-1-benzopyran-4-one (Compound 39)

(1) Substantially the same manner as that in Example 1 (1) was repeated except that 385 mg (1.00 mmol) of compound II-1 obtained in Reference Example 1 was used and 0.39 mL (5.0 mmol) of dimethylformamide was used in place of iodomethane, to give 402 mg of ethyl 3,5-difluoro-4-formyl-6-pivaloylamino-2-(2-tetrahydropyranyloxy)benzoate (yield: 97%).

NMR (90MHz, $CDCl_3$) δ (ppm) 1.29 (s, 9H), 1.39 (t, 3H, J=7.0Hz), 1.4-2.0 (m, 6H), 3.4-4.1 (m, 2H), 4.37 (q, 2H, J=7.0Hz), 5.3-5.4 (m, 1H), 7.52 (brs, 1H), 10.32 (s, 1H)
FAB-MS (M/Z) 414 ($M^+$+H)
Molecular formula $C_{20}H_{25}F_2NO_6$=413

(2) 22.3 g (54.0 mmol) of the above ethyl 3,5-difluoro-4-formyl-6-pivaloylamino-2-(2-tetrahydropyranyloxy)benzoate was dissolved in 260 mL of methanol, 1.02 g (27.0 mmol) of sodium borohydride was added under ice-cooling and the mixture was stirred at the same temperature for 1 hour. Water was added to the reaction solution, the solvent was distilled off to about 50 mL under reduced pressure, water was further added and the mixture was extracted twice with ethyl acetate. The organic layer was washed once with an aqueous saturated solution of sodium chloride, dried over anhydrous sodium sulfate and the solvent was distilled off under reduced pressure to give 22.0 g of ethyl 3,5-difluoro-4-hydroxymethyl-6-pivaloylamino-2-(2-tetrahydropyranyloxy)benzoate (yield: 98%).

NMR (90MHz, $CDCl_3$) δ (ppm) 1.29 (s, 9H), 1.38 (t, 3H, J=7.3Hz), 1.4-2.0 (m, 6H), 3.4-4.1 (m, 2H), 4.35 (q, 2H, J=7.3Hz), 4.7-4.8 (brs, 2H), 5.2-5.4 (m, 1H), 7.68 (brd, 1H)
FAB-MS (M/Z) 416 ($M^+$+H)

Molecular formula $C_{20}H_{27}F_2NO_6$=415

(3) 213 mg (0.50 mmol) of the above ethyl 3,5-difluoro-4-hydroxymethyl-6-pivaloylamino-2-(2-tetrahydropyranyloxy)benzoate was dissolved in 5 mL of dichloromethane, 68 mg (1.0 mmol) of imidazole and 302 mg (2.00 mmol) of tert-butyldimethylsilyl chloride were added and the mixture was heated at reflux for 30 minutes. The reaction solution was cooled on ice, water was added and the mixture was extracted once with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (n-hexane:ethyl acetate=4:1) to give 256 mg of ethyl 4-(tert-butyldimethylsilyloxy)methyl-3,5-difluoro-6-pivaloylamino-2-(2-tetrahydropyranyloxy)benzoate (yield: 98%).

NMR (90MHz, $CDCl_3$) δ (ppm) 0.03 (s, 6H), 0.83 (s, 9H), 1.23 (s, 9H), 1.32 (t, 3H, J=7.0Hz), 1.4-2.0 (m, 6H), 3.4-4.1 (m, 2H), 4.28 (q, 2H, J=7.0Hz), 4.70 (t, 2H, J=1.7Hz), 5.2-5.3 (m, 1H), 7.52 (brs, 1H)
FAB-MS (M/Z) 525 ($M^+$+H)
Molecular formula $C_{26}H_{41}F_2NO_6Si$=525

(4) Substantially the same manner as that in Example 1 (2) was repeated except that 29.0 g (55.2 mmol) of the above ethyl 4-tert-butyldimethylsilyloxymethyl-3,5-difluoro-6-pivaloylamino-2-(2-tetrahydropyranyloxy)benzoate and 11.4 g (48.2 mmol) of compound IV-1 obtained in Reference Example 2 were used, to give 9.69 g of 6,8-difluoro-2-(3-fluoro-4-pivaloylaminophenyl)-7-hydroxymethyl-5-pivaloylamino-4H-1-benzopyran-4-one (yield: 40%).

NMR (90MHz, DMSO-$d_6$) δ (ppm) 1.27 (s, 9H), 1.29 (s, 9H), 4.6-4.7 (m, 2H), 5.56 (t, 1H, J=5.7Hz), 7.07 (s, 1H), 7.7-8.0 (m, 3H), 9.20 (brs, 1H), 10.0 (brs, 1H)
EIMS (M/Z) 504 ($M^+$)
Molecular formula $C_{26}H_{27}F_3N_2O_5$=504

(5) 40 mL of ethanol and 20 mL of concentrated hydrochloric acid were added to 903 mg (1.79 mmol) of the above 6,8-difluoro-2-(3-fluoro-4-pivaloylaminophenyl)-7-hydroxymethyl-5-pivaloylamino-4H-1-benzopyran-4-one and the mixture was heated at reflux for 6.5 hours. The reaction solution was cooled on ice and adjudted to pH 7 to 8 by addition of a 10N aqueous solution of sodium hydroxide thereto, and the precipitated crystals were collected by filtration. The crystals were purified by silica gel column chromatography (chloroform:methanol=40:1-9:1) and triturated with ethyl acetate to give 364 mg of Compound 39 (yield: 60%).

NMR (270MHz, DMSO-$d_6$) δ (ppm) 4.5-4.6 (m, 2H), 5.44 (t, 1H, J=4.9Hz), 6.11 (brs, 2H), 6.69 (s, 1H), 6.87 (t, 1H, J=8.4Hz), 7.03 (brs, 2H), 7.5-7.7 (m, 2H)
EIMS (M/Z) 336 ($M^+$)
Molecular formula $C_{16}H_{11}F_3N_2O_3$=336

Example 40

5-Amino-2-(4-amino-3-fluorophenyl)-6,8-difluoro-7-(hydroxysulfonyloxy)methyl-4H-1-benzopyran-4-one (Compound 40)

10 mL of concentrated sulfuric acid was added to 1.00 g (1.98 mmol) of 6,8-difluoro-2-(3-fluoro-4-pivaloylaminophenyl)-7-hydroxymethyl-5-pivaloylamino-4H-1-benzopyran-4-one and the mixture was stirred at 50 °C for 20 minutes. The reaction solution was cooled on ice, ice water was added and the precipitated crystals were collected by filtration. The crystals were purified by silica gel column chromatography (chloroform:methanol=9:1-chloroform:methanol:aqueous ammonia=40:10:1) and triturated with ethanol to give 293 mg of Compound 40 (yield: 36%).

NMR (270MHz, DHSO-$d_6$) δ (ppm) 4.91 (s, 2H), 6.12 (brs, 2H), 6.70 (s, 1H), 6.88 (t, 1H, J=8.9Hz), 7.06 (brs, 2H), 7.61 (dd, 1H, J=8.6, 2.0Hz), 7.67 (dd, 1H, J=12.9, 1.7Hz)
FAB-MS (M/Z) 417 ($M^+$+H)
Molecular formula $C_{16}H_{11}F_3N_2O_6S$=416

Example 41

5-Amino-2-(4-amino-3-fluorophenyl)-7-aminomethyl-6,8-difluoro-4H-1-benzopyran-4-one (Compound 41)

(1) 3.30 g (6.55 mmol) of 6,8-difluoro-2-(3-fluoro-4-pivaloylaminophenyl)-7-hydroxymethyl-5-pivaloylamino-4H-1-benzopyran-4-one obtained in Example 39 (4) was dissolved in 70 mL of dimethylformamide, 4.6 mL of triethylamine and 1.0 mL of methanesulfonyl chloride were added under ice-cooling and the mixture was stirred at room temperature for 10 minutes. Water was added to the reaction solution and the mixture was extracted twice with ethyl acetate. The organic layer was washed once with water and once with an aqueous saturated solution of sodium chloride, and dried over anhydrous sodium sulfate, and the solvent was distilled off under reduced pressure to give 3.57 g of 6,8-difluoro-2-(3-fluoro-4-pivaloylaminophenyl)-7-(methylsulfonyloxy)methyl-5-pivaloylamino-4H-1-benzopyran-4-one (yield: 94%).

NMR (90MHz, $CDCl_3$) δ (ppm) 1.36 (9H, s), 1.38 (9H, s), 3.10 (s, 3H), 5.48 (brs, 2H), 6.69 (s, 1H), 7.5-7.9 (m, 3H), 8.61 (t, 1H, J=8.4Hz), 10.4 (brs, 1H)
FAB-MS (M/Z) 583 ($M^+$+H)
Molecular formula $C_{27}H_{29}F_3N_2O_7S$=582

(2) 400 mg (0.687 mmol) of the above 6,8-difluoro-2-(3-fluoro-4-pivaloylaminophenyl)-7-(methylsulfonyloxy)methyl-5-pivaloylamino-4H-1-benzopyran-4-one was dissolved in 50 mL of an about 6N solution of ammonia in methanol and the solution was stirred at room temperature for 9 hours. Water was added to the reaction solution and the mixture was extracted twice with chloroform. The organic layer was washed once with water and once with an aqueous saturated solution of sodium chloride and dried over sodium sulfate. The solvent was distilled off under reduced pressure and the residue was purified by silica gel column chromatography (chloroform:methanol=30:1) to give 142 mg of 7-aminomethyl-6,8-difluoro-2-(3-fluoro-4-pivaloylaminophenyl)-5-pivaloylamino-4H-1-benzopyran-4-one (yield: 41%).

NMR (90MHz, $CDCl_3$) δ (ppm) 1.37 (s, 9H), 1.38 (s, 9H), 4.1-4.2 (m, 2H), 6.60 (s, 1H), 7.5-7.9 (m, 3H), 8.60 (t, 1H, J=8.8Hz), 10.5 (brs, 1H)
FAB-MS (M/Z) 504 ($M^+$+H)
Molecular formula $C_{26}H_{28}F_3N_3O_4$=503

(3) Substantially the same manner as that in Example 13 (2) was repeated except that 2.99 g (5.94 mmol) of the above 7-aminomethyl-6,8-difluoro-2-(3-fluoro-4-pivaloylaminophenyl)-5-pivaloylamino-4H-1-benzopyran-4-one was used, and the resulting compound was purified by silica gel column chromatography (chloroform:methanol=20:1-chloroform:methanol:aqueous ammonia=9:1:1) and triturated with chloroform, to give 1.59 g of Compound 41 (yield: 80%).

NMR (270MHz, DMSO-$D_6$) δ (ppm) 3.80 (s, 2H), 6.08 (brs, 2H), 6.67 (s, 1H), 6.87 (t, 1H, J=8.9Hz), 7.00 (brs, 2H), 7.59 (dd, 1H, J=8.4, 2.0Hz), 7.66 (dd, 1H, J=12.9, 2.0Hz)
FAB-MS (M/Z) 336 ($M^+$+H)
Molecular formula $C_{16}H_{12}F_3N_3O_2$=335

Example 42

Hydrochloride of 5-amino-2-(4-amino-3-fluorophenyl)-7-dimethylaminomethyl-6,8-difluoro-4H-1-benzopyran-4-one (Compound 42)

(1) 800 mg (1.37 mmol) of 6,8-difluoro-2-(3-fluoro-4-pivaloylaminophenyl)-7-(methylsulfonyloxy)methyl-5- pivaloylamino-4H-1-benzopyran-4-one obtained in Example 41 (1) was dissolved in 10 mL of dimethylformamide, 558 mg (6.85 mmol) of dimethylamine hydrochloride and 945 mg (6.85 mmol) of potassium carbonate were added and the mixture was stirred at room temperature for 45 minutes. Water was added to the reaction solution and the mixture was extracted once with ethyl acetate. The organic layer was washed once with water and once with an aqueous saturated solution of sodium chloride, and dried over anhydrous sodium sulfate, and the solvent was distilled off under reduced pressure to give 727 mg of 7-dimethylaminomethyl-6,8-difluoro-2-(3-fluoro-4-pivaloylaminophenyl)-5-pivaloylamino-4H-1-benzopyran-4-one (yield: 100%).

NMR (90MHz, $CDCl_3$) δ (ppm) 1.36 (s, 9H), 1.38 (s, 9H), 2.36 (s, 6H), 3.81 (brs, 2H), 6.67 (s, 1H), 7.5-7.8 (m,

3H), 8.59 (t, 1H, J=8.4Hz), 10.4 (brs, 1H)
FAB-MS (M/Z) 532 ($M^+$+H)
Molecular formula $C_{28}H_{32}F_3N_3O_4$=531

(2) Substantially the same manner as that in Example 6 (3) was repeated except that 715 mg (1.35 mmol) of the above 7-dimethylaminomethyl-6,8-difluoro-2-(3-fluoro-4-pivaloylaminophenyl)-5-pivaloylamino-4H-1-benzopyran-4-one was used, and the resulting compound was purified by silica gel column chromatography (chloroform:methanol=30:1-9:1) and recrystallised from ethanol/methanol, to give 360 mg of Compound 42 (yield: 73%). This compound was dissolved in 60 mL of ethanol, 2 mL of a 1N hydrochloric acid/2-propanol solution was added dropwise and 60 mL of isopropyl ether was further added. The precipitated crystals were collected by filtration to give a hydrochloride of Compound 42.

NMR (270MHz, DMSO-$d_6$) δ (ppm) 2.84 (s, 6H), 4.47 (brs, 2H), 6.16 (brs, 2H), 6.76 (s, 1H), 6.88 (t, 1H, J=8.9Hz), 7.25 (brs, 2H), 7.61 (dd, 1H, J=8.6, 2.0Hz), 7.67 (dd, 1H, J=12.9, 2.0Hz), 10.7 (brs, 1H)
FAB-MS (M/Z) 364 ($M^+$+H)
Molecular formula $C_{18}H_{16}F_3N_3O_2$=363

Example 43

5-Amino-2-(4-amino-3-fluorophenyl)-6,8-difluoro-7-methoxymethyl-4H-1-benzopyran-4-one (Compound 43)

(1) 800 mg (1.37 mmol) of 6,8-difluoro-2-(3-fluoro-4-pivaloylaminophenyl)-7-(methylsulfonyloxy)methyl-5-pivaloylamino-4H-1-benzopyran-4-one obtained in Example 41 (1) was dissolved in 200 mL of methanol and the solution was heated at reflux for 24 hours. The solvent was distilled off under reduced pressure, the residue was dissolved in chloroform, washed once with an aqueous saturated solution of sodium chloride and dried over anhydrous sodium sulfate. The solvent was distilled off under reduced pressure and the residue was purified by silica gel column chromatography (chloroform:acetonitrile=20:1) to give 610 mg of 6,8-difluoro-2-(3-fluoro-4-pivaloylaminophenyl)-7-methoxymethyl-5-pivaloylamino-4H-1-benzopyran-4-one (yield: 86%).

NMR (90MHz, $CDCl_3$) δ (ppm) 1.36 (s, 9H), 1.38 (s, 9H), 3.44 (s, 3H), 4.72 (t, 2H, J=1.8Hz), 6.67 (s, 1H), 7.6-7.9 (m, 3H), 8.60 (t, 1H, J=8.6Hz), 10.4 (brs, 1H)
FAB-MS (M/Z) 519 ($M^+$+H)
Molecular formula $C_{27}H_{29}F_3N_2O_5$=518

(2) Substantially the same manner as that in Example 13 (2) was repeated except that 610 mg (1.18 mmol) of the above 6,8-difluoro-2-(3-fluoro-4-pivaloylaminophenyl)-7-methoxymethyl-5-pivaloylamino-4H-1-benzopyran-4-one was used, and the resulting compound was purified by silica gel column chromatography (chloroform: acetonitrile=9:1) and recrystallized from ethyl acetate, to give 267 mg of Compound 43 (yield: 65%).

NMR (270MHz, DMSO-$d_6$) δ (ppm) 3.31 (s, 3H), 4.57 (brs, 2H), 6.10 (brs, 2H), 6.69 (s, 1H), 6.87 (t, 1H, J=8.9Hz), 7.06 (brs, 1H), 7.5-7.7 (m, 2H)
EIMS (M/Z) 350 ($M^+$)
Molecular formula $C_{17}H_{13}F_3N_2O_3$=350

Example 44

7-Acetoxymethyl-5-amino-2-(4-amino-3-fluorophenyl)-6,8-difluoro-4H-1-benzopyran-4-one (Compound 44)

6.02 g (11.9 mmol) of 6,8-difluoro-2-(3-fluoro-4-pivaloylaminophenyl)-7-hydroxymethyl-5-pivaloylamino-4H-1-benzopyran-4-one obtained in Example 39 (4) was dissolved in a mixed solvent of 160 mL of acetic acid and 40 mL of concentrated sulfuric acid and the solution was stirred at 100 °C for 35 minutes. The reaction solution was cooled on ice, poured into ice water and the mixture was extracted twice with ethyl acetate. The organic layer was washed twice with a 1N aqueous solution of sodium hydroxide, once with water and once with an aqueous saturated solution of sodium chloride and dried over anhydrous sodium sulfate. The solvent was distilled off under reduced pressure, the residue was purified by silica gel column chromatography (chloroform:methanol=100:1) and recrystallized from chloroform/methanol/n=hexane to give 2.43 g of Compound 44 (yield: 54%).

NMR (270MHz, $CDCl_3$) δ (ppm) 2.11 (s, 3H), 5.29 (t, 2H, J=1.5Hz), 6.49 (s, 1H), 6.84 (t, 1H, J=8.7Hz), 7.5-7.6 (m,

2H)
FAB-MS (M/Z) 379 ($M^+$+H)
Molecular formula $C_{18}H_{13}F_3N_2O_4$=378

Example 45

5-Amino-2-(4-amino-3-fluorophenyl)-6,8-difluoro-7-(1-propanoyloxymethyl)-4H-1-benzopyran-4-one (Compound 45)

Substantially to the same manner as that in Example 44 was repeated except that 1.06 g (2.10 mmol) of 6,8-difluoro-2-(3-fluoro-4-pivaloylaminophenyl)-7-hydroxymethyl-5-pivaloylamino-4H-1-benzopyran-4-one, 32 mL of propionic acid and 8 mL of concentrated sulfuric acid were used, and the resulting compound was purified by silica gel column chromatography (chloroform:acetonitrile=20:1) and high performance liquid chromatography, to give 261 mg of Compound 45 (yield: 32%).

NMR (270MHz, DMSO-$d_6$) δ (ppm) 1.04 (t, 3H, J=7.6Hz), 2.36 (q, 2H, J=7.6Hz), 5.24 (brs, 2H), 6.10 (brs, 2H), 6.70 (s, 1H), 6.87 (t, 1H, J=8.6Hz), 7.10 (brs, 2H), 7.59 (dd, 1H, J=8.6, 2.0Hz), 7.65 (dd, 1H, J=12.9, 2.0Hz)
EIMS (M/Z) 392 ($M^+$)
Molecular formula $C_{19}H_{15}F_3N_2O_4$=392

Example 46

5-Amino-2-(4-amino-3-fluorophenyl)-6,8-difluoro-7-(1-hexanoyloxymethyl)-4H-1-benzopyran-4-one (Compound 46)

Substantially the same manner as that in Example 44 was repeated except that 1.06 g (2.10 mmol) of 6,8-difluoro-2-(3-fluoro-4-pivaloylaminophenyl)-7-hydroxymethyl-5-pivaloylamino-4H-1-benzopyran-4-one, 24 mL of hexanoic acid and 6 mL of concentrated sulfuric acid were used, and the resulting compound was purified by silica gel column chromatography (chloroform:acetonitrile=30:1-20:1) and recrystallized from ethyl acetate/n-hexane, to give 489 mg of Compound 46 (yield: 55%).

NMR (270MHz, $CDCl_3$) δ (ppm) 0.88 (t, 3H, J=6.4Hz), 1.2-1.3 (m, 4H), 1.64 (quint., 2H, J=7.4Hz), 2.35 (t, 2H, J=7.4Hz), 5.29 (s, 2H), 6.49 (s, 1H), 6.84 (t, 1H, J=8.9Hz), 7.5-7.6 (m, 2H)
EIMS (M/Z) 434 ($M^+$)
Molecular formula $C_{22}H_{21}F_3N_2O_4$=434

Reference Example 1

Ethyl 3,5-difluoro-6-pivaloylamino-2-(2-tetrahydropyranyloxy)benzoate (Compound II-1)

(1) 104 g (796 mmol) of 2,4-difluorophenol was dissolved in 800 mL of dichloromethane, 132 mL of trimethylamine and 92.0 mL of ethyl chloroformate were added under ice-cooling and the mixture was stirred at -10 to 0 °C for 2 hours. The reaction solution was washed with an aqueous saturated solution of sodium chloride, and dried over anhydrous magnesium sulfate, and the solvent was distilled off under reduced pressure to give 156 g of O-ethoxycarbonyl-2,4-difluorophenol (yield: 97%)

NMR (90MHz, $CDCl_3$), δ (ppm) 1.39 (t, 3H, J=7.0Hz), 4.33 (q, 2H, J=7.0Hz), 6.7-7.3 (m, 3H)
MS (M/Z) 202 ($M^+$)
Molecular formula $C_9H_8F_2O_3$=202

(2) 50.5 g (250 mmol) of the above O-ethoxycarbonyl-2,4-difluorophenol was dissolved in 115 mL of concentrated sulfuric acid, 15.9 mL of fuming nitric acid was added thereto while keeping an internal temperature at 10 to 20 °C and the mixture was stirred at the same temperature for 1 hour. The reaction solution was poured into ice water and the mixture was extracted with 500 mL of ethyl acetate. The organic layer was washed twice with an aqueous saturated solution of sodium chloride, dried over anhydrous sodium sulfate and the solvent was distilled off under reduced pressure. The residue was dissolved in 1.0 L of methanol, 50 mL of water and 40 g of sodium bicarbonate were added and the mixture was stirred at room temperature for 16 hours. The reaction solution was filtered and methanol was distilled off under reduced pressure. 200 mL of water was added to adjust the pH to 5 and the mixture was extracted twice with ethyl acetate. The organic layer was washed once with 400 mL of water and once with 400 mL of an aqueous saturated solution of sodium chloride, and dried over anhydrous sodium sulfate, and the solvent

was distilled off under reduced pressure to give 41.6 g of 2,4-difluoro-5-nitrophenol (yield: 95%).

NMR (90MHz, $CDCl_3$) δ (ppm) 7.23 (t, 1H, J=9.9Hz), 7.76 (dd, 1H, J=8.6, 7.3Hz)
MS (M/Z) 175 ($M^+$)
Molecular formula $C_6H_3F_2NO_3$=175

(3) 24.9 g (142 mmol) of the above 2,4-difluoro-5-nitrophenol was dissolved in 150 mL of ethyl acetate, 5.0 g of 10% palladium/carbon was added and the mixture was stirred at 50 to 60 °C for 27 hours under a stream of hydrogen. The gaseous phase in the reaction vessel was substituted with nitrogen, the reaction solution was filtered by suction and the solvent was distilled off under reduced pressure. The residue was triturated with n-hexane to give 19.8 g of 5-amino-2,4-difluorophenol (yield: 96%).

NMR (90MHz, $CDCl_3$) δ (ppm) 4.75 (brs, 2H), 6.37 (t, 1H, J=9.1Hz), 6.87 (t, 1H, J=11.1Hz), 9.21 (s, 1H)
MS (M/Z) 145 ($M^+$)
Molecular formula $C_6H_5F_2NO$=145

(4) 18.9 g (130 mmol) of the above 5-amino-2,4-difluorophenol was dissolved in 45 mL of pyridine, 16.0 mL of pivaloyl chloride was added dropwise under ice-cooling and the mixture was stirred at the same temperature for 30 minutes. 1N hydrochloric acid was added to the reaction solution and the mixture was extracted with ether. The organic layer was washed once with 1N hydrochloric acid, once with water and once with an aqueous saturated solution of sodium chloride and dried over anhydrous magnesium sulfate. The solvent was distilled off under reduced pressure and the residue was triturated with n-hexane to give 27.0 g of 2,4-difluoro-5-pivaloylaminophenol (yield: 91%).

NMR (90MHz, $CDCl_3$) δ (ppm) 1.35 (s, 9H), 6.90 (t, 1H, J=10.4Hz), 7.65 (brs, 1H), 7.94 (brs, 1H), 8.24 (dd, 1H, J=9.1, 8.0Hz)
MS (M/Z) 229 ($M^+$)
Molecular formula $C_{11}H_{13}F_2NO_2$=229

(5) 2.15 g (9.39 mmol) of the above 2,4-difluoro-5-pivaloylaminophenol was dissolved in 40 mL of dichloromethane, 4.3 mL of 3,4-dihydro-2H-pyran and 44 mg of camphorsulfonic acid were added and the mixture was stirred at room temperature for 4.3 hours. The reaction solution was added to a 5% aqueous solution of potassium carbonate and the mixture was extracted with chloroform. The organic layer was washed once with water and once with an aqueous saturated solution of sodium chloride and dried over anhydrous magnesium sulfate. The solvent was distilled off under reduced pressure and the residue was triturated with n-hexane to give 2.51 g of 2,4-difluoro-5-pivaloylamino-O-(2-tetrahydropyranyl)phenol (yield: 85%).

NMR (90MHz, $CDCl_3$) δ (ppm) 1.31 (s, 9H), 1.4-2.2 (m, 6H), 3.4-4.2 (m, 2H), 5.43 (brs, 1H), 6.89 (t, 1H, J=10.4Hz), 7.44 (brs, 1H), 8.25 (t, 1H, J=8.5Hz)
MS (M/Z) 313 ($M^+$)
Molecular formula $C_{16}H_{21}F_2NO_3$=313

(6) 31.3 g (100 mmol) of the above 2,4-difluoro-5-pivaloylamino-O-(2-tetrahydropyranyl)phenol was dissolved in 300 mL of tetrahydrofuran, 40 mL of hexamethylphosphoric triamide was added and the solution was cooled to -78 °C. To this solution was added 140 mL of a 1.6M solution of n-butyl lithium in n-hexane while keeping an internal temperature at -60 °C or below and the mixture was stirred at the same temperature for 1 hour. 19 mL of ethyl chloroformate was added and the mixture was stirred while gradually raising the temperature. After 2.2 hours, an internal temperature reached -15 °C, water was added thereto and the mixture was extracted with ethyl acetate. The organic layer was washed once with water and once with an aqueous saturated solution of sodium chloride, and dried over anhydrous magnesium sulfate, and the solvent was distilled off under reduced pressure. Trituration with n-hexane afforded 23.4 g of compound II-1 (yield: 61%).

NMR (90MHz, $CDCl_3$) δ (ppm) 1.28 (s, 9H), 1.38 (t, 3H, J=7.0Hz), 1.4-2.0 (m, 6H), 3.3-4.1 (m, 2H), 4.36 (q, 2H, J=7.0Hz), 5.32 (brs, 1H), 7.00 (dd, 1H, J=10.7, 9.6Hz), 7.57 (brs, 1H)
FAB-MS (M/Z) 386 ($M^+$+H)
Molecular formula $C_{19}H_{25}F_2NO_5$=385

Reference Example 2

3'-Fluoro-4'-pivaloylaminoacetophenone (Compound IV-1)

(1) 250 g (1.32 mol) of 4-bromo-2-fluoroaniline was dissolved in 500 mL of pyridine, 178 mL (1.45 mol) of pivaloyl chloride was added dropwise under ice-cooling and the mixture was stirred for 10 minutes. The reaction solution was poured into 1.5 L of ice water and the precipitated crystals were collected by filtration. The crystals were washed with 1N hydrochloric acid and water and dried by heating at 40 to 60 °C under reduced pressure to give 350 g of 4-bromo-2-fluoro-N-pivaloylaniline (yield: 97%).
(2) 70.6 g (258 mmol) of the above 4-bromo-2-fluoro-N-pivaloylaniline was dissolved in 500 mL of toluene under argon atmosphere, 108 mL (310 mmol) of 1-ethoxyvinyltributyltin and 1.80 g (2.57 mmol) of bis(triphenylphosphine)palladium (II) chloride were added and the mixture was stirred at 100 °C for 5 hours. The reaction solution was cooled on ice, 500 mL of 2N hydrochloric acid was added, the mixture was stirred at room temperature for 2 hours and the insoluble matters were filtered off. The filtrate was extracted once with ethyl acetate, 500 mL of a 10% aqueous solution of ammonium fluoride was added to the organic layer and the mixture was stirred at room temperature for 3 hours. The insoluble matters were filtered off, and the organic layer was washed once with water and once with an aqueous saturated solution of sodium chloride and dried over anhydrous sodium sulfate. The solvent was distilled off under reduced pressure and the residue was purified by silica gel column chromatography (n-hexane:ethyl acetate=6:1-4:1) to give 60.8 g of compound IV-1 (yield: 99%).

NMR (90MHz, $CDCl_3$) δ (ppm) 1.35 (s, 9H), 2.57 (s, 3H), 7.6-7.9 (m, 3H), 8.53 (t, 1H, J=8.4Hz)
EIMS (M/Z) 237 ($M^+$)
Molecular formula $C_{13}H_{16}FNO_2$=237

Reference Example 3

3'-Chloro-4'-pivaloylaminoacetophenone (Compound IV-2)

(1) Substantially the same manner as that in Reference Example 2 (1) was repeated except that 20.6 g (100 mmol) of 4-bromo-2-chloroaniline was used, to give 27.8 g of 4-bromo-2-chloro-N-pivaloylaniline (yield: 96%).
(2) Substantially the same manner as that in Reference Example 2 (2) was repeated except that 2.91 g (10.0 mmol) of the above 4-bromo-2-chloro-N-pivaloylaniline was used, to give 1.64 g of compound IV-2 (yield: 65%).

NMR (90MHz, $CDCl_3$) δ (ppm) 1.30 (t, 3H, J=7.7Hz), 1.35 (s, 9H), 2.57 (s, 3H), 2.65 (q, 2H, J=7.7Hz), 7.47 (brs, 1H), 7.7-7.9 (m, 2H), 8.25 (d, 1H, J=9.0Hz)
EIMS (M/Z) 247 ($M^+$)
Molecular formula $C_{15}H_{21}NO_2$=247

Reference Example 4

3'-Ethyl-4'-pivaloylaminoacetophenone (Compound IV-3)

(1) Substantially the same manner as that in Reference Example 2 (1) was repeated except that 4.68 g (19.8 mmol) of 4-bromo-2-ethylaniline was used, to give 5.10 g of 4-bromo-2-ethyl-N-pivaloylaniline (yield: 90%).
(2) Substantially the same manner as that in Reference Example 2 (2) was repeated except that 4.28 g (15.0 mmol) of the above 4-bromo-2-ethyl-N-pivaloylaniline was used, to give 3.23 g of compound IV-3 (yield: 87%).

NMR (90MHz, $CDCl_3$) δ (ppm) 1.36 (s, 9H), 2.57 (s, 3H), 7.80 (dd, 1H, J=8.8, 2.0Hz), 8.01 (d, 1H, J=2.0Hz), 8.20 (brs, 1H), 8.58 (d, 1H, J=8.6Hz)
EIMS (M/Z) 253 ($M^+$)
Molecular formula $C_{13}H_{16}{}^{35}ClNO_2$=253

Reference Example 5

3',5'-Dichloro-4'-pivaloylaminoacetophenone (Compound-IV-4)

(1) 12.0 g (50.0 mmol) of 4-bromo-2,6-dichloroaniline hydrochloride was dissolved in 50 mL of pyridine, 9.90 mL (80.0 mmol) of pivaloyl chloride and 0.61 g (5.0 mmol) of N,N-dimethylaminopyridine were added and the mixture

was stirred at 40 °C for 28 hours. Thereafter, substantially the same manner as that in Reference Example 2 (1) was repeated to give 15.7 g of 4-bromo-2,6-dichloro-N-pivaloylaniline (yield: 97%).

(2) Substantially the same manner as that in Reference Example 2 (2) was repeated except that 11.4 g (35.0 mmol) of the above 4-bromo-2,6-dichloro-N-pivaloylaniline was used and recrystallization from toluene/n-hexane was carried out in place of purification by silica gel column chromatography, to give 8.93 g of compound IV-4 (yield: 89%).

NMR (90MHz, $CDCl_3$) δ (ppm) 1.38 (s, 9H), 2.58 (s, 3H), 7.26 (brs, 1H), 7.90 (s, 2H)
EIMS (M/Z) 287 ($M^+$)
Molecular formula $C_{13}H_{15}{}^{35}Cl_2NO_2$=287

Reference Example 6

6,8-Difluoro-2-(3-fluoro-4-pivaloylaminophenyl)-5-pivaloylamino-4H-1-benzopyran-4-one (Compound VIII-1)

(1) 200 g of ethyl 6-(N-ethoxycarbonyl-N-pivalolamino)-2-(2-tetrahydropyranyloxy)benzoate obtained according to the known method (JP-A-61-78) was dissolved in 900 mL of ethanol, 300 mL of concentrated hydrochloric acid was added and the mixture was heated at reflux for 3.5 hours. The reaction solution was cooled on ice, 500 mL of water was added and the resulting crystals were collected by filtration to give 89.1 g of ethyl 6-(N-ethoxycarbonylamino)-2-hydroxybenzoate (yield: 74%).

NMR (90MHz, $CDCl_3$) δ (ppm) 1.31 (t, 3H, J=7.0Hz), 1.50 (t, 3H, J=7.0Hz), 4.22 (q, 2H, J=7.0Hz), 4.53 (q, 2H, J=7.0Hz), 6.65 (dd, 1H, J=8.2, 1.2Hz), 7.37 (t, 1H, J=8.4Hz), 7.86 (dd, 1H, J=8.4, 1.1Hz), 9.48 (brs, 1H), 10.74 (s, 1H)
MS (M/Z) 253 ($M^+$)
Molecular formula $C_{12}H_{15}NO_5$=253

(2) 2.0 g of the above ethyl 6-(N-ethoxycarbonylamino)-2-hydroxybenzoate was dissolved in 30 mL of dichloroethane, 5.0 g of Onoda Florinate FP-T700 (Wako Pure Chemical Industries Ltd.) was added and the mixture was heated at reflux for 3.7 hours. 1.25 g of FP-T700 was further added and the mixture was heated at reflux for 50 minutes. 1.25 g of FP-T700 was further added and the mixture was heated at reflux for 1 hours. The reaction solution was made acidic by addition of 1N hydrochloric acid thereto, the mixture was extracted with ether, the organic layer was washed with water and an aqueous saturated solution of sodium chloride, and dried over anhydrous sodium sulfate, and the solvent was distilled off under reduced pressure. The residue was purified by silica gel column chromatography (n-hexane:ethyl acetate=9:1-3:1) to give 690 mg of ethyl 3,5-difluoro-6-(N-ethoxycarbonylamino)-2-hydroxybenzoate (yield: 30%).

NMR (90MHz, $CDCl_3$) δ (ppm) 1.29 (t, 3H, J=7.0Hz), 1.43 (t, 3H, J=7.1Hz), 4.21 (q, 2H, J=7.2Hz), 4.47 (q, 2H, J=7.1Hz), 6.83 (brs, 1H), 7.13 (t, 1H, J=9.9Hz), 10.54 (s, 1H)
MS (M/Z) 289 ($M^+$)
Molecular formula $C_{12}H_{13}F_2NO_5$=289

(3) 5.72 g of the above ethyl 3,5-difluoro-6-(N-ethoxycarbonylamino)-2-hydroxybenzoate was dissolved in 70 mL of dichloromethane under argon atmosphere, 4.13 mL of diisopropylethylamine and 1.80 mL of chloromethyl methyl ether were added under ice-cooling and the mixture was stirred at 0 °C for 20 minutes. Dilute hydrochloric acid was added to the reaction solution and the mixture was extracted with ether. The organic layer was washed with water and an aqueous saturated solution of sodium chloride, and dried over anhydrous sodium sulfate, and the solvent was distilled off under reduced pressure to give ethyl 3,5-difluoro-6-(N-ethoxycarbonylamino)-2-methoxymethoxybenzoate.

NMR (90MHz, $CDCl_3$) δ (ppm) 1.27 (t, 3H, J=7.1Hz), 1.38 (t, 3H, J=7.3Hz), 3.55 (s, 3H), 4.19 (q, 2H, J=7.1Hz), 4.39 (q, 2H, J=7.1Hz), 5.11 (s, 2H), 6.56 (brs, 1H), 7.01 (t, 1H, J=10.0Hz)
MS (M/Z) 333 ($M^+$)
Molecular formula $C_{14}H_{17}F_2NO_6$=333

(4) The above ethyl 3,5-difluoro-6-(N-ethoxycarbonylamino)-2-methoxymethoxybenzoate was dissolved in 35 mL of tetrahydrofuran under ice-cooling, 792 mg of sodium hydride (60% oil dispersion) and 1.69 mL of pivaloyl chloride were added and the mixture was stirred at 0 °C for 25 minutes. 396 mg of sodium hydride (60% oil dispersion) and 0.85 mL of pivaloyl chloride were further added and the mixture was stirred at 0 °C for 20 minutes. 158 mg of

sodium hydride (60% oil dispersion) and 0.34 mL of pivaloyl chloride were further added and the mixture was stirred 0 °C for 1.2 hours. An aqueous saturated solution of ammonium chloride was added to the reaction solution and the mixture was extracted with ether. The organic layer was washed with an aqueous saturated solution of sodium chloride, and dried over anhydrous sodium sulfate, and the solvent was distilled off under reduced pressure to give 6.63 g of ethyl 3,5-difluoro-6-(N-ethoxycarbonyl-N-pivaloylamino)-2-methoxymethoxybenzoate (two stage yield: 80%).

NMR (90MHz, $CDCl_3$) δ (ppm) 1.20 (t, 3H, J=7.0Hz), 1.33 (t, 3H, J=7.0Hz), 1.39 (s, 9H), 3.55 (s, 3H), 4.18 (q, 2H, J=7.0Hz), 4.33 (q, 2H, J=7.0Hz), 5.12 (s, 2H), 7.00 (dd, 1H, J=10.2, 9.1Hz)
MS (M/Z) 417 ($M^+$)
Molecular formula $C_{19}H_{25}F_2NO_7$=417

(5) A solution of 4.19 g of the above ethyl 3,5-difluoro-6-(N-ethoxycarbonyl-N-pivaloylamino)-2-methoxymethoxybenzoate and 1.98 g of 3'-fluoro-4'-pivaloylaminoacetophenone dissolved in 23 mL of dioxane was added dropwise to a suspension obtained by adding 10 mL of dioxane to 737 mg of sodium hydride (60% oil dispersion) and the mixture was heated at reflux for 2.3 hours. The reaction solution was cooled, water was added, the aqueous layer was washed with n-hexane and further extracted with ethyl acetate. The ethyl acetate layer was washed with an aqueous saturated solution of sodium chloride, dried over anhydrous sodium sulfate and purified by silica gel column chromatography (chloroform: acetone=40:1-30:1) to give 3.37 g of a 1,3-diketone compound (yield: 75%).
(6) 3.37 g of the above 1,3-diketone compound was dissolved in 80 mL of ethanol, 20 mL of concentrated hydrochloric acid was added and the mixture was stirred at room temperature for 6.6 hours. The reaction solution was cooled on ice, 100 mL of water was added, and the precipitated crystals were collected by filtration to give 2.72 g of compound VIII-1 (yield: 91%).

NMR (90MHz, $CDCl_3$) δ (ppm) 1.36 (s, 9H), 1.38 (s, 9H), 6.68 (s, 1H), 7.35 (t, 1H, J=9.8Hz), 7.5-7.9 (m, 2H), 8.60 (t, 1H, J=8.4Hz)
MS (M/Z) 474 ($M^+$)
Molecular formula $C_{25}H_{25}F_3N_2O_4$=474

Reference Example 7

3',4',5'-Trifluoro-2'-hydroxy-6'-pivaloylaminoacetophenone (Compound XIV)

(1) 7.40 g (50.0 mmol) of 2,3,4-trifluorophenol was dissolved in 50 mL of dichloromethane, 8.30 mL (60.0 mmol) of triethylamine and 5.74 mL (60.0 mmol) of ethyl chloroformate were added under ice-cooling and the mixture was stirred at room temperature for 20 minutes. Water was added to the reaction solution and the mixture was extracted once with chloroform. The organic layer was washed once with an aqueous saturated solution of sodium chloride, and dried over anhydrous sodium, sulfate and the solvent was distilled off under reduced pressure to give 9.83 g of O-ethoxycarbonyl-2,3,4-trifluorophenol (yield: 89%).

NMR (90MHz, $CDCl_3$) δ (ppm) 1.40 (t, 3H, J=7.0Hz), 4.34 (q, 2H, J=7.0Hz), 6.9-7.1 (m, 2H)

(2) 9.82 g (44.6 mmol) of the above O-ethoxycarbonyl-2,3,4-trifluorophenol was dissolved in 25 mL of concentrated sulfuric acid under ice-cooling, 8.0 mL of fuming nitric acid was added at an internal temperature of 50 °C or below and the mixture was further stirred for 2 hours. The reaction solution was poured into 300 mL of ice water and the mixture was extracted once with ethyl acetate. The organic layer was washed once with water and twice with an aqueous saturated solution of sodium chloride and dried over anhydrous sodium sulfate. The solvent was distilled off under reduced pressure and the residue was purified by silica gel column chromatography (n-hexane:ethyl acetate=20:1) to give 1.44 g of O-ethoxycarbonyl-2,3,4-trifluoro-5-nitrophenol (yield: 12%)

NMR (90MHz, $CDCl_3$) δ (ppm) 1.42 (t, 3H, J=7.3Hz), 4.39 (q, 2H, J=7.3Hz), 7.92 (td, 1H, J=7.0, 2.4Hz)
FAB-MS (Negative) (M/Z) 264 ($M^+$+H)
Molecular formula $C_9H_6F_3NO_5$=265

(3) 1.43 g (5.40 mmol) of the above O-ethoxycarbonyl-2,3,4-trifluoro-5-nitrophenol was dissolved in 20 mL of ethyl acetate under argon atmosphere, 200 mg of 10% palladium/carbon was added thereto, the gaseous phase in the reaction vessel was substituted with hydrogen and the mixture was stirred at room temperature for 4 hours. The reaction solution was filtered and the solvent was distilled off under reduced pressure to give 1.27 g of 5-amino-O-ethoxycarbonyl-2,3,4-trifluorophenol (yield: 100%).

NMR (90MHz, $CDCl_3$) δ (ppm) 1.38 (t, 3H, J=7.3Hz), 3.45 (brs, 2H), 4.34 (q, 2H, J=7.3Hz), 6.3-6.5 (m, 1H)
EIMS (M/Z) 235 ($M^+$)
Molecular formula $C_9H_8F_3NO_3$=235

(4) 1.27 g (5.40 mmol) of the above 5-amino-O-ethoxycarbonyl-2,3,4-trifluorophenol was dissolved in 5 mL of pyridine, 0.80 mL (6.5 mmol) of pivaloyl chloride was added under ice-cooling and the mixture was stirred at the same temperature for 15 minutes. 1N hydrochloric acid was added to the reaction solution and the mixture was extracted once with ethyl acetate. The organic layer was washed once with water and once with an aqueous saturated solution of sodium chloride, dried over anhydrous sodium sulfate and the solvent was distilled off under reduced pressure. The residue was dissolved in 25 mL of methanol, 1.40 g (10.1 mmol) of potassium carbonate and 15 mL of water were added and the mixture was stirred at room temperature for 4 hours. The reaction solution was adjusted to pH 2 by addition of 2N hydrochloric acid thereto and the mixture was extracted once with ethyl acetate. The organic layer was washed once with water and once with an aqueous saturated solution of sodium chloride, and dried over anhydrous sodium sulfate, and the solvent was distilled off under reduced pressure to give 1.20 g of 2,3,4-trifluoro-5-pivaloylaminophenol (two stage yield: 90%).

NMR (90MHz, $CDCl_3$) δ (ppm) 1.36 (s, 9H), 7.66 (brs, 1H), 8.02 (ddd, 1H, J=8.4, 7.3, 2.6Hz), 8.45 (brs, 1H)
EIMS (M/Z) 247 ($M^+$)
Molecular formula $C_{11}H_{12}F_3NO_2$=247

(5) 1.19 g (4.82 mmol) of the above 2,3,4-trifluoro-5-pivaloylaminophenol was dissolved in 50 mL of dichloromethane, 0.88 mL (9.6 mmol) of 3,4-dihydro-2H-pyran and 22 mg (0.095 mmol) of dl-camphorsulfonic acid were added and the mixture was stirred at room temperature for 1 hour. The reaction solution was poured into 100 mL of a 10% aqueous solution of potassium carbonate and the mixture was extracted once with chloroform. The organic layer was washed once with an aqueous saturated solution of sodium chloride and dried over anhydrous sodium sulfate. The solvent was distilled off under reduced pressure and the residue was triturated with n-hexane to give 1.25 g of 2,3,4-trifluoro-5-pivaloylamino-O-tetrahydropyranylphenol (yield: 78%).

(6) 3.60 g (10.9 mmol) of the above 2,3,4-trifluoro-5-pivaloylamino-O-tetrahydropyranylphenol was dissolved in 50 mL of tetrahydrofuran, 14 mL of a 1.6 M solution of n-butyl lithium in n-hexane was added dropwise to the solution at an internal temperature of -60 °C or below, the mixture was warmed to -35 °C and cooled again to not higher than -60 °C. About 2 mL of acetaldehyde was added thereto in a gaseous condition and the mixture was stirred for 20 minutes. Water was added to the reaction solution, the mixture was warmed to room temperature and extracted once with ethyl acetate. The organic layer was washed once with water and once with an aqueous saturated solution of sodium chloride and dried over anhydrous sodium sulfate. The solvent was distilled off under reduced pressure and the residue was purified by silica gel column chromatography (n-hexane:ethyl acetate=5:1-3:1) to give 2.45 g of a mixture of diastereomers of 2,3,4-trifluoro-6-(1-hydroxyethyl)-5-pivaloylamino-O-tetrahydropyranylphenol (yield: 60%).

FAB-MS (M/Z) 376 ($M^+$+H)
Molecular formula $C_{18}H_{24}F_3NO_4$=375

(7) 2.45 g (6.53 mmol) of the above 2,3,4-trifluoro-6-(1-hydroxyethyl)-5-pivaloylamino-O-tetrahydropyranylphenol was dissolved in 50 mL of acetone, a Jones reagent was added thereto under ice-cooling until a starting material was consumed and 2 mL of 2-propanol was added. Water was added to the reaction solution and the mixture was extracted twice with ethyl acetate. The organic layer was washed twice with water and once with an aqueous saturated solution of sodium chloride and dried over anhydrous sodium sulfate. The solvent was distilled off under reduced pressure, the residue was dissolved in 40 mL of tetrahydrofuran, 10 mL of 1N hydrochloric acid was added and the mixture was stirred at room temperature for 40 minutes. Water was added to the reaction solution and the mixture was extracted once with ethyl acetate. The organic layer was washed once with an aqueous saturated solution of sodium chloride and dried over anhydrous sodium sulfate. The solvent was distilled off under reduced pressure and the residue was purified by silica gel column chromatography (chloroform:ethyl acetate=50:1) to give 1.26 g of compound XIV (two stage yield: 75%).

NMR (90MHz, $CDCl_3$) δ (ppm) 1.34 (s, 9H), 2.50 (s, 3H), 7.55 (brs, 1H), 11.1 (brs, 1H)
FAB-MS (M/Z) 290 ($M^+$+H)
Molecular formula $C_{13}H_{14}F_3NO_3$=289

Reference Example 8

4-Acetylamino-3-fluorobenzoic acid (Compound XV-1)

696 mg (3.0 mmol) of 4-bromo-2-fluoroacetanilide was dissolved in 12 mL of tetrahydrofuran under argon atmosphere and the solution was cooled to not higher than -60 °C. 4.1 mL of a 1.6M solution of n-butyl lithium in n-hexane was added to the solution and the mixture was stirred for 20 minutes. About 2 g of dry ice was added thereto and the mixture was stirred for 1.5 hours. Water was added to the reaction solution, the mixture was warmed to room temperature, a 1N aqueous solution of sodium hydroxide was added to adjust the pH to 10 or above, ethyl acetate was added and the two layers were separated. 4N hydrochloric acid was added to the aqueous layer to adjust the pH to 1 and the mixture was stirred under ice-cooling. The precipitated crystals were collected by filtration to give 378 mg of compound XV-1 (yield: 64%).

NMR (90MHz, DMSO-$d_6$) δ (ppm) 2.14 (s, 3H), 7.6-7.8 (m, 2H), 8.19 (t, 1H, J=8.4Hz), 9.90 (brs, 1H)
EIMS (M/Z) 197 ($M^+$)
Molecular formula $C_9H_8FNO_3$=197

**Claims**

**1.** 5-Aminoflavone derivatives represented by the formula (I):

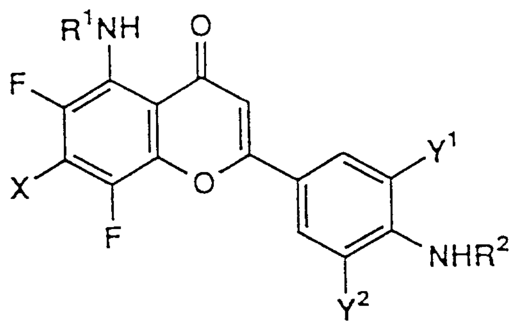

(I)

wherein $R^1$ and $R^2$ are the same or different and represent hydrogen or substituted or unsubstituted $C_1$-$C_8$ alkyl, X represents substituted or unsubstituted $C_1$-$C_8$ alkyl, $C_2$-$C_6$ alkenyl, $C_2$-$C_6$ alkynyl, halogen, hydroxy, substituted or unsubstituted $C_1$-$C_8$ alkoxy, $NR^3R^4$ (wherein $R^3$ and $R^4$ are the same or different and represent hydrogen, or substituted or unsubstituted $C_1$-$C_8$ alkyl, or $R^3$ and $R^4$ are taken together to form a heterocyclic group containing the nitrogen atom in the ring), $C_1$-$C_8$ alkylthio, $C_1$-$C_8$ alkylsulfinyl, $C_1$-$C_8$ alkylsulfonyl, carboxy, $C_1$-$C_8$ alkoxycarbonyl, $C_1$-$C_7$ alkanoyl, azido, cyano, substituted or unsubstituted carbamoyl or $C_1$-$C_8$ alkylthiothiocarbonyl, $Y^1$ and $Y^2$ are the same or different and represent hydrogen, halogen or $C_1$-$C_8$ alkyl, wherein the substituted $C_1$-$C_8$ alkyl and alkoxy have the same or different 1 to 3 substituents, selected from, halogen, hydroxy, $C_1$-$C_8$ alkoxy, $C_1$-$C_8$ alkanoyloxy, hydroxysulfonyloxy and $NR^5R^6$ (wherein $R^5$ and $R^6$ are the same as defined for $R^3$ and $R^4$) and the substituted carbamoyl has the same or different 1 to 2 substituents, selected from $C_1$-$C_8$ alkyl or pharmaceutically acceptable salts thereof;

**2.** The 5-aminoflavone derivatives according to claim 1, wherein $R^1$ and $R^2$ are hydrogen: or pharmaceutically acceptable salts thereof.

**3.** The 5-aminoflavone derivatives according to claim 2, wherein one of $Y^1$ and $Y^2$ is hydrogen and the other is fluorine: or pharmaceutically acceptable salts thereof.

**4.** The 5-aminoflavone derivatives according to claim 3, wherein X is substituted or unsubstituted lower alkyl: or pharmaceutically acceptable salts thereof.

**5.** The 5-aminoflavone derivatives according to claim 4 which is:

5-amino-2-(4-amino-3-fluorophenyl)-6,8-difluoro-7-methyl-4H-1-benzopyran-4-one,

5-amino-2-(4-amino-3-fluorophenyl)-6,8-difluoro-7-hydroxymethyl-4H-1-benzopyran-4-one,
5-amino-2-(4-amino-3-fluorophenyl)-7-aminomethyl-6,8-difluoro-4H-1-benzopyran-4-one,
hydrochloride of 5-amino-2-(4-amino-3-fluorophenyl)-7-dimethylaminomethyl-6,8-difluoro-4H-1-benzopyran-4-one,
7-acetoxymethyl-5-amino-2-(4-amino-3-fluorophenyl)-6,8-difluoro-4H-1-benzopyran-4-one,
5-amino-2-(4-amino-3-fluorophenyl)-6,8-difluoro-7-(1-propanoyloxymethyl)-4H-1-benzopyran-4-one, or
5-amino-2-(4-amino-3-fluorophenyl)-6,8-difluoro-7-(1-hexanoyloxymethyl)-4H-1-benzopyran-4-one:
or pharmaceutically acceptable salts thereof.

6. The 5-aminoflavone derivatives according to claim 3, wherein X is $NR^3R^4$ (wherein $R^3$ and $R^4$ are the same as defined above) or azido, or pharmaceutically acceptable salts thereof.

7. The 5-aminoflavone derivatives according to claim 6 which is:

5-amino-2-(4-amino-3-fluorophenyl)-7-azido-6,8-difluoro-4H-1-benzopyran-4-one,
5,7-diamino-2-(4-amino-3-fluorophenyl)-6,8-difluoro-4H-1-benzopyran-4-one, or
5-amino-2- (4-amino-3-fluorophenyl)-7-dimethylamino-6,8-difluoro-4H-1-benzopyran-4-one,
or pharmaceutically acceptable salts thereof.

8. A pharmaceutical composition containing a 5-aminoflavone derivative as defined in any of claims 1 to 7, optionally in combination with a pharmaceutically acceptable carrier and/or diluent.

9. Use of a 5-aminoflavone derivative as defined in any of claims 1 to 7, for the preparation of a pharmaceutical composition possessing antibacterial, anti-estrogenic and/or antitumor activity.

**Patentansprüche**

1. 5-Aminoflavonderivate der Formel (I):

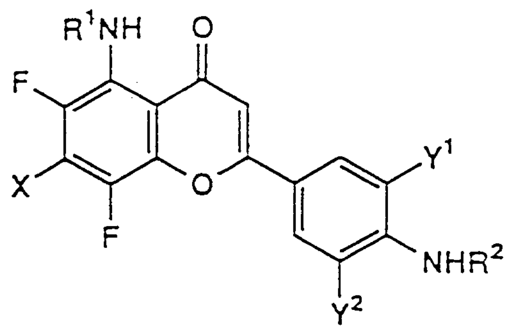

(I)

wobei $R^1$ und $R^2$ gleich oder verschieden sind und ein Wasserstoffatom oder einen substituierten oder unsubstituierten $C_1$-$C_8$-Alkylrest darstellen, X einen substituierten oder unsubstituierten $C_1$-$C_8$-Alkylrest, $C_2$-$C_6$-Alkenylrest, $C_2$-$C_6$-Alkinylrest, Halogenatom, Hydroxygruppe, substituierten oder unsubstituierten $C_1$-$C_8$-Alkoxyrest, den Rest $NR^3R^4$ (wobei $R^3$ und $R^4$ gleich oder verschieden sind und ein Wasserstoffatom oder einen substituierten oder unsubstituierten $C_1$-$C_8$-Alkylrest darstellen, oder $R^3$ und $R^4$ zusammen einen heteroyclischen Rest bilden, der ein Stickstoffatom im Ring enthält), $C_1$-$C_8$-Alkylthiorest, $C_1$-$C_8$-Alkylsufinylrest, $C_1$-$C_8$-Alkylsulfonylrest, Carboxygruppe, $C_1$-$C_8$-Alkoxycarbonylrest, $C_1$-$C_7$-Alkanoylrest, Azidogruppe, Cyanogruppe, substituierten oder unsubstituierten Carbamoylrest oder $C_1$-$C_8$-Alkylthiothiocarbonylrest darstellt, $Y^1$ und $Y^2$ gleich oder verschieden sind und ein Wasserstoffatom, Halogenatom oder einen $C_1$-$C_8$-Alkylrest darstellen, wobei die substituierten $C_1$-$C_8$-Alkylreste und Alkoxyreste die gleichen oder verschiedene 1 bis 3 Substituenten haben, ausgewählt aus Halogenatomen, Hydroxygruppen, $C_1$-$C_8$-Alkoxyresten, $C_1$-$C_8$-Alkanoyloxyresten, Hydroxysulfonyloxygruppen und den Resten $NR^5R^6$ (wobei $R^5$ und $R^6$ wie für $R^3$ und $R^4$ definiert sind), und der substituierte Carbamoylrest die gleichen oder verschiedene 1 bis 2 Substituenten hat,
ausgewählt aus $C_1$-$C_8$-Alkylresten,
oder ihre pharmazeutisch verträglichen Salze.

2. 5-Aminoflavonderivate nach Anspruch 1, wobei $R^1$ und $R^2$ Wasserstoffatome darstellen, oder ihre pharmazeutisch verträglichen Salze.

3. 5-Aminoflavonderivate nach Anspruch 2, wobei einer der Reste $Y^1$ und $Y^2$ ein Wasserstoffatom darstellt und der andere ein Fluoratom, oder ihre pharmazeutisch verträglichen Salze.

4. 5-Aminoflavonderivate nach Anspruch 3, wobei X einen substituierten oder unsubstituierten Niederalkylrest darstellt, oder ihre pharmazeutisch verträglichen Salze.

5. 5-Aminoflavonderivate nach Anspruch 4, nämlich

5-Amino-2-(4-amino-3-fluorphenyl)-6,8-difluor-7-methyl-4H-1-benzopyran-4-on,
5-Amino-2-(4-amin-3-fluorphenyl)-6,8-difluor-7-hydroxymethyl-4H-1-benzopyran-4-on,
5-Amino-2-(4-amino-3-fluorphenyl)-6,8-difluor-7-aminomethyl-4H-1-benzopyran-4-on,
5-Amino-2-(4-amino-3-fluorphenyl)-6,8-difluor-7-dimethylaminomethyl-4H-1-benzopyran-4-on-Hydrochlorid,
7-Acetoxymethyl-5-amino-2-(4-amino-3-fluorphenyl)-6,8-difluor-4H-1-benzopyran-4-on,
5-Amino-2-(4-amino-3-fluorphenyl)-6,8-difluor-7-(1-propanoyloxymethyl)-4H-1-benzopyran-4-on oder
5-Amino-2-(4-amino-3-fluorphenyl)-6,8-difluor-7-(1-hexanoyloxymethyl)-4H-1-benzopyran-4-on,
oder ihre pharmazeutisch verträglichen Salze.

6. 5-Aminoflavonderivate nach Anspruch 3, wobei X einen Rest $NR^3R^4$ (wobei $R^3$ und $R^4$ wie vorstehend definiert sind) oder eine Azidogruppe darstellt, oder ihre pharmazeutisch verträglichen Salze.

7. 5-Aminoflavonderivate nach Anspruch 6, nämlich

5-Amino-2-(4-amino-3-fluorphenyl)-7-azido-6,8-difluor-4H-1-benzopyran-4-on,
5,7-Diamino-2-(4-amino-3-fluorphenyl)-6,8-difluor-4H-1-benzopyran-4-on oder
5-Amino-2-(4-amino-3-fluorphenyl)-7-dimethylamino-6,8-difluor-4H-1-benzopyran-4-on,
oder ihre pharmazeutisch verträglichen Salze.

8. Arzneimittel, das ein 5-Aminoflavonderivat nach einem der Ansprüche 1 bis 7 enthält, gegebenenfalls in Kombination mit einem pharmazeutisch verträglichen Träger und/oder Verdünnungsmittel.

9. Verwendung eines 5-Aminoflavonderivats nach einem der Ansprüche 1 bis 7 zur Herstellung eines Arzneimittels, das antibakterielle, anti-östrogene und/oder Antitumorwirkung besitzt.

## Revendications

1. Dérives de 5-aminoflavone représentés par la formule (I) :

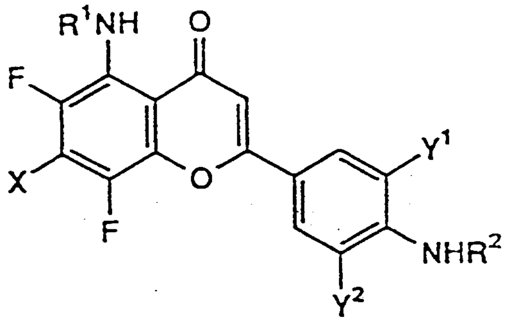

(I)

dans laquelle :

- $R^1$ et $R^2$ sont identiques ou différents et représentent hydrogène ou alkyle en $C_1$-$C_8$ substitué ou non substitué ;
- X représente alkyle en $C_1$-$C_8$ substitué ou non substitué, alcényle en $C_2$-$C_6$, alcynyle en $C_2$-$C_6$, halogène,

hydroxy, alcoxy en $C_1$-$C_8$ substitué ou non substitué, $NR^3R^4$ (où $R^3$ et $R^4$ sont identiques ou différents et représentent hydrogène ou alkyle en $C_1$-$C_8$ substitué ou non substitué, ou bien $R^3$ et $R^4$ sont pris ensemble pour former un groupe hétérocyclique contenant l'atome d'azote dans le cycle), alkylthio en $C_1$-$C_8$, alkylsulfinyle en $C_1$-$C_8$, alkylsulfonyle en $C_1$-$C_8$, carboxy, (alcoxy en $C_1$-$C_8$)-carbonyle, alcanoyle en $C_1$-$C_7$, azido, cyano, carbamoyle substitué ou non substitué ou (alkyl en $C_1$-$C_8$)-thiothiocarbonyle ;

- $Y^1$ et $Y^2$ sont identiques ou différents et représentent hydrogène, halogène ou alkyle en $C_1$-$C_8$,
- les groupes alkyle en $C_1$-$C_8$ et alcoxy en $C_1$-$C_8$ qui sont substitués ayant 1 à 3 substituants, identiques ou différents, choisis parmi halogène, hydroxy, alcoxy en $C_1$-$C_8$, alcanoyloxy en $C_1$-$C_8$, hydroxysulfonyloxy et $NR^5R^6$ (où $R^5$ et $R^6$ sont les mêmes que ceux définis pour $R^3$ et $R^4$) et le groupe carbamoyle substitué ayant 1 ou 2 substituants, identiques ou différents, choisis parmi alkyle en $C_1$-$C_8$ ;

ou les sels pharmaceutiquement acceptables de ces dérivés.

**2.** Dérivés de 5-aminoflavone selon la revendication 1, dans lesquels $R^1$ et $R^2$ représentent hydrogène ; ou les sels pharmaceutiquement acceptables de ces dérivés.

**3.** Dérivés de 5-aminoflavone selon la revendication 2, dans lesquels l'un parmi $Y^1$ et $Y^2$ représente hydrogène et l'autre représente fluor ; ou les sels pharmaceutiquement acceptables de ces dérivés.

**4.** Dérivés de 5-aminoflavone selon la revendication 3, dans lesquels X représente alkyle inférieur substitué ou non substitué ; ou les sels pharmaceutiquement acceptables de ces dérivés.

**5.** Dérivés de 5-aminoflavone selon la revendication 4, qui sont :

- la 5-amino-2-(4-amino-3-fluorophényl)-6,8-difluoro-7-méthyl-4H-1-benzopyran-4-one ;
- la 5-amino-2-(4-amino-3-fluorophényl)-6,8-difluoro-7-hydroxyméthyl-4H-1-benzopyran-4-one ;
- la 5-amino-2-(4-amino-3-fluorophényl)-7-aminométhyl-6,8-difluoro-4H-1-benzopyran-4-one ;
- le chlorhydrate de 5-amino-2-(4-amino-3-fluorophényl)-7-diméthylaminométhyl-6,8-difluoro-4H-1-benzopyran-4-one ;
- la 7-acétoxyméthyl-5-amino-2-(4-amino-3-fluorophényl)-6,8-difluoro-4H-1-benzopyran-4-one ;
- la 5-amino-2-(4-amino-3-fluorophényl)-6,8-difluoro-7-(1-propanoyloxyméthyl)-4H-1-benzopyran-4-one ; ou
- la 5-amino-2-(4-amino-3-fluorophényl)-6,8-difluoro-7-(1-hexanoyloxyméthyl)-4H-1-benzopyran-4-one ;

ou les sels pharmaceutiquement acceptables de ces dérivés.

**6.** Dérivés de 5-aminoflavone selon la revendication 3, dans lesquels X représente $NR^3R^4$ (où $R^3$ et $R^4$ sont les mêmes que ceux définis ci-dessus) ou azido, ou les sels pharmaceutiquement acceptables de ces dérivés.

**7.** Dérivés de 5-aminoflavone selon la revendication 6, qui sont :

- la 5-amino-2-(4-amino-3-fluorophényl)-7-azido-6,8-difluoro-4H-1-benzopyran-4-one ;
- la 5,7-diamino-2-(4-amino-3-fluorophényl)-6,8-difluoro-4H-1-benzopyran-4-one ; ou
- la 5-amino-2-(4-amino-3-fluorophényl)-7-diméthylamino-6,8-difluoro-4H-1-benzopyran-4-one,

ou les sels pharmaceutiquement acceptables de ces dérivés.

**8.** Composition pharmaceutique contenant un dérivé de 5-aminoflavone tel que défini à l'une quelconque des revendications 1 à 7, le cas échéant en combinaison avec un support et/ou diluant pharmaceutiquement acceptable.

**9.** Utilisation d'un dérivé de 5-aminoflavone tel que défini à l'une quelconque des revendications 1 à 7, pour la préparation d'une composition pharmaceutique possédant une activité antibactérienne, anti-oestrogénique et/ou antitumorale.